# EUROPEAN PATENT APPLICATION

(11) **EP 4 491 270 A1**
(43) Date of publication of application: **15.01.2025**
(21) Application number: 24187156.5
(22) Date of filing: 08.07.2024
(51) Int. Cl.: B01J 31/18, C07C 29/48

(54) **IRON COMPLEXES FOR ENANTIOSELECTIVE CIS DIHYDROXYLATION OF ALKENES**

(30) Priority: 12.07.2023 US 202363526430 P
(71) Applicant: The University of Hong Kong, Hong Kong (HK); Laboratory for Synthetic Chemistry and Chemical Biology Limited, Hong Kong Shatin (HK)
(72) Inventor: CHE, Chi Ming, Hong Kong (HK); WANG, Tingting, Hong Kong (HK)
(74) Representative: HGF

(57) **Abstract**

Methods for asymmetric *cis*-dihydroxylation ("AD") of styrenes and conjugated dienes to produce *cis*-diols of styrene and conjugated diene or tetraols of conjugated diene with high yield (i.e., a yield ≥ 30%) and high enantioselectivity (i.e., an enantiometric excess ≥ 60%) are disclosed. The method uses an iron-based catalyst, such as one or more Fe(II) complexes, as the catalyst. The method generally includes: (i) maintaining a reaction mixture at a temperature for a period of time sufficient to form a product, where the reaction mixture contains a styrene or conjugated diene, one or more iron-based catalyst(s), an oxidant, and a solvent, and where the product contains a *cis*-diol or tetraol.

## Description

### CROSS-REFERENCE TO RELATED APPLICATION

This application claims the benefit of and priority to U.S. Provisional Application No. 63/526,430 filed July 12, 2023, the entire content of which is incorporated herein by reference for all purpose in its entirety.

### FIELD OF THE INVENTION

This invention is generally in the field of asymmetric hydroxylation of alkenes, in particular styrene, styrene derivatives, and conjugated dienes, using iron-based catalysts.

### BACKGROUND OF THE INVENTION

Alkenes are abundant in industrial chemistry and transformation of alkenes into value-added products are still hot spot in organic chemistry (Tetrahedron Lett. 2015, 56, 3732; Chem. Rec. 2019, 19, 440*;* Angew. Chem. Int. Ed. 2020, 59, 7990). Asymmetric *cis-*dihydroxylation of alkenes is an effective method of constructing vicinal diols prevalent in natural pharmaceuticals and active molecules (Chem. Soc. Rev. 2011, 40, 114; Tetrahedron: Asymmetry, 2017, 28, 987; Russ. Chem. Rev, 2019, 88, 1094; Molecules, 2023, 28, 2722). Currently, AD reactions are mainly performed with Os catalysts such as the commercial products AD-mix-α/β developed by Sharpless and co-workers. Nevertheless, the high toxicity of Os catalysts, together with the high cost and poor atom economy of the catalytic systems, has raised serious concerns in pharmaceutical industry and other practical synthetic applications. Moreover, the electrophilic nature of the Os oxidants favors electron-rich alkenes over electron-deficient alkenes, and a higher Os loading is typically required for the latter substrates which causes more serious Os contamination in the products.

Several metal catalysts, including Ru, Mn, and Fe, together with other strategies, have been exploited in the literature as Os-free alkene *cis*-dihydroxylation methods, yet it is still challenging for these methods to replace the current Sharpless dihydroxylation reaction due to their limitations in substrate, product yield, stereoselectivity (enantioselectivity and *cis-*/*trans-diol* selectivity), and/or chemoselectivity (e.g. diol/epoxide selectivity) (J. Am. Chem. Soc., 2005, 127, 14239; Angew. Chem. Int. Ed. 2008, 47, 1887; Chem. Commun., 2011, 47, 11204; Angew. Chem. Int. Ed. 2016, 55, 10253; Schmidt, S. and Plietker, B. 2017 Dihydroxylations of Olefins and Related Reactions. In Applied Homogeneous Catalysis with Organometallic Compounds, eds B. Cornils, W.A. Herrmann, M. Beller and R. Paciello). Rieske dioxygenases are a class of enzymes that catalyze biological AD reactions, and the nonheme Fe active center has stimulated extensive bioinorganic research in developing Fe-catalyzed AD reactions as a green alternative to the Sharpless AD reaction. The use of H₂O₂ is also more favorable in terms of atom economy than the terminal oxidants used in Os catalytic systems (e.g. ferricyanide). There are four effective chiral N₄ ligand applied in Fe-catalyzed asymmetric *cis*-dihydroxylation including 6-Me₂-BPMCN, 6-Me₂-BPBP, 2-Me₂-BQCN, 2-Me₂-BQPN. However, when it comes to styrene substrates, [Fe(*R*,*R*-2-Me₂-BPMCN)(OTf)₂] and Fe(*R*,*R*-2-Me₂-BQCN)(OTf)₂] gave the diol products with low enantioselectivities *(2% ee* and 17.5% *ee*) (Angew. Chem. Int. Ed. 2008, 47, 1887; Angew. Chem. Int. Ed. 2016, 55, 10253). Optically pure 1,2-diols of teminal aromatic alkenes are important in bioactive molecules; however, methods and catalysts that afford high reactivity/selectivity are still lacking

Asymmetric *cis*-dihydroxylation of conjugated dienes is an attractive method of producing four contiguous chiral centers. For mono-dihydroxylation of electron-deficient conjugated dienes, Osmium-based catalyst prefererentially gave mono-dihydroxylation of most electron-rich double bond (J. Am. Chem. Soc. 1992, 114, 7570). It is challenging to achieve *cis*-dihydroxylation of the two double bonds simultaneously and avoid poor regiocontrol and over-oxidation. Chiral *cis*-diols of electron-deficient conjugated dienes were achieved through two separate dihydroxylation reactions catalyzed by Osmium-based catalyst [Tetrahedron Letters, 2011, 52, 6366]. However, efficient asymmetric *cis-*dihydroxylation of conjugated diene in a single reaction is still lacking.

There remains a need to develop improved methods for asymmetric *cis-*dihydroxylation of styrenes and conjugated dienes.

Therefore, it is the object of the present invention to provide methods for asymmetric *cis*-dihydroxylation of styrenes and conjugated dienes.

It is a further object of the present invention to provide iron-based catalysts for catalyzing the asymmetric *cis*-dihydroxylation of styrenes and conjugated dienes.

It is a further object of the present invention to provide *cis*-diol products formed from asymmetric *cis*-dihydroxylation of styrenes and conjugated dienes.

### SUMMARY OF THE INVENTION

Fe(II) complexes for catalyzing asymmetric *cis*-dihydroxylation (AD) of styrenes and conjugated dienes have been developed. Methods for asymmetric *cis*-dihydroxylation (AD) of styrenes and conjugated dienes to produce *cis*-diols of styrenes and conjugated dienes, and tetraols of conjugated dienes have been developed. In some forms, the AD of styrenes and conjugated dienes catalyzed with the Fe(II) complexes using the disclosed methods have high yield and/or high enantioselectivity.

The methods described herein use the iron-based catalyst, such as one or more Fe(II) complexes, as the catalyst, and a terminal oxidant, such as H₂O₂. In some forms, the described methods can be performed under mild reaction conditions (e.g., up to about 30°C at 1 atm, such as room temperature at 1atm). In some forms, the described methods can be performed below 0°C at 1atm (e.g., about -30°C). The methods described herein have at least the following advantages: (1) *cis*-diols of styrenes and conjugated dienes and tetraols of conjugated dienes can be produced with high yield and high enantioselectivity; (2) the use of biocompatible iron-based catalysts is environmentally friendly and cost efficient; and (3) the waste generated from the reaction is non-toxic.

The method generally includes: (i) maintaining a reaction mixture at a temperature for a period of time sufficient to form a product, where the reaction mixture contains a styrene, a styrene derivative, or a conjugated diene, one or more iron-based catalyst(s), a solvent, and optionally an oxidant (e.g., hydrogen peroxide). The product contains a *cis-*diol or a tetraol. In some forms, the methods disclosed herein convert a styrene or a styrene derivative to a *cis*-diol via AD reaction. In some forms, the methods disclosed herein convert a conjugated diene to a *cis*-diol via AD reaction. In some forms, the methods disclosed herein convert a conjugated diene to a tetraol via AD reaction.

In some forms, the specific reaction conditions, such as the amount of the oxidant, the amount of the catalyst(s), the specific catalyst, the reaction temperature, and reaction time period used in step (i) can be selected based on the reactant and the desired product.

For example, the reaction mixture contains a styrene or a derivative thereof and 0.1 mol% to 20 mol% iron catalyst, and can be maintained at a temperature below freezing temperature, such as -30°C, for a period of time in a range from about 20 minutes to about 3 hours, from about 30 minutes to about 2 hours, or from about 30 minutes to 1 hour, such as about 30 minutes, to form a product containing a *cis*-diol.

For example, the reaction mixture contains a 1,3-diol and up to 6 mol% iron catalyst, and can be maintained at room temperature for a period of time in a range from about 20 minutes to about 3 hours, from about 30 minutes to about 2 hours, or from about 30 minutes to 1 hour, such as about 30 minutes, to form a product containing a *cis*-diol.

For example, the reaction mixture contains a 1,3-diol and >6 mol% iron catalyst, and can be maintained at room temperature for a period of time in a range from about 30 minutes to about 3 hours, from about 1 hour to about 3 hours, or from about 1 hour to about 2 hour, such as about 1 hour, to form a product containing a tetraol.

Further, in some forms, one can choose the iron-based catalyst in the AD reaction to achieve a desired stereochemistry. For example, when the disclosed methods convert a conjugated diene to a *cis*-diol or tetraol via AD reaction catalyzed by Fe(*S*,*S*-PQCN-6)(OTf)₂ described herein, the cis-diol or tetraol of the conjugated diene can have a (2*S*,3*R*) or (*2S,3R,4S,5R*) stereochemistry. For example, when the disclosed methods convert a conjugated diene to a *cis*-diol or tetraol via AD reaction catalyzed by Fe(S,S-2-Me₂-BQCN)(OTf)₂ described herein, the cis-diol or tetraol of the conjugated diene can have a (*2R,3S*) or (2*R*,3*S,4R,5S*) stereochemistry.

Optionally, the method can also include adding the oxidant into a pre-mixture that contains the substrate, iron catalyst, and solvent to form the reaction mixture prior to step (i); stirring the reaction mixture prior to and/or during step (i); and/or purifying the product, optionally by column chromatography, subsequent to step (i). The oxidant may be added into the pre-mixture using any suitable technique, such as by a swift injection or a continuous injection using a syringe pump over a period of time (e.g., about 30 mins), at room temperature. In some forms, the specific technique for adding the oxidant can be selected based on the desired product. For example, when the desired product is a cis-diol, the oxidant, in the form of a solution, may be added into the pre-mixture using a syringe pump over a suitable time period (such as a time period ranging from 20 mins to 1 hour, e.g., about 30 mins), at room temperature. For example, when the desired product is a tetraol, the oxidant, in the form of a solution, may be added into the pre-mixture directly (i.e., a swift injection) at room temperature.

### BRIEF DESCRIPTION OF THE DRAWINGS

**Figure 1** is a schematic diagram showing the X-ray crystal structure of [*Fe*(*S,S-*PQCN-1)(OTf)₂].
**Figure 2** is a schematic diagram showing the X-ray crystal structure of (*2R,3S,4R,5S*)*-***5h***.*
**Figures 3A and 3B** are schematic diagrams showing DFT calculations on transition states for the asymmetric *cis*-dihydroxylation of styrene by the Fe-dioxo calculated at the B3LYP-D3/def2-TZVP(PCM)//B3LYP-D3/def2-SVP(PCM) level of theory.
**Figure 4** is a schematic diagram showing the proposed correlation between dcc and ee for the asymmetric *cis*-dihydroxylation of styrene by chiral unsymmetric Fe-N4 complexes based on DFT calculations calculated at the B3LYP-D3/def2-TZVP(PCM)// B3LYP-D3/def2-SVP(PCM) level of theory.

### DETAILED DESCRIPTION OF THE INVENTION

It is to be understood that the disclosed compounds, compositions, and methods are not limited to specific synthetic methods, specific analytical techniques, or to particular reagents unless otherwise specified, and, as such, may vary. It is also to be understood that the terminology used herein is for the purpose of describing particular forms and embodiments only and is not intended to be limiting.

"Substituted," as used herein, refers to all permissible substituents of the compounds or functional groups described herein. In the broadest sense, the permissible substituents include acyclic and cyclic, branched and unbranched, carbocyclic and heterocyclic, aromatic and nonaromatic substituents of organic compounds. Illustrative substituents include, but are not limited to, halogens, hydroxyl groups, or any other organic groupings containing any number of carbon atoms, preferably 1-14 carbon atoms, and optionally include one or more heteroatoms such as oxygen, sulfur, or nitrogen grouping in linear, branched, or cyclic structural formats. Representative substituents include a substituted or unsubstituted alkyl, a substituted or unsubstituted alkenyl, a substituted or unsubstituted alkynyl, a substituted or unsubstituted heterocyclyl, a substituted or unsubstituted phenyl, a substituted or unsubstituted aryl, a substituted or unsubstituted heteroaryl, a substituted or unsubstituted polyaryl, a substituted or unsubstituted polyheteroaryl, a substituted or unsubstituted aralkyl, a halogen, a hydroxyl, an alkoxy, a phenoxy, an aroxy, a silyl, a thiol, an alkylthio, a substituted alkylthio, a phenylthio, an arylthio, a cyano, an isocyano, a nitro, a substituted or unsubstituted carbonyl, a carboxyl, an amino, an amido, an oxo, a sulfinyl, a sulfonyl, a sulfonic acid, a phosphonium, a phosphanyl, a phosphoryl, a phosphonyl, an amino acid. Such a substituted or unsubstituted alkyl, a substituted or unsubstituted alkenyl, a substituted or unsubstituted alkynyl, a substituted or unsubstituted heterocyclyl, a substituted or unsubstituted phenyl, a substituted or unsubstituted aryl, a substituted or unsubstituted heteroaryl, a substituted or unsubstituted polyaryl, a substituted or unsubstituted polyheteroaryl, a substituted or unsubstituted aralkyl, a halogen, a hydroxyl, an alkoxy, a phenoxy, an aroxy, a silyl, a thiol, an alkylthio, a substituted alkylthio, a phenylthio, an arylthio, a cyano, an isocyano, a nitro, a substituted or unsubstituted carbonyl, a carboxyl, an amino, an amido, an oxo, a sulfinyl, a sulfonyl, a sulfonic acid, a phosphonium, a phosphanyl, a phosphoryl, a phosphonyl, and an amino acid can be further substituted.

Heteroatoms such as nitrogen may have hydrogen substituents and/or any permissible substituents of organic compounds described herein which satisfy the valences of the heteroatoms. It is understood that "substitution" or "substituted" includes the implicit proviso that such substitution is in accordance with permitted valence of the substituted atom and the substituent, and that the substitution results in a stable compound, *i.e.,* a compound that does not spontaneously undergo transformation such as by rearrangement, cyclization, elimination, etc.

"Alkyl," as used herein, refers to the radical of saturated aliphatic groups, including straight-chain alkyl groups, branched-chain alkyl, and cycloalkyl (alicyclic). In some forms, a straight chain or branched chain alkyl has 30 or fewer carbon atoms in its backbone (e.g., C₁-C₃₀ for straight chains, C₃-C₃₀ for branched chains), 20 or fewer, 15 or fewer, or 10 or fewer. Alkyl includes methyl, ethyl, *n*-propyl, isopropyl, *n*-butyl, isobutyl, *t*-butyl, pentyl, hexyl, heptyl, octyl, decyl, tetradecyl, hexadecyl, eicosyl, tetracosyl and the like. Likewise, a cycloalkyl is a non-aromatic carbon-based ring composed of at least three carbon atoms, such as a nonaromatic monocyclic or nonaromatic polycyclic ring containing 3-30 carbon atoms, 3-20 carbon atoms, or 3-10 carbon atoms in their ring structure, and have 5, 6 or 7 carbons in the ring structure. Cycloalkyls containing a polycyclic ring system can have two or more non-aromatic rings in which two or more carbons are common to two adjoining rings (i.e., "fused cycloalkyl rings"). Examples of cycloalkyl groups include, but are not limited to, cyclopropyl, cyclobutyl, cyclopentyl, cyclohexyl, cycloheptyl, cyclooctanyl, etc.

"Substituted alkyl" refers to alkyl moieties having one or more substituents replacing a hydrogen on one or more carbons of the hydrocarbon backbone. Such substituents can be any substituents described above, e.g., halogen (such as fluorine, chlorine, bromine, or iodine), hydroxyl, carbonyl (such as a carboxyl, alkoxycarbonyl, formyl, or an acyl), thiocarbonyl (such as a thioester, a thioacetate, or a thioformate), aryl, alkoxyl, aralkyl, phosphonium, phosphanyl, phosphonyl, phosphoryl, phosphate, phosphonate, a phosphinate, amino, amido, amidine, imine, cyano, nitro, azido, oxo, sulfhydryl, thiol, alkylthio, silyl, sulfinyl, sulfate, sulfonate, sulfamoyl, sulfonamido, sulfonyl, heterocyclyl, an aromatic or heteroaromatic moiety. -NRR', wherein R and R' are independently hydrogen, alkyl, or aryl, and wherein the nitrogen atom is optionally quaternized; -SR, wherein R is a phosphonyl, a sulfinyl, a silyl a hydrogen, an alkyl, or an aryl; -CN; -NO₂; -COOH; carboxylate; -COR, -COOR, or -CON(R)₂, wherein R is hydrogen, alkyl, or aryl; imino, silyl, ether, haloalkyl (such as -CF3, -CH₂-CF₃, -CCl₃); -CN; -NCOCOCH₂CH_{2;} -NCOCOCHCH; and -NCS; and combinations thereof.

It will be understood by those skilled in the art that the moieties substituted on the hydrocarbon chain can themselves be substituted, if appropriate. For instance, the substituents of a substituted alkyl may include halogen, hydroxy, nitro, thiols, amino, aralkyl, azido, imino, amido, phosphonium, phosphanyl, phosphoryl (including phosphonate and phosphinate), oxo, sulfonyl (including sulfate, sulfonamido, sulfamoyl and sulfonate), and silyl groups, as well as ethers, alkylthios, carbonyls (including ketones, aldehydes, carboxylates, and esters), haloalkyls, -CN and the like. Cycloalkyls can be substituted in the same manner.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths.

"Heteroalkyl," as used herein, refers to straight or branched chain, or cyclic carbon-containing alkyl radicals, or combinations thereof, containing at least one heteroatom on the carbon backbone. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkyl group" is a cycloalkyl group as defined above where at least one of the carbon atoms of the ring is substituted with a heteroatom such as, but not limited to, nitrogen, oxygen, sulphur, or phosphorus.

The term "alkenyl" as used herein is a hydrocarbon group of from 2 to 24 carbon atoms and structural formula containing at least one carbon-carbon double bond. Alkenyl groups include straight-chain alkenyl groups, branched-chain alkenyl, and cycloalkenyl. A cycloalkenyl is a non-aromatic carbon-based ring composed of at least three carbon atoms and at least one carbon-carbon double bond, such as a nonaromatic monocyclic or nonaromatic polycyclic ring containing 3-30 carbon atoms and at least one carbon-carbon double bond, 3-20 carbon atoms and at least one carbon-carbon double bond, or 3-10 carbon atoms and at least one carbon-carbon double bond in their ring structure, and have 5, 6 or 7 carbons and at least one carbon-carbon double bond in the ring structure. Cycloalkenyls containing a polycyclic ring system can have two or more non-aromatic rings in which two or more carbons are common to two adjoining rings (i.e., "fused cycloalkenyl rings") and contain at least one carbon-carbon double bond. Asymmetric structures such as (AB)C=C(C'D) are intended to include both the *E* and *Z* isomers. This may be presumed in structural formulae herein wherein an asymmetric alkene is present, or it may be explicitly indicated by the bond symbol C. The term "alkenyl" as used throughout the specification, examples, and claims is intended to include both "unsubstituted alkenyls" and "substituted alkenyls," the latter of which refers to alkenyl moieties having one or more substituents replacing a hydrogen on one or more carbons of the hydrocarbon backbone. The term "alkenyl" also includes "heteroalkenyl."

The term "substituted alkenyl" refers to alkenyl moieties having one or more substituents replacing one or more hydrogen atoms on one or more carbons of the hydrocarbon backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, oxo, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

"Heteroalkenyl," as used herein, refers to straight or branched chain, or cyclic carbon-containing alkenyl radicals, or combinations thereof, containing at least one heteroatom. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkenyl group" is a cycloalkenyl group where at least one of the carbon atoms of the ring is substituted with a heteroatom such as, but not limited to, nitrogen, oxygen, sulphur, or phosphorus.

The term "alkynyl group" as used herein is a hydrocarbon group of 2 to 24 carbon atoms and a structural formula containing at least one carbon-carbon triple bond. Alkynyl groups include straight-chain alkynyl groups, branched-chain alkynyl, and cycloalkynyl. A cycloalkynyl is a non-aromatic carbon-based ring composed of at least three carbon atoms and at least one carbon-carbon triple bond, such as a nonaromatic monocyclic or nonaromatic polycyclic ring containing 3-30 carbon atoms and at least one carbon-carbon triple bond, 3-20 carbon atoms and at least one carbon-carbon triple bond, or 3-10 carbon atoms and at least one carbon-carbon triple bond in their ring structure, and have 5, 6 or 7 carbons and at least one carbon-carbon triple bond in the ring structure. Cycloalkynyls containing a polycyclic ring system can have two or more non-aromatic rings in which two or more carbons are common to two adjoining rings (i.e., "fused cycloalkynyl rings") and contain at least one carbon-carbon triple bond. Asymmetric structures such as (AB)C=C(C"D) are intended to include both the *E* and *Z* isomers. This may be presumed in structural formulae herein wherein an asymmetric alkyne is present, or it may be explicitly indicated by the bond symbol C. The term "alkynyl" as used throughout the specification, examples, and claims is intended to include both "unsubstituted alkynyls" and "substituted alkynyls," the latter of which refers to alkynyl moieties having one or more substituents replacing a hydrogen on one or more carbons of the hydrocarbon backbone. The term "alkynyl" also includes "heteroalkynyl."

The term "substituted alkynyl" refers to alkynyl moieties having one or more substituents replacing one or more hydrogen atoms on one or more carbons of the hydrocarbon backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

"Heteroalkynyl," as used herein, refers to straight or branched chain, or cyclic carbon-containing alkynyl radicals, or combinations thereof, containing at least one heteroatom. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkynyl group" is a cycloalkynyl group where at least one of the carbon atoms of the ring is substituted with a heteroatom such as, but not limited to, nitrogen, oxygen, sulphur, or phosphorus.

"Aryl," as used herein, refers to C₄-C₂₆-membered aromatic rings or fused ring systems containing one aromatic ring and optionally one or more non-aromatic rings. Examples of aryl groups are benzene, tetralin, indane, etc.

The term "substituted aryl" refers to an aryl group, wherein one or more hydrogen atoms on one or more aromatic rings are substituted with one or more substituents including, but not limited to, halogen, azide, alkyl, aralkyl, alkenyl, alkynyl, cycloalkyl, hydroxyl, alkoxy, carbonyl (such as a ketone, aldehyde, carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (such as CF₃, -CH₂-CF₃, -CCl₃), -CN, aryl, heteroaryl, and combinations thereof.

"Heterocyclo" and "heterocyclyl" are used interchangeably, and refer to a cyclic radical attached via a ring carbon or nitrogen atom of a monocyclic ring or polycyclic ring system containing 3-30 ring atoms, 3-20 ring atoms, 3-10 ring atoms, or 5-6 ring atoms, where the polycyclic ring system contains one or more non-aromatic rings and optionally one or more aromatic rings, where at least one non-aromatic ring contains carbon and one to four heteroatoms each selected from the group consisting of non-peroxide oxygen, sulfur, and N(Y) wherein Y is absent or is H, O, C₁-C₁₀ alkyl, phenyl or benzyl, and optionally containing 1-3 double bonds and optionally substituted with one or more substituents. Heterocyclyl are distinguished from heteroaryl by definition. Heterocycles can be a heterocycloalkyl, a heterocycloalkenyl, a heterocycloalkynyl, etc, such as piperazinyl, piperidinyl, piperidonyl, 4-piperidonyl, dihydrofuro[2,3-*b*]tetrahydrofuran, morpholinyl, piperazinyl, piperidinyl, piperidonyl, 4-piperidonyl, piperonyl, pyranyl, 2H-pyrrolyl, 4*H-*quinolizinyl, quinuclidinyl, tetrahydrofuranyl, 6*H*-1,2,5-thiadiazinyl. Heterocyclic groups can optionally be substituted with one or more substituents as defined above for alkyl and aryl.

The term "heteroaryl" refers to C₃-C₂₆-membered aromatic rings or fused ring systems containing one aromatic ring and optionally one or more non-aromatic rings, in which one or more carbon atoms on the aromatic ring structure have been substituted with a heteroatom. Suitable heteroatoms include, but are not limited to, oxygen, sulfur, and nitrogen. Examples of heteroaryl groups pyrrole, furan, thiophene, imidazole, oxazole, thiazole, triazole, tetrazole, pyrazole, pyridine, pyrazine, pyridazine and pyrimidine, and the like. Examples of heteroaryl rings include, but are not limited to, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, furanyl, furazanyl, imidazolidinyl, imidazolinyl, imidazolyl, 1H-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, pteridinyl, purinyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, quinoxalinyl, tetrahydroisoquinolinyl, tetrahydroquinolinyl, tetrazolyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl and xanthenyl. One or more of the rings can be substituted as defined below for "substituted heteroaryl."

The term "substituted heteroaryl" refers to a heteroaryl group in which one or more hydrogen atoms on one or more heteroaromatic rings are substituted with one or more substituents including, but not limited to, halogen, azide, alkyl, aralkyl, alkenyl, alkynyl, cycloalkyl, hydroxyl, alkoxy, carbonyl (such as a ketone, aldehyde, carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (such as CF₃, -CH₂-CF₃, -CCl₃), -CN, aryl, heteroaryl, and combinations thereof.

The term "polyaryl" refers to a fused ring system that includes two or more aromatic rings and optionally one or more non-aromatic rings. Examples of polyaryl groups are naphthalene, anthracene, phenanthrene, chrysene, pyrene, corannulene, coronene, etc. When a fused ring system containing two or more aromatic rings and optionally one or more non-aromatic rings, in which one or more carbon atoms on two or more aromatic ring structures have been substituted with a heteroatom, the fused ring system can be referred to as a "polyheteroaryl". When a fused ring system containing two or more aromatic rings and optionally one or more non-aromatic rings, in which one or more carbon atoms in the fused ring system is substituted with a heteroatom it can be referred to as a "heteropolyaryl."

The term "substituted polyaryl" refers to a polyaryl in which one or more of the aryls are substituted, with one or more substituents including, but not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, alkylthio, sulfate, sulfonate, sulfamoyl, sulfoxide, sulfonamido, sulfonyl, heterocyclyl, alkylaryl, haloalkyl, -CN, aryl, heteroaryl, and combinations thereof. When a polyheteroaryl is involved, the chemical moiety can be referred to as a "substituted polyheteroaryl."

The term "cyclic ring" or "cyclic group" refers to a substituted or unsubstituted monocyclic ring or a substituted or unsubstituted polycyclic ring (such as those formed from single or fused ring systems), such as a substituted or unsubstituted cycloalkyl, a substituted or unsubstituted cycloalkenyl, a substituted or unsubstituted cycloalkynyl, or a substituted or unsubstituted heterocyclyl, that have from three to 30 carbon atoms, as geometric constraints permit. The substituted cycloalkyls, cycloalkenyls, cycloalkynyls, and heterocyclyls are substituted as defined above for the alkyls, alkenyls, alkynyls, and heterocyclyls, respectively.

The term "aralkyl" as used herein is an aryl group or a heteroaryl group having an alkyl, alkynyl, or alkenyl group as defined above attached to the aromatic group, such as an aryl, a heteroaryl, a polyaryl, or a polyheteroaryl. An example of an aralkyl group is a benzyl group.

The terms "alkoxyl" or "alkoxy," "aroxy" or "aryloxy," generally describe compounds represented by the formula -OR^{v}, wherein R^{v} includes, but is not limited to, a substituted or unsubstituted alkyl, a substituted or unsubstituted alkenyl, a substituted or unsubstituted alkynyl, a substituted or unsubstituted cycloalkyl, a substituted or unsubstituted heterocyclyl, a substituted or unsubstituted cycloalkenyl, a substituted or unsubstituted heterocycloalkenyl, a substituted or unsubstituted aryl, a substituted or unsubstituted heteroaryl, a substituted or unsubstituted polyaryl, a substituted or unsubstituted polyheteroaryl, a substituted or unsubstituted arylalkyl, a substituted or unsubstituted heteroalkyl, a substituted or unsubstituted alkylaryl, a substituted or unsubstituted alkylheteroaryl, a substituted or unsubstituted aralkyl, a substituted or unsubstituted carbonyl, a phosphonium, a phosphanyl, a phosphonyl, a sulfinyl, a silyl, a thiol, an amido, and an amino. Exemplary alkoxyl groups include methoxy, ethoxy, propyloxy, tert-butoxy and the like. A "lower alkoxy" group is an alkoxy group containing from one to six carbon atoms. An "ether" is two functional groups covalently linked by an oxygen as defined below. Accordingly, the substituent of an alkyl that renders that alkyl an ether is or resembles an alkoxyl, such as can be represented by one of -O-alkyl, -O-alkenyl, -O-alkynyl, -O-arakyl, -O-aryl, -O-heteroaryl, -O-polyaryl, -O-polyheteroaryl, -O-heterocyclyl, etc.

The term "substituted alkoxy" refers to an alkoxy group having one or more substituents replacing one or more hydrogen atoms on one or more carbons of the alkoxy backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, oxo, sulfate, sulfonate, sulfamoyl, sulfonamido, sulfonyl, heterocyclyl, alkylaryl, haloalkyl, -CN, aryl, heteroaryl, and combinations thereof.

The term "ether" as used herein is represented by the formula A²OA¹, where A² and A¹ can be, independently, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl, a substituted or unsubstituted polyaryl, a substituted or unsubstituted polyheteroaryl, a phosphonium, a phosphanyl, a phosphonyl, a sulfinyl, a silyl, a thiol, a substituted or unsubstituted carbonyl, an alkoxy, an amido, or an amino, described above.

The term "polyether" as used herein is represented by the formula: 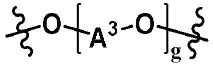 where A³ can be, independently, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl, a substituted or unsubstituted polyaryl, a substituted or unsubstituted polyheteroaryl, a phosphonium, a phosphanyl, a substituted or unsubstituted carbonyl, an alkoxy, an amido, or an amino, described above; g can be a positive integer from 1 to 30.

The term "phenoxy" is art recognized and refers to a compound of the formula -OR^{v} wherein R^{v} is C₆H₅ (*i.e.,* -O-C₆H₅). One of skill in the art recognizes that a phenoxy is a species of the aroxy genus.

The term "substituted phenoxy" refers to a phenoxy group, as defined above, having one or more substituents replacing one or more hydrogen atoms on one or more carbons of the phenyl ring. Such substituents include, but are not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, and combinations thereof.

The terms "aroxy" and "aryloxy," as used interchangeably herein, are represented by -O-aryl or -O-heteroaryl, wherein aryl and heteroaryl are as defined herein.

The terms "substituted aroxy" and "substituted aryloxy," as used interchangeably herein, represent -O-aryl or -O-heteroaryl, having one or more substituents replacing one or more hydrogen atoms on one or more ring atoms of the aryl and heteroaryl, as defined herein. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The term "amino" as used herein includes the group 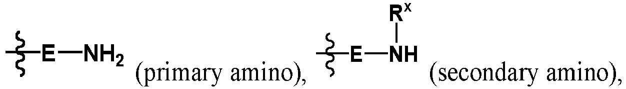 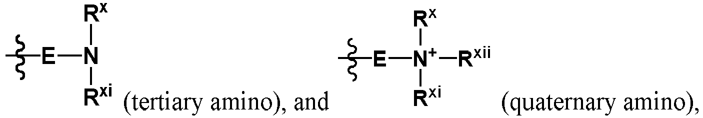 wherein, E is absent, or E is substituted or unsubstituted alkyl, substituted or unsubstituted alkenyl, substituted or unsubstituted alkynyl, substituted or unsubstituted aralkyl, substituted or unsubstituted aryl, substituted or unsubstituted heteroaryl, a substituted or unsubstituted polyaryl, a substituted or unsubstituted polyheteroaryl, substituted or unsubstituted heterocyclyl, wherein independently of E, R^{x}, R^{xi}, and R^{xii} each independently represent a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. The term "quaternary amino" also includes the groups where the nitrogen, R^{x}, R^{xi}, and R^{xii} with the N⁺ to which they are attached complete a heterocyclyl or heteroaryl having from 3 to 14 atoms in the ring structure. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The terms "amide" or "amido" are used interchangeably, refer to both "unsubstituted amido" and "substituted amido" and are represented by the general formula: 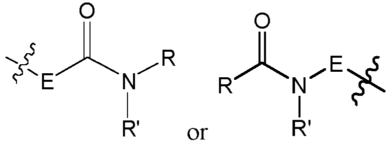 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted aryl, substituted or unsubstituted heteroaryl, a substituted or unsubstituted polyaryl, a substituted or unsubstituted polyheteroaryl, or a substituted or unsubstituted heterocyclyl, wherein independently of E, R and R' each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or Rand R' taken together with the N atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. In some forms, when E is oxygen, a carbamate is formed. It is understood by those of ordinary skill in the art, that the E groups listed above are divalent (e.g., methylene, ethane-1,2-diyl, ethene-1,2-diyl, 1,4-phenylene, cyclohexane-1,2-diyl).

"Carbonyl," as used herein, is art-recognized and includes such moieties as can be represented by the general formula: 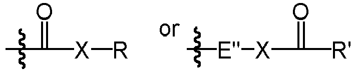 wherein X is a bond, or represents an oxygen or a sulfur, and R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R", or a pharmaceutical acceptable salt; E" is absent, or E" is a substituted or unsubstituted alkyl, a substituted or unsubstituted alkenyl, a substituted or unsubstituted alkynyl, a substituted or unsubstituted aralkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl; R' represents a hydrogen, a substituted or unsubstituted alkyl, a substituted or unsubstituted alkenyl, a substituted or unsubstituted alkynyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R"; R" represents a hydroxyl group, a substituted orunsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, and combinations thereof. It is understood by those of ordinary skill in the art, that the E" groups listed above are divalent (e.g., methylene, ethane-1,2-diyl, ethene-1,2-diyl, 1,4-phenylene, cyclohexane-1,2-diyl). Where X is oxygen and R is defined as above, the moiety is also referred to as a carboxyl group. When X is oxygen and R is hydrogen, the formula represents a "carboxylic acid." Where X is oxygen and R' is hydrogen, the formula represents a "formate." Where X is oxygen and R or R' is not hydrogen, the formula represents an "ester." In general, where the oxygen atom of the above formula is replaced by a sulfur atom, the formula represents a "thiocarbonyl" group. Where X is sulfur and R or R' is not hydrogen, the formula represents a "thioester." Where X is sulfur and R is hydrogen, the formula represents a "thiocarboxylic acid." Where X is sulfur and R' is hydrogen, the formula represents a "thioformate." Where X is a bond and R is not hydrogen, the above formula represents a "ketone." Where X is a bond and R is hydrogen, the above formula represents an "aldehyde."

The term "phosphanyl" is represented by the formula 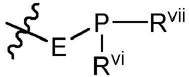 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted cycloalkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R^{vi} and R^{vii} each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi} and R^{vii} taken together with the P atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphonium" is represented by the formula 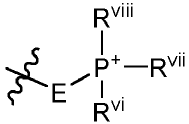 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted cycloalkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R^{vi}, R^{vii}, and R^{viii} each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi}, R^{vii}, and R^{viii} taken together with the P⁺ atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphonyl" is represented by the formula 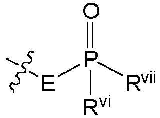 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, oxygen, alkoxy, aroxy, or substituted alkoxy or substituted aroxy, wherein, independently of E, R^{vi} and R^{vii} are independently a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi} and R^{vii} taken together with the P atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphoryl" defines a phosphonyl in which E is absent, oxygen, alkoxy, aroxy, substituted alkoxy or substituted aroxy, as defined above, and independently of E, R^{vi} and R^{vii} are independently hydroxyl, alkoxy, aroxy, substituted alkoxy or substituted aroxy, as defined above. When E is oxygen, the phosphoryl cannot be attached to another chemical species, such as to form an oxygen-oxygen bond, or other unstable bonds, as understood by one of ordinary skill in the art. When E, R^{vi} and R^{vii} are substituted, the substituents include, but are not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfinyl" is represented by the formula 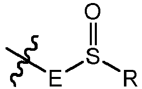 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted heterocyclyl, a substituted or unsubstituted polyaryl, a substituted or unsubstituted polyheteroaryl, wherein independently of E, R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a silyl, a thiol, an amido, an amino, or -(CH₂)ₘ-R‴, or E and R taken together with the S atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonyl" is represented by the formula 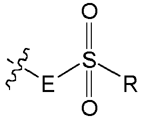 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted heterocyclyl, a substituted or unsubstituted polyaryl, a substituted or unsubstituted polyheteroaryl, wherein independently of E, R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R‴, or E and R taken together with the S atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonic acid" refers to a sulfonyl, as defined above, wherein R is hydroxyl, and E is absent, or E is substituted or unsubstituted cycloalkyl, substituted or unsubstituted heterocyclyl, substituted or unsubstituted alkylaryl, substituted or unsubstituted arylalkyl, substituted or unsubstituted aryl, a substituted or unsubstituted polyaryl, a substituted or unsubstituted polyheteroaryl, or substituted or unsubstituted heteroaryl. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfate" refers to a sulfonyl, as defined above, wherein E is absent, oxygen, alkoxy, aroxy, substituted alkoxy or substituted aroxy, as defined above, and R is independently hydroxyl, alkoxy, aroxy, substituted alkoxy or substituted aroxy, as defined above. When E is oxygen, the sulfate cannot be attached to another chemical species, such as to form an oxygen-oxygen bond, or other unstable bonds, as understood by one of ordinary skill in the art. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonate" refers to a sulfonyl, as defined above, wherein E is oxygen, alkoxy, aroxy, substituted alkoxy or substituted aroxy, as defined above, and R is independently hydrogen, substituted or unsubstituted alkyl, substituted or unsubstituted alkenyl, substituted or unsubstituted alkynyl, substituted or unsubstituted amino, substituted or unsubstituted cycloalkyl, substituted or unsubstituted heterocyclyl, substituted or unsubstituted aralkyl, substituted or unsubstituted alkylaryl, substituted or unsubstituted arylalkyl, substituted or unsubstituted aryl, substituted or unsubstituted heteroaryl, a substituted or unsubstituted polyaryl, a substituted or unsubstituted polyheteroaryl, -(CH₂)ₘ-R‴, R‴ represents a hydroxy group, substituted or unsubstituted carbonyl group, an aryl, a cycloalkyl ring, a cycloalkenyl ring, a heterocycle, an amido, an amino, or a polycycle; and m is zero or an integer ranging from 1 to 8. When E is oxygen, sulfonate cannot be attached to another chemical species, such as to form an oxygen-oxygen bond, or other unstable bonds, as understood by one of ordinary skill in the art. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfamoyl" refers to a sulfonamide or sulfonamide represented by the formula 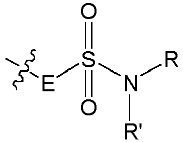 wherein E is absent, or E is substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted cycloalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R and R' each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R‴, or Rand R' taken together with the N atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "silyl group" as used herein is represented by the formula -SiRR'R," where R, R', and R" can be, independently, a hydrogen, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl (e.g. a substituted or unsubstituted alkylaryl, a substituted or unsubstituted arylalkyl, etc.), a substituted or unsubstituted polyaryl, a substituted or unsubstituted polyheteroaryl, a substituted or unsubstituted carbonyl, a phosphonium, a phosphanyl, a phosphonyl, a sulfinyl, a thiol, an amido, an amino, an alkoxy, or an oxo, described above. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The terms "thiol" are used interchangeably and are represented by -SR, where R can be a hydrogen, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl (e.g. a substituted or unsubstituted alkylaryl, a substituted or unsubstituted arylalkyl, etc.), a substituted or unsubstituted polyaryl, a substituted or unsubstituted polyheteroaryl, a substituted or unsubstituted carbonyl, a phosphonium, a phosphanyl, an amido, an amino, an alkoxy, an oxo, a phosphonyl, a sulfinyl, or a silyl, described above. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The disclosed compounds and substituent groups, can, independently, possess two or more of the groups listed above. For example, if the compound or substituent group is a straight chain alkyl group, one of the hydrogen atoms of the alkyl group can be substituted with a hydroxyl group, an alkoxy group, etc. Depending upon the groups that are selected, a first group can be incorporated within second group or, alternatively, the first group can be pendant (i.e., attached) to the second group. For example, with the phrase "an alkyl group comprising an ester group," the ester group can be incorporated within the backbone of the alkyl group. Alternatively, the ester can be attached to the backbone of the alkyl group. The nature of the group(s) that is (are) selected will determine if the first group is embedded or attached to the second group.

The compounds and substituents can be substituted, independently, with the substituents described above in the definition of "substituted."

The numerical ranges disclose individually each possible number that such a range could reasonably encompass, as well as any sub-ranges and combinations of sub-ranges encompassed therein. For example, in a given range carbon range of C₃-C₉, the range also discloses C₃, C₄, C₅, C₆, C₇, C₈, and C₉, as well as any subrange between these numbers (for example, C₄ -C₆), and any possible combination of ranges possible between these values. In yet another example, a given temperature range may be from about 25 °C to 30 °C, where the range also discloses temperatures that can be selected independently from about 25, 26, 27, 28, 29, and 30 °C, as well as any range between these numbers (for example, 26 to 28 °C), and any possible combination of ranges between these values.

Use of the term "about" is intended to describe values either above or below the stated value, which the term "about" modifies, to be within a range of approximately +/-10%. When the term "about" is used before a range of numbers (i.e., about 1-5) or before a series of numbers (i.e., about 1, 2, 3, 4, etc.) it is intended to modify both ends of the range of numbers and/or each of the numbers recited in the entire series, unless specified otherwise.

The disclosed compounds and substituent groups, can, independently, possess two or more of the groups listed above. For example, if the compound or substituent group is a straight chain alkyl group, one of the hydrogen atoms of the alkyl group can be substituted with a hydroxyl group, an alkoxy group, etc. Depending upon the groups that are selected, a first group can be incorporated within second group or, alternatively, the first group can be pendant (*i*.*e*., attached) to the second group. For example, with the phrase "an alkyl group comprising an ester group," the ester group can be incorporated within the backbone of the alkyl group. Alternatively, the ester can be attached to the backbone of the alkyl group. The nature of the group(s) that is (are) selected will determine if the first group is embedded or attached to the second group.

The compounds and substituents can be substituted with, independently, with the substituents described above in the definition of "substituted."

### II. Methods for Asymmetric cis-Dihydroxylation of Styrenes and Conjugated Dienes

Methods for asymmetric *cis*-dihydroxylation ("AD") of an alkene, such as styrenes or derivatives thereof and conjugated dienes, to produce *cis*-diols or tetraols of the alkene, have been developed. The methods described herein use an iron-based catalyst, such as one or more Fe(II) complexes, as the catalyst, and an oxidant, such as hydrogen peroxide. In some forms, AD of styrenes or derivatives thereof and/or conjugated dienes, catalyzed with the Fe(II) complexes, have high yield (e.g., a yield ≥ 14% or ≥ 30%, such as a yield from 14% to about 99% or from 30% to about 99%) and/or high enantioselectivity (e.g., an enantiometric excess (*"ee"*) ≥ 60%, such as an *ee* from about 68% to about 99.9% or >99.9%).

In some forms, AD of styrenes or derivatives thereof, catalyzed with the Fe(II) complexes, have a yield ≥ 30% (such as a yield from 30% to about 99%) and/or an enantioselectivity >_ 60% (such as an *ee* from about 68% to about 99.9% or >99.9%).

In some forms, AD of conjugated dienes, catalyzed with the Fe(II) complexes, have a yield ≥ 14% or ≥ 30% (such as a yield from 14% to about 99% or from 30% to about 99%) and/or an enantioselectivity >_ 60% (such as an *ee* from about 68% to about 99.9% or >99.9%).

The methods described herein have at least the following advantages: (1) *cis*-diols of alkenes can be produced with high yield and/or high enantioselectivity; (2) the use of biocompatible iron-based catalysts is environmentally friendly and cost efficient; and (3) the waste generated from the reaction is non-toxic. In some forms, the methods described herein can produce *cis*-diols of alkenes with high yield and high enantioselectivity

The methods described herein overcome the disadvantages associated with conventional AD reactions of alkenes using Os-based catalysts (e.g., the commercial products AD-mix-α/β developed by Sharpless and co-workers), which are expensive due to the low abundance of osmium metal on earth and have environmental and health concerns due to the production of overstoichiometric amounts of toxic wastes. For example, in AD reactions using osmium-based catalysts, K₃Fe(CN)₆ is typically added as a terminal oxidant, which produces an equivalent waste of K₄Fe(CN)₆. Over-stoichiometric amount of K₂CO₃ is also needed as an additive in osmium-based catalysis. The methods described herein can use hydrogen peroxide as the terminal oxidant, which is a green oxidant and is converted to the two/four hydroxyl (OH) groups onto product molecules with 100% atom efficiency and do not need any additive.

The method generally includes: (i) maintaining a reaction mixture at a temperature for a period of time sufficient to form a product. The reaction mixture contains an alkene (such as a styrene, a derivative of styrene, or a conjugated diene), one or more iron-based catalyst(s), an oxidant, and a solvent. The product contains a *cis*-diol (e.g., when the alkene is a styrene, a derivative of styrene, or a conjugated diene) or tetraol (e.g., when the alkene is conjugated diene).

The reaction mixture can be formed by dissolving an alkene (such as a styrene, a derivative of styrene, or a conjugated diene) and iron-based catalyst(s) in the solvent prior to reaction. Suitable solvents for forming the reaction mixture can dissolve the alkene and the iron-based catalyst(s). For example, a styrene, styrene derivative, or conjugated diene has a solubility of at least 0.01 M and the iron-based catalyst(s) has a solubility of at least 0.0015 M in the solvent. An exemplary solvent suitable for forming the reaction mixture include, but are not limited to an alcohol, such as a C₁-C₆ alcohol, such as methanol, ethanol, propanol, etc.

Optionally, the method also includes, prior to step (i), adding the oxidant (e.g., a hydrogen peroxide solution) into a pre-mixture that contains the alkene (such as a styrene, a derivative of styrene, or a conjugated diene), the one or more iron-based catalyst(s), and the solvent, to form the reaction mixture. The oxidant may be added in the form of a solution formed by dissolving the oxidant in an oxidant solvent. The oxidant solvent may be the same or different from the solvent forming the pre-mixture. For example the solvent forming the pre-mixture and the oxidant solvent forming the oxidant solution are the same, such as an alcohol, for example, methanol.

The oxidant can be added into the pre-mixture using any suitable technique. For example, the oxidant, in the form of a solution, is added into the pre-mixture using a syringe pump over a suitable time period (such as a time period ranging from 20 mins to 1 hour, e.g., about 30 mins), at room temperature. For example, the oxidant, in the form of a solution, is added into the pre-mixture directly at room temperature.

The specific technique for adding the oxidant may be selected based on the desired product. For example, when the desired product is a cis-diol, the oxidant, in the form of a solution, may be added into the pre-mixture using a syringe pump over a suitable time period (such as a time period ranging from 20 mins to 1 hour, e.g., about 30 mins), at room temperature. For example, when the desired product is a tetraol, the oxidant, in the form of a solution, may be added into the pre-mixture directly at room temperature.

Optionally, the method further includes stirring the reaction mixture prior to and/or during step (i); and/or purifying the product, optionally by column chromatography, subsequent to step (i).

### A. Maintaining a reaction mixture at a temperature for a period of time sufficient to form a product

Generally, a reaction mixture containing an alkene (such as a styrene, a derivative of styrene, or a conjugated diene), one or more iron-based catalyst(s), and an oxidant disclosed herein in a suitable solvent is maintained at a suitable temperature for a period of time sufficient to form a product containing *cis-*diols or tetraols of the alkenes.

In some forms, the specific reaction conditions, such as the amount of the oxidant, the amount of the catalyst(s), the specific catalyst, the reaction temperature, and reaction time period used in step (i) can be selected based on the reactant and the desired product.

In some forms, the methods disclosed herein convert a styrene or a styrene derivative to a *cis*-diol via AD reaction. In some forms, the methods disclosed herein convert a conjugated diene to a *cis*-diol via AD reaction. In some forms, the methods disclosed herein convert a conjugated diene to a tetraol via AD reaction.

The alkenes (i.e., substrates), such as styrene, styrene derivatives, and conjugated dienes described herein can be synthesized using methods known in the art of organic chemical synthesis or are commercially available. For example, the target substrates can be synthesized by reacting one or more corresponding reactants in a suitable solvent. The reaction solution containing the one or more corresponding reactants can be stirred at a suitable temperature for a suitable time to form a product containing the target substrates. The product containing the target substrates can be purified to isolate the target substrates.

### 1. Iron-based Catalysts

The AD reactions of alkenes described herein are catalyzed by an iron-based catalyst, optionally more than one iron-based catalysts. For example, the AD reactions of a styrene, a styrene derivative, or a conjugated diene in the reaction mixture are catalyzed by one or more Fe(II) complex(es). In some forms, the Fe(II) complex or each Fe(II) complex of the two or more catalysts in the reaction mixture can have the structure of Formula IX: 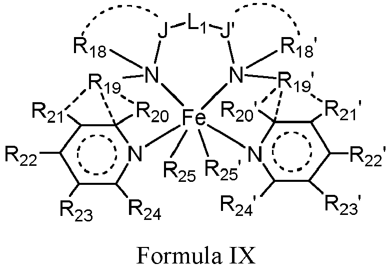 where: (a) J and J' can be independently a bond (single, double, or triple), a substituted or unsubstituted alkyl, a substituted or unsubstituted alkenyl, a substituted or unsubstituted cyclic group, a substituted or unsubstituted aryl, or a substituted or unsubstituted polyaryl; (b) L₁ can be a bond or 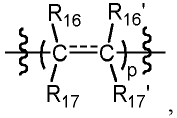 R₁₆, R₁₆', R₁₇, and R₁₇' can be independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' together and/or R₁₇ and R₁₇' together, with the carbon atoms to which they are attached, can form a substituted or unsubstituted aryl, a substituted or unsubstituted polyaryl, or a substituted or unsubstituted cyclic group, and p can be an integer from 1 to 10, from 1 to 8, from 1 to 6, from 1 to 4, from 1 to 3, or 1 or 2; (c) R₁₈-R₂₄ and R₁₈'-R₂₄' can be independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl; (d) R₂₅ and R₂₅' can be independently a leaving group; (e) ------ can be absent or a bond (single, double, or triple); and (f) the substituents can be independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.

In some forms of Formula IX, R₁₉, R₂₀, and R₂₁ together and/or R₁₉', R₂₀', and R₂₁' together, with the carbon atoms to which they are attached, can be form a substituted or unsubstituted aryl, a substituted or unsubstituted polyaryl, or a substituted or unsubstituted cyclic group.

In some forms, the Fe(II) complex or each Fe(II) complex of the two or more catalysts in the reaction mixture can have the structure of Formula X, XI, or XIII: 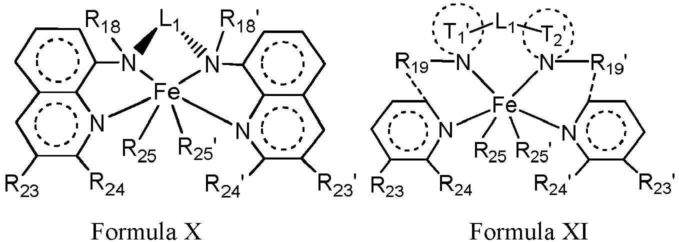 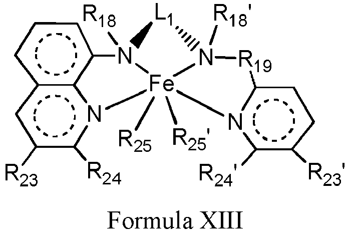 where: (a) L₁ can be a bond or 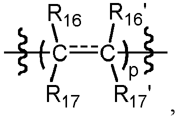 R₁₆, R₁₆', R₁₇, and R₁₇' can be independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' together and/or R₁₇ and R₁₇' together, with the carbon atoms to which they are attached, can form a substituted or unsubstituted aryl, a substituted or unsubstituted polyaryl, or a substituted or unsubstituted cyclic group, and p is an integer from 1 to 10, from 1 to 8, from 1 to 6, from 1 to 4, from 1 to 3, or 1 or 2; (b) T₁' and T₂' can be independently a substituted or unsubstituted heteroaryl, a substituted or unsubstituted heteropolyaryl, or a substituted or unsubstituted heterocyclic group; (c) R₁₈, R₂₃, R₂₄, R₁₈', R₂₃', and R₂₄' can be independently a hydrogen, a substituted or unsubstituted alkyl, a substituted or unsubstituted alkenyl, a substituted or unsubstituted aryl, a substituted or unsubstituted alkylaryl, a substituted or unsubstituted polyaryl; (d) R₁₉ and R₁₉' can be independently a substituted or unsubstituted alkyl, a substituted or unsubstituted alkenyl, a substituted or unsubstituted aryl, a substituted or unsubstituted alkylaryl, a substituted or unsubstituted polyaryl; (e) R₂₅ and R₂₅' can be independently a leaving group; and (f) the substituents can be as defined above for Formula IX.

In some forms of Formulae IX-XI and XIII, L₁ can be a bond, 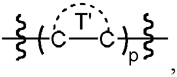 or 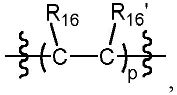 each occurrence of T' can be a substituted or unsubstituted monocyclic group, a substituted or unsubstituted polycyclic group, a substituted or unsubstituted aryl, or a substituted or unsubstituted polyaryl, each occurrence of R₁₆ and R₁₆' can be independently a substituted or unsubstituted phenyl, and p is an integer from 1 to 10, from 1 to 8, from 1 to 6, from 1 to 4, from 1 to 3, or 1 or 2.

In some forms of Formulae IX-XI and XIII, L₁ can be 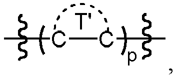 each occurrence of T' is a substituted or unsubstituted monocyclic group (e.g. an unsubstituted monocycloalkyl, such as an unsubstituted C₃-C₆ monocycloalkyl) or a substituted or unsubstituted polycyclic group (e.g. an unsubstituted polycycloalkyl) and p is an integer from 1 to 3, or 1 or 2, such as 1.

In some forms of Formulae IX-XI and XIII, R₂₅ and R₂₅' can be independently a triflate, a tosylate, a mesylate, a halide, a nitrate, a phosphate, a thioether, an amino, a carboxylate, a phenoxide, an alkoxyl, or an amido, such as a triflate.

In some forms of Formulae IX-XI and XIII, R₂₃ and R₂₃' can be hydrogen, and R₂₄ and R₂₄' can be an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₂ alkyl, such as a methyl.

In some forms of Formulae IX-XI and XIII, R₁₈ can be an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₂ alkyl, such as a methyl.

In some forms of Formulae IX-XI and XIII, R₁₈' can be an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 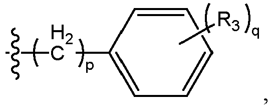 p and q are independently an integer from 0 to 5, and each occurrence of R₃ is independently hydrogen or an unsubstituted C₁-C₁₀ alkyl (e.g., a methyl, an ethyl, an isopropyl, a tert-butyl, etc.), or two neighboring R₃ together with the carbon to which they are attached form an unsubstituted aryl or unsubstituted polyaryl.

In some forms, the Fe(II) complex for catalyzing the AD of alkenes can have the structure of Formula XII, XIV, or XV. 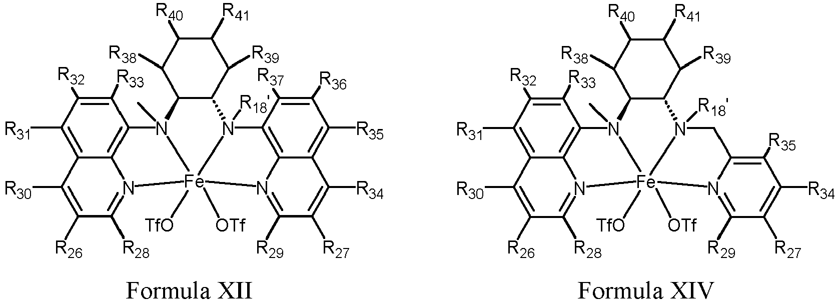 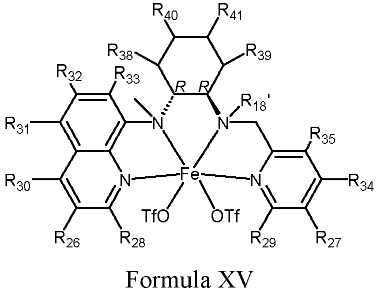 where: (a) R₂₆ and R₂₇ can be independently hydrogen, a substituted or unsubstituted cyclic group, a substituted or unsubstituted aryl, or a substituted or unsubstituted polyaryl; (b) R₂₈-R₄₁ can be independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl; (c) R₁₈' can be an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 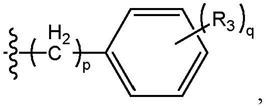 p and q are independently an integer from 0 to 5, and each occurrence of R₃ is independently hydrogen or an unsubstituted C₁-C₁₀ alkyl (e.g., a methyl, an ethyl, an isopropyl, a tert-butyl, etc.), or two neighboring R3 together with the carbon to which they are attached form an unsubstituted aryl or unsubstituted polyaryl; and (d) the substituents can be independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof. In some forms of Formula XII, R₂₆ and R₂₇ are not hydrogen.

In some forms of Formula XII, R₂₆ and R₂₇ can be independently a substituted or unsubstituted aryl, or a substituted or unsubstituted polyaryl; (b) R₂₈-R₄₁ can be independently a hydrogen, a substituted or unsubstituted alkyl, or a substituted or unsubstituted alkenyl; and (c) the substituents can be independently a substituted or unsubstituted alkyl, a substituted or unsubstituted alkenyl, or a substituted or unsubstituted alkynyl, or a combination thereof.

In some forms, for any of Formulae XIV and XV, R₂₆ and R₂₇ can be independently hydrogen, a substituted or unsubstituted alkyl, or a substituted or unsubstituted aryl; (b) R₂₈ and R₂₉ can be independently a hydrogen, a substituted or unsubstituted alkyl, or a substituted or unsubstituted alkenyl; (c) R₃₀-R₄₁ can be independently hydrogen or a substituted or unsubstituted alkyl; and (d) the substituents can be independently a substituted or unsubstituted alkyl, a substituted or unsubstituted alkenyl, or a substituted or unsubstituted alkynyl, or a combination thereof.

In some forms of Formula XII, (a) R₂₆ and R₂₇ can be independently a substituted or unsubstituted aryl; (b) R₂₈-R₄₁ can be independently a hydrogen, an unsubstituted alkyl, or an unsubstituted alkenyl; and (c) the substituents can be independently an unsubstituted alkyl, an unsubstituted alkenyl, an unsubstituted alkynyl, or a combination thereof.

In some forms of Formula XII, (a) R₂₆ and R₂₇ can be independently an unsubstituted aryl and (b) R₂₈-R₄₁ can be independently a hydrogen, an unsubstituted alkyl, or an unsubstituted alkenyl.

In some forms, for any of Formulae XIV and XV, (a) R₂₆ and R₂₇ can be hydrogen, (b) R₂₈ and R₂₉ can be independently a substituted or unsubstituted alkyl, such as an unsubstituted C₁-C₈ alkyl, and (c) R₃₀-R₄₀ can be hydrogen.

In some forms, the Fe(II) complex or each Fe(II) complex of the two or more catalysts in the reaction mixture can have the structure of: 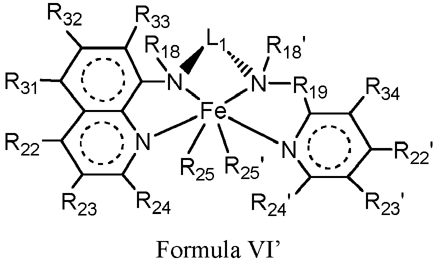 where: (a) L₁ can be a bond or 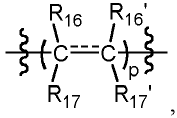 R₁₆, R₁₆', R₁₇, and R₁₇' can be independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' and/or R₁₇ and R₁₇' form a substituted or unsubstituted aryl, a substituted or unsubstituted polyaryl, or a substituted or unsubstituted cyclic group, and p can be an integer from 1 to 10, from 1 to 8, form 1 to 6, from 1 to 5, from 1 to 4, from 1 to 3, or 1 or 2; (b) R₁₈, R₂₃, R₂₄, R₁₈', R₂₃', and R₂₄' can be independently a hydrogen, a substituted or unsubstituted alkyl, a substituted or unsubstituted alkenyl, a substituted or unsubstituted aryl, a substituted or unsubstituted alkylaryl, or a substituted or unsubstituted polyaryl; (c) R₁₉ can be a substituted or unsubstituted alkyl, a substituted or unsubstituted alkenyl, a substituted or unsubstituted aryl, a substituted or unsubstituted alkylaryl, or a substituted or unsubstituted polyaryl; (d) R₂₂, R₂₂', and R₃₁-R₃₄ can be independently a hydrogen, a substituted or unsubstituted alkyl, a halogen, an amino, or an alkoxyl; (e) R₂₅ and R₂₅' can be independently a leaving group; and (f) the substituents can be independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.

In some forms, R₂₂, R₂₂', and R₃₁-R₃₄ can be independently a hydrogen, -NRR', a methoxy, or an ethoxy, where R and R' can be independently hydrogen or an unsubstituted alkyl, such as an unsubstituted C₁-C₆ alkyl, for example, methyl. In some forms, R₃₂-R₃₄ can be hydrogen. In some forms, the compound can have a structure of Formula VI' above.

In some forms, L₁ can be 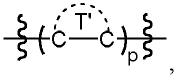 each occurrence of T' can be a substituted or unsubstituted monocyclic group or a substituted or unsubstituted polycyclic group and p is an integer from 1 to 3, or 1 or 2. In some forms, T' can be a substituted or unsubstituted C₃-C₆ monocycloalkyl group, and optionally wherein the substituent(s), when present, can be independently an unsubstituted phenyl or a phenyl substituted with one or more unsubstituted alkyl(s), such as one or more unsubstituted C₁-C₆ alkyls.

In some forms, L₁ can be 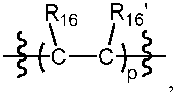 each occurrence of R₁₆ and R₁₆' can be independently a substituted or unsubstituted phenyl, and p can be an integer from 1 to 3, or 1 or 2. In some forms, R₁₆ and R₁₆' can be unsubstituted phenyl.

In some forms, R₂₅ and R₂₅' can be independently a triflate, a tosylate, a mesylate, a halide, a nitrate, a phosphate, a thioether, an amino, a carboxylate, a phenoxide, an alkoxyl, or an amido. In some forms, R₂₅ and R₂₅' can be triflate.

In some forms, R₁₈ can be an unsubstituted C₁-C₆ alkyl, such as methyl or ethyl.

In some forms, R₁₈' can be an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 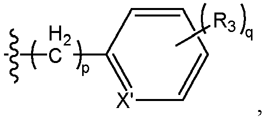 X' can be carbon or nitrogen; p and q can be independently an integer from 0 to 5, and each occurrence of R₃ can be independently hydrogen, a halide (e.g., fluoride, chloride, bromide), an unsubstituted C₁-C₁₀ alkyl (e.g., a methyl, an ethyl, an isopropyl, a tert-butyl, etc.), or an unsubstituted phenyl, or two neighboring R₃ together with the carbon to which they are attached form an unsubstituted aryl or unsubstituted polyaryl. In some forms, R₁₈' can be methyl or 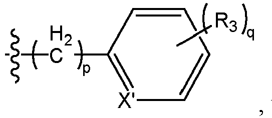 wherein X' can be nitrogen or carbon, p can be 1 or 2, such as 1, q can be an integer from 1 to 4, each occurrence of R₃ can be independently hydrogen, a halide, an unsubstituted C₁-C₆ alkyl, an unsubstituted phenyl, or two neighboring R₃ together with the carbon to which they are attached form an unsubstituted aryl.

In some forms, R₁₉ can be a substituted or unsubstituted alkylene, optionally a substituted or unsubstituted methylene or ethylene group. In some forms, R₁₉ can be an unsubstituted methylene group or a methylene group substituted with an unsubstituted C₁-C₆ alkyl, an unsubstituted phenyl, or an unsubstituted C₃-C₆ monocycloalkyl group.

In some forms, R₂₃ and R₂₃' can be hydrogen, and R₂₄ and R₂₄' can be independently an unsubstituted C₁-C₁₀ alkyl, an unsubstituted C₁-C₈ alkyl, an unsubstituted C₁-C₆ alkyl, an unsubstituted C₁-C₄ alkyl, or an unsubstituted C₁-C₂ alkyl, such as a methyl. In some forms, R₂₄ and R₂₄' can be methyl.

In some forms, R₂₄ and R₂₄' can be methyl; and R₂₂, R₂₂', R₂₃, R₂₃', and R₃₁-R₃₄ can be hydrogen.

In some forms, R₂₂ and R₂₂' can be independently hydrogen, -NRR', a methoxy, or an ethoxy, where R and R' can be independently hydrogen or an unsubstituted alkyl, such as an unsubstituted C₁-C₆ alkyl, for example, methyl; R₂₄ and R₂₄' can be methyl; and R₂₃, R₂₃', and R₃₁-R₃₄ can be hydrogen.

In some forms, R₃₁ and R₂₂' can be independently hydrogen, -NRR', a methoxy, or an ethoxy, where R and R' can be independently hydrogen or an unsubstituted alkyl, such as an unsubstituted C₁-C₆ alkyl, for example, methyl; R₂₄ and R₂₄' can be methyl; and R₂₂, R₂₃, R₂₃', and R₃₂-R₃₄ can be hydrogen.

Exemplary iron-based catalysts suitable for use in the AD reaction in step (i) are presented below.

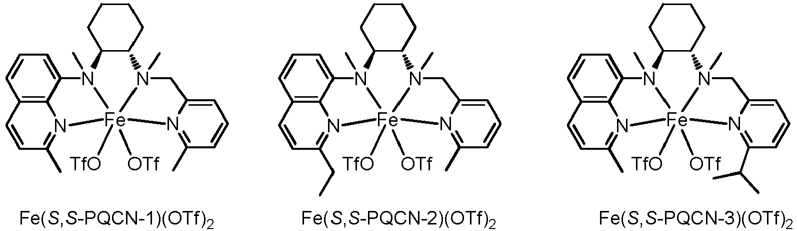

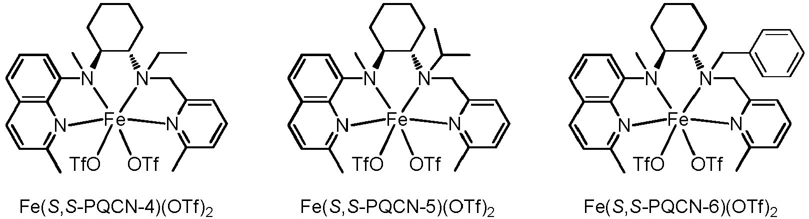

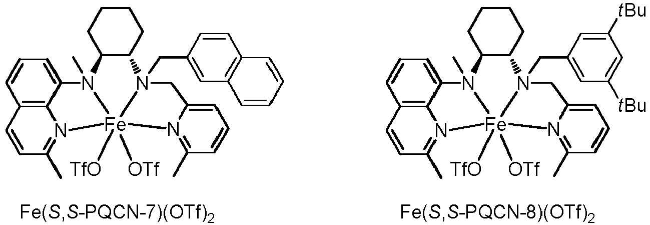

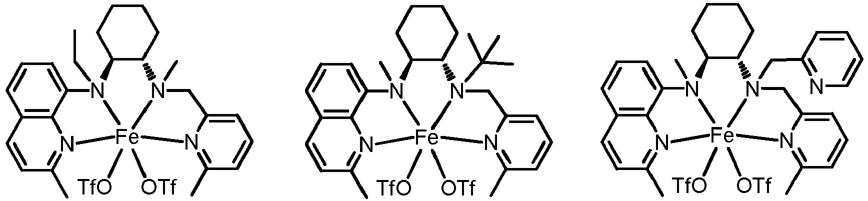

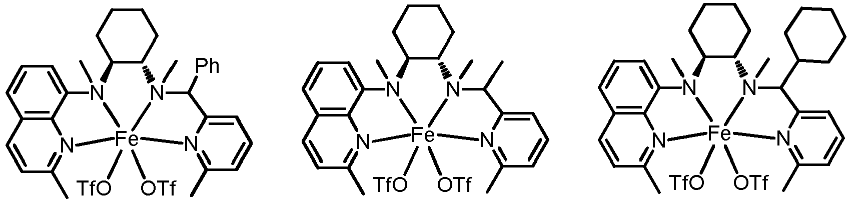

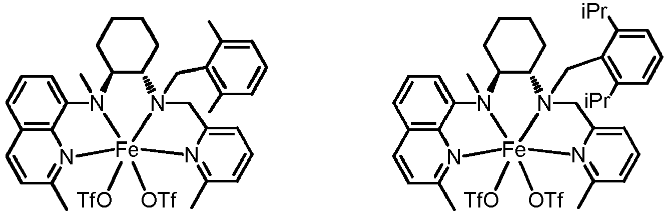

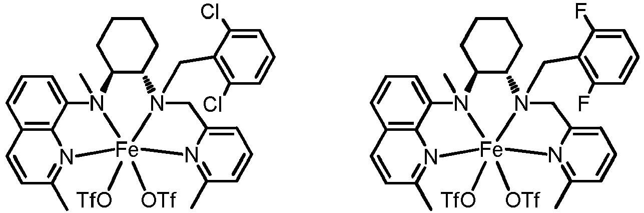

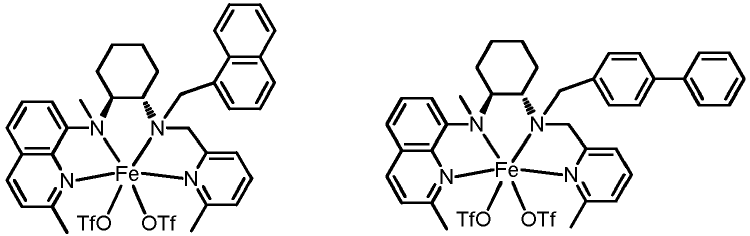

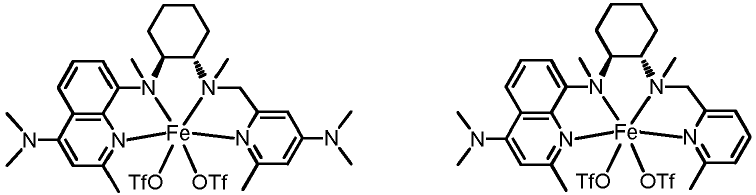

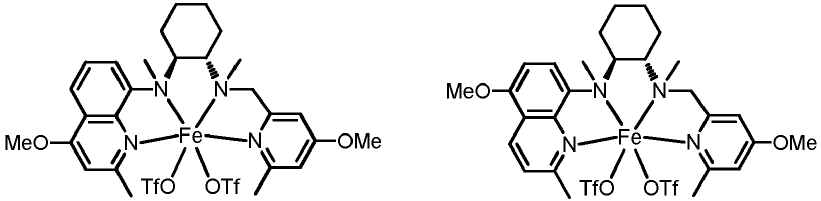

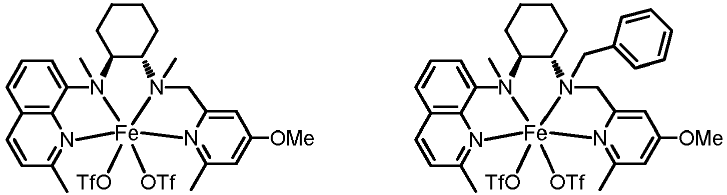

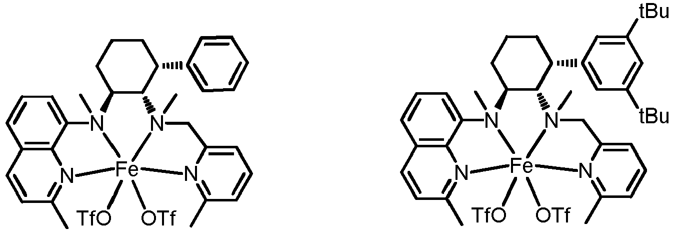

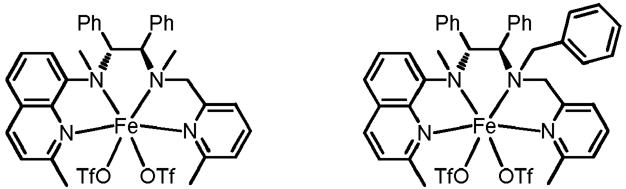

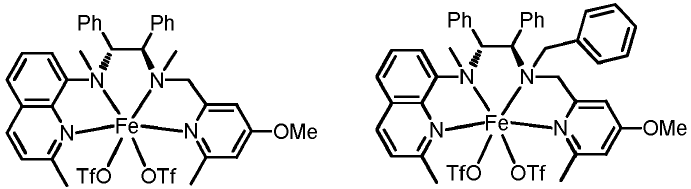

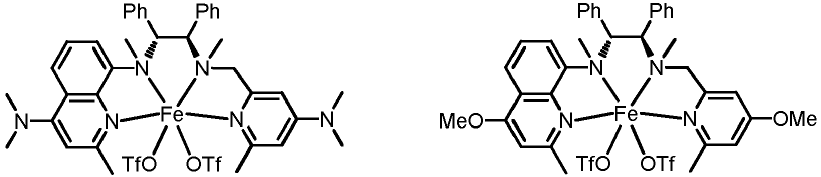

In some forms, the iron-based catalysts suitable for use in the AD reaction in step (i) are any one of those presented below.

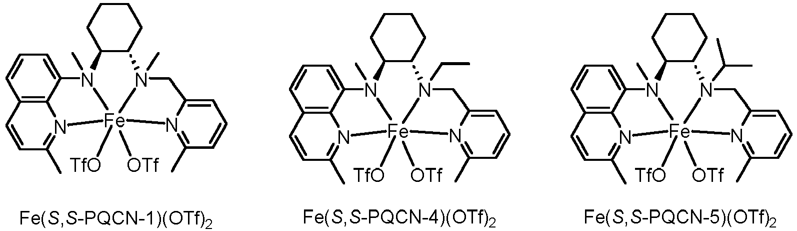

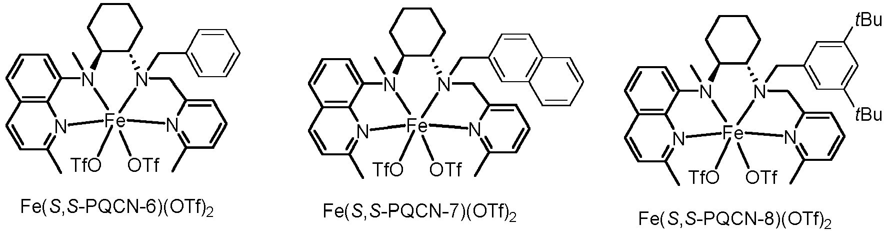

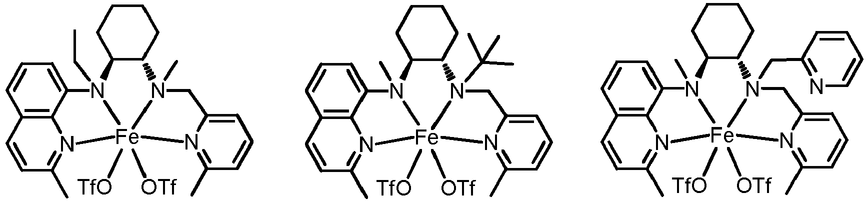

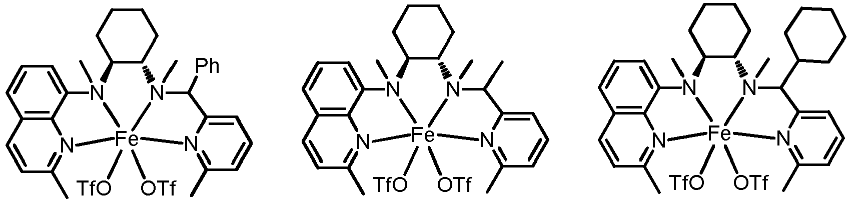

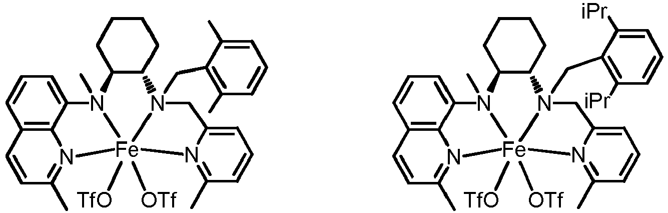

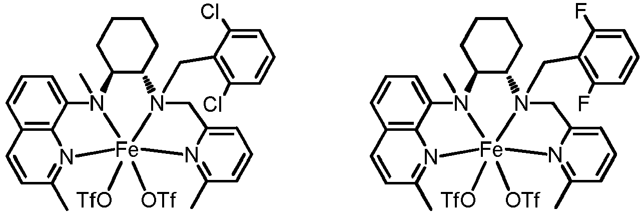

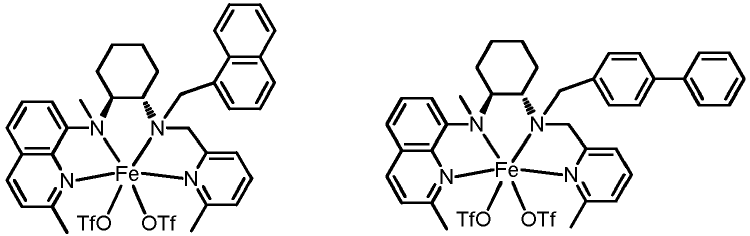

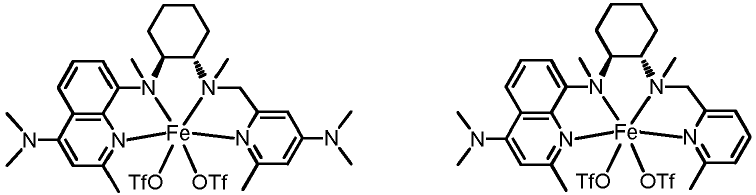

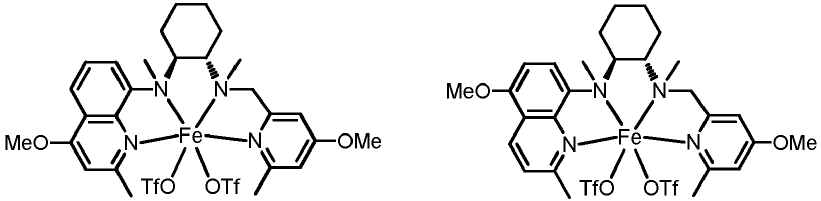

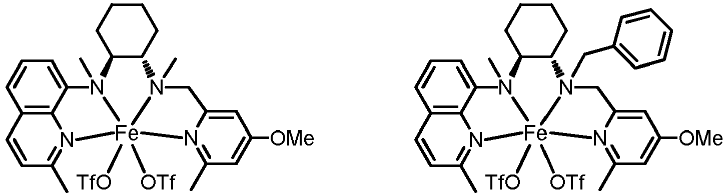

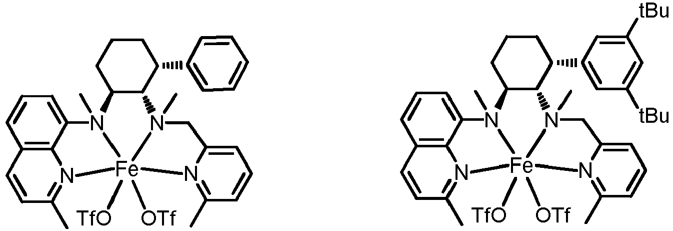

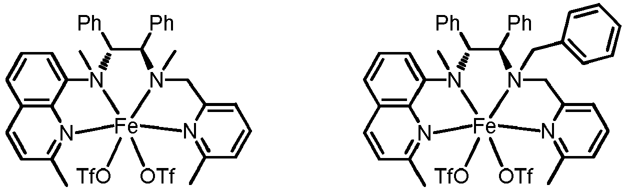

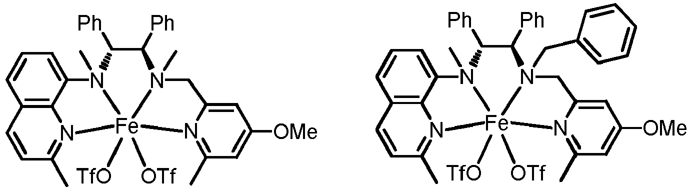

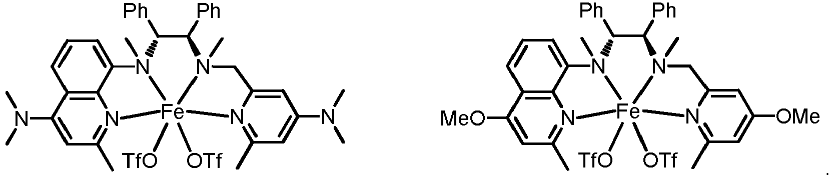

In some forms, the iron-based catalyst used in the AD reaction in step (i) is:

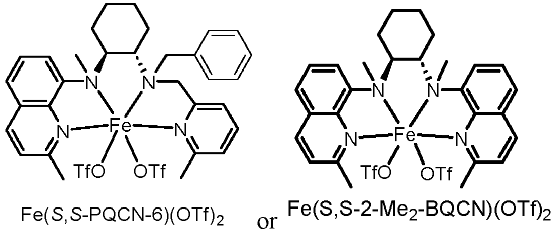

Generally, the total amount of the one or more iron-based catalyst(s) in the reaction mixture is up to 20 mol%, such as ranging from 0.1 mol% to 20 mol%. The total amount of the one or more catalyst(s) in the reaction mixture can be calculated using the formula: mol% of the iron-based catalyst(s) = [(the sum of the nos. of moles of the one or more iron-based catalyst(s))/(the sum of the nos. of moles of the alkene(s)]×100%. In some forms, one can choose the amount of the iron-based catalyst in the AD reaction to achieve a specific product.

For example, when the disclosed methods convert a styrene or a styrene derivative to a cis-diol via AD reaction, the total amount of the one or more iron-based catalyst(s) in the reaction mixture is up to 10 mol%, up to 8 mol%, up to 6 mol%, up to 5 mol%, up to 3 mol%, at least 0.1 mol%, at least 0.5 mol%, in a range from about 0.1 mol% to about 10 mol%, from about 0.1 mol% to about 8 mol%, from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%.

For example, when the disclosed methods convert a conjugated diene to a cis-diol via AD reaction, the total amount of the one or more iron-based catalyst(s) in the reaction mixture is up to 6 mol%, up to 5 mol%, up to 3 mol%, at least 0.1 mol%, at least 0.5 mol%, in a range from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%.

In some forms, when the disclosed methods convert a conjugated diene to a tetraol via AD reaction, > 6 mol%, at least 7 mol%, at least 8 mol%, in a range from about 6.5 mol% to about 20 mol%, from about 7 mol% to about 20 mol%, from about 7 mol% to about 15 mol%, from about 6.5 mol% to about 15 mol%, from about 8 mol% to about 20 mol%, from about 8 mol% to about 15 mol%, from about 7 mol% to about 12 mol%, or from about 8 mol% to about 12 mol%, such as about 10 mol%.

In some forms, one can choose the iron-based catalyst in the AD reaction to achieve a desired stereochemistry. For example, when the disclosed methods convert a conjugated diene to a *cis*-diol or tetraol via AD reaction catalyzed by Fe(*S*,*S*-PQCN-6)(OTf)₂ described above, the cis-diol or tetraol of the conjugated diene can have a (2*S*,3*R*) or (*2S,3R,4S,5R*) stereochemistry. For example, when the disclosed methods convert a conjugated diene to a *cis*-diol or tetraol via AD reaction catalyzed by Fe(*S*,*S*-2-Me₂-BQCN)(OTf)₂ described above, the cis-diol or tetraol of the conjugated diene can have a (*2R,3S*) or (2*R*,3*S,4R,5S*) stereochemistry.

The iron-based catalysts and the ligands forming the iron-based catalysts described herein can be synthesized using methods known in the art of organic chemical synthesis. For example, the target Fe(II) complexes can be synthesized by reacting a corresponding ligand, optionally more than one corresponding ligand, with an iron precursor in a suitable solvent. Exemplary solvents include organic solvents, such as acetonitrile. The corresponding ligand(s) can be prepared using methods known in the art, such as those described in the Examples. The reaction solution containing the one or more corresponding ligands and the iron precursor can be stirred at a suitable temperature, such as room temperature and optionally under an inert gas atmosphere, such as nitrogen atmosphere, for a suitable time to form a product containing the target iron-based catalysts. The product containing the target iron-based catalysts can be purified and optionally recrystallized to isolate the target iron-based catalysts.

### 2. Conversion of Styrene or Derivative Thereof to cis- Diol

In some forms, the methods disclosed herein convert a styrene or a styrene derivative to a *cis*-diol via AD reaction. The reaction mixture contains a styrene or a derivative thereof, optionally more than one styrene or derivative thereof, serving as the substrate in the catalytic reaction. Following step (i), a product containing one or more *cis-*diols converted from the styrene or styrene derivative in the reaction mixture, is formed. In some forms, the product can also contain unreacted substrates, i.e., unreacted styrene or styrene derivative, and/or the iron-based catalysts.

In these forms, the reaction mixture is typically maintained at a temperature below 0°C (e.g., about -30°C) for a period of time in a range from about 20 minutes to about 3 hours, from about 20 minutes to about 2 hours, or from about 20 minutes to 1 hour (e.g., about 30 mins). In these forms, the total mole amount of the oxidant in the reaction mixture is typically in a range from about 1-time to about 10-time, from about 1.5-time to about 6-time, or from about 1-time to about 5-time of the total mole amount of the styrene or styrene derivative.

### a. Styrene Substrate and cis-Diol Product

In some forms, the styrene or styrene derivative in the reaction mixture can have the structure of Formula I and the *cis*-diol converted therefrom has a structure of Formula I': 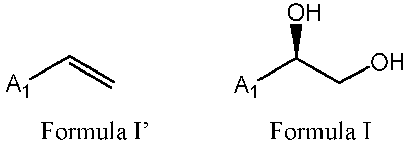 where A₁ can be a substituted aryl, an unsubstituted aryl, a substituted polyaryl, an unsubstituted polyaryl, a substituted heteroaryl, an unsubstituted heteroaryl, a substituted heteropolyaryl, or an unsubstituted heteropolyaryl.

In some forms, the styrene or styrene derivative in the reaction mixture can have the structure of Formula II and the *cis*-diol converted therefrom has a structure of Formula II': 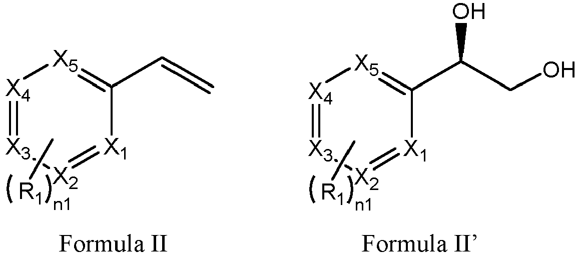 where: (a) X₁-X₅ can be independently carbon, nitrogen, oxygen, or sulfur; (b) n1 can be an integer from 0 to 5; (c) each occurrence of R₁ can be independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₁ groups together with the carbon atoms to which they are attached form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group; and (d) the substituents, when present, can be independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

In some forms, each occurrence of R₁ can be independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, - C(=O)OR₄, or 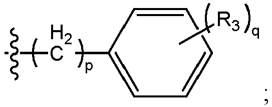 or two neighboring R₁ groups together with the carbon atoms to which they are attached can form a substituted aryl or an unsubstituted aryl, and the substituents are independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 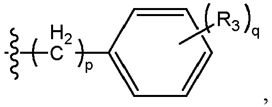 where p and q can be independently an integer from 0 to 5, and R₂-R₄ can be independently hydrogen, an unsubstituted C₁-C₁₀ alkyl, halide, or -O-(C₁-C₁₀ alkyl).

In some forms, n1 is not 0, and at least one R₁, at least two R₁, or at least three R₁ can be electron-withdrawing group(s). In some forms, n1 is 1, and R₁ can be an electron-withdrawing group at the meta-position or para-position. In some forms, n1 is 1 and R₁ can be 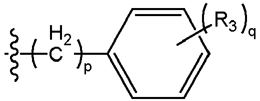 at the ortho-position, p and q can be independently an integer from 0 to 5, and each occurrence of R₃ can be independently hydrogen, an unsubstituted C₁-C₁₀ alkyl, halide, or -O-(C₁-C₁₀ alkyl).

In some forms, X₁ can be nitrogen and X₂-X₅ can be carbon.

In some forms, two neighboring R₁ groups together with the carbon atoms to which they are attached form a substituted aryl or an unsubstituted aryl, and the substituents can be independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 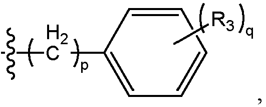 p and q can be independently an integer from 0 to 5, and R₂-R₄ can be independently hydrogen, an unsubstituted C₁-C₁₀ alkyl, halide, or -O-(C₁-C₁₀ alkyl).

Exemplary *cis*-diols converted from a styrene or styrene derivative are presented below.

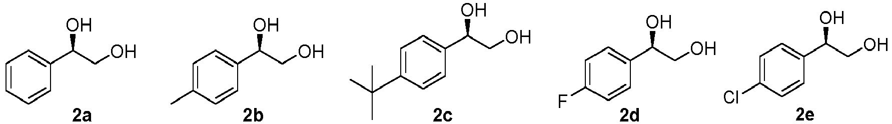

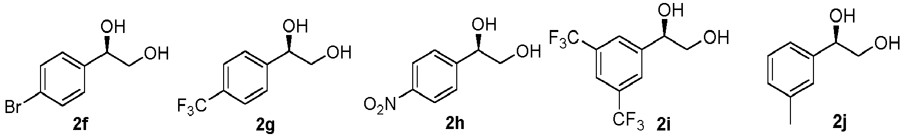

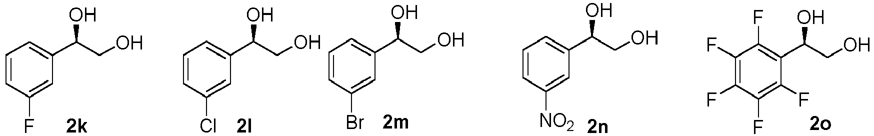

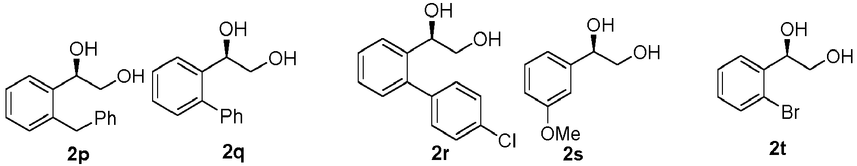

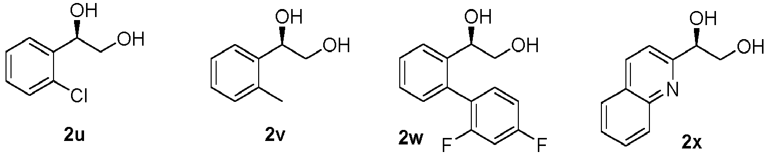

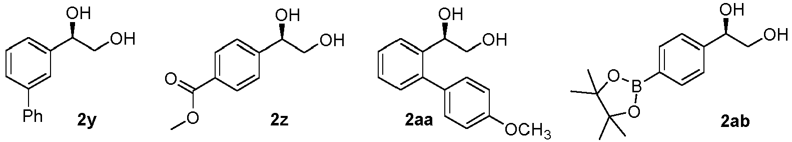

### b. Characterization

The disclosed methods for asymmetric *cis*-dihydroxylation of styrene and styrene derivatives can be characterized by *cis*-diol yield and enantiomeric excess (*"ee"*) of *cis-*diol in the product.

Generally, the *cis*-diol(s) in the product formed from the AD reaction of styrene or styrene derivative(s) using the disclosed methods can have a total yield of at least 40%, at least 50%, in a range from about 40% to about 99%, from about 50% to about 99%, from about 60% to about 99%, from about 70% to about 99%, or from about 80% to about 99%. The yield of *cis*-diol(s) in the product can be calculated using the formula: *cis*-diol yield = (experimentally obtained total no. of mole of *cis*-diol(s))/(theoretical total no. of mole of *cis*-diol(s))×100%. The experimentally obtained total no. of mole of the *cis*-diol(s) can be determined using known methods, such as using NMR (e.g. ¹H NMR, ¹⁹F NMR, and/or ³¹P NMR) spectroscopy with an internal standard of known quantity, such as using a known quantity of PhTMS as the internal standard.

Generally, the *cis*-diol or each cis-diol of two or more *cis*-diols in the product formed from the AD reactions of styrene or styrene derivative(s) using the disclosed methods can have an enantiomeric excess ("*ee*") of at least 60%, at least 70%, at least 75%, at least 80%, up to 100%, in a range from about 60% to 100%, from about 60% to 100%, from about 70% to 100%, from about 80% to 100%, from about 90% to 100%, or from about 95% to 100%, from about 60% to 99%, from about 70% to 99%, from about 80% to 99%, from about 90% to 99%, or from about 95% to 99%. The *ee* of *cis*-diol(s) in the product can be calculated using the formula: *ee* = [(moles of enantiomer - moles of another enantiomer)/total moles of both enantiomers] × 100%. The *ee* of each *cis*-diol can be determined using known methods, such as using chiral HPLC or a polarimeter. In some forms, the *ee* of each *cis*-diol can be determined using chiral HPLC.

In some forms, the above-described yield and *ee* of *cis*-diol(s) in the product formed from the AD reaction of styrene and styrene derivatives are obtained using the disclosed methods catalyzed with the Fe(II) complex of Formula VI'.

Specific exemplary *cis*-diols and their corresponding yields and *ee* values obtained using styrene or styrene derivatives as substrate are described in the Examples below.

### 2. Conversion of conjugated diene to cis-Diol or Tetraol

In some forms, the methods disclosed herein convert a conjugated diene to a *cis-*diol or tetraol via AD reaction. The reaction mixture contains a conjugated diene, optionally more than conjugated diene, serving as the substrate in the catalytic reaction. Following step (i), a product containing one or more *cis*-diols or tetraols converted from the conjugated diene in the reaction mixture, is formed. In some forms, the product can also contain unreacted substrates, i.e., unreacted conjugated diene, and/or the iron-based catalysts.

Generally, the total mole amount of the oxidant in the reaction mixture is typically in a range from about 1-time to about 10-time, from about 1.5-time to about 6-time, or from about 1-time to about 5-time of the total mole amount of the conjugated diene.

In some forms, the amount of the catalyst, the reaction temperature, and reaction time period can be selected such that the product from the AD reaction of a conjugated diene is a *cis*-diol or a tetraol.

For example, when the desired product from the AD reaction of the conjugated diene is a *cis*-diol, the total amount of the one or more iron-based catalyst(s) in the reaction mixture can be up to 6 mol%, up to 5 mol%, up to 3 mol%, at least 0.1 mol%, at least 0.5 mol%, in a range from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%; and the reaction mixture can be maintained at room temperature (i.e., from about 20 °C to about 22 °C, at 1atm) for a period of time in a range from about 20 minutes to about 3 hours, from about 20 minutes to about 2 hours, from about 20 minutes to 1 hour, or from about 20 mins to about 50 mins (e.g., about 30 mins). Further, prior to the AD reaction, the oxidant can be added into a pre-mixture formed by the conjugated diene, iron-based catalyst(s), and solvent using a syringe pump over a time period ranging from 20 mins to 1 hour, such as about 30 mins, at room temperature.

For example, when the desired product from the AD reaction of the conjugated diene is a tetraol, the total amount of the one or more iron-based catalyst(s) in the reaction mixture is > 6 mol%, at least 7 mol%, at least 8 mol%, in a range from about 6.5 mol% to about 20 mol%, from about 7 mol% to about 20 mol%, from about 7 mol% to about 15 mol%, from about 6.5 mol% to about 15 mol%, from about 8 mol% to about 20 mol%, from about 8 mol% to about 15 mol%, from about 7 mol% to about 12 mol%, or from about 8 mol% to about 12 mol%, such as about 10 mol%; and the reaction mixture can be maintained at room temperatures for a period of time of at least 1 hour, in a range from 1 hour to about 5 hours, from 1 hour to about 4 hours, from 1 hour to about 3 hours, from 1 hour to about 2 hours (e.g., 1 hour). Further, prior to the AD reaction, the oxidant can be directly added into a pre-mixture formed by the conjugated diene, iron-based catalyst(s), and solvent, at room temperature.

### a. conjugated diene Substrate and cis-Diol Product

In some forms, the conjugated diene in the reaction mixture can have the structure of Formula VII and the *cis*-diol converted therefrom has a structure of Formula VII' or VII": 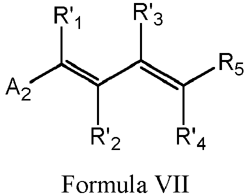 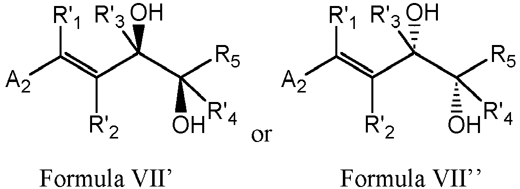 where: (a) A₂ can be a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl; (b) R₅ can be - C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl; (c) R'₁-R'₄ can be independently hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and (d) the substituents can be independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

As described above, in some forms, one may choose the iron-based catalyst in the AD reaction to achieve a desired stereochemistry. For example, when the disclosed methods convert a conjugated diene to a *cis*-diol via AD reaction catalyzed by Fe(*S*,*S-*PQCN-6)(OTf)₂ described above, the cis-diol of the conjugated diene can have a (2*S*,3*R*) stereochemistry, such as the structure of Formula VII'. For example, when the disclosed methods convert a conjugated diene to a *cis*-diol via AD reaction catalyzed by Fe(*S*,*S*-2-Me₂-BQCN)(OTf)₂ described above, the cis-diol of the conjugated diene can have a (2*R*,3*S*) stereochemistry, such as the structure of Formula VII".

### b. Characterization of cis-Diol product

The disclosed methods for asymmetric *cis*-dihydroxylation of conjugated diene to form *cis-*diol can be characterized by *cis*-diol yield and enantiomeric excess (*"ee"*) of *cis-*diol in the product.

Generally, the *cis*-diol(s) in the product formed from the AD reaction of conjugated diene using the disclosed methods can have a total yield of at least 10%, at least 14%, at least 30%, at least 40%, in a range from about 14% to about 99%, from about 30% to about 99%, from about 40% to about 99%, from about 50% to about 99%, from about 60% to about 99%, or from about 70% to about 99%. The yield of *cis*-diol(s) in the product can be calculated using the method described above for styrene.

Generally, the *cis*-diol or each *cis*-diol of two or more *cis*-diols in the product formed from the AD reaction of conjugated diene using the disclosed methods can have an enantiomeric excess ("*ee*") of at least 80%, at least 85%, up to 100%, in a range from about 80% to 100%, from about 85% to 100%, from about 90% to 100%, from about 80% to 99.9%, from about 85% to 99.9%, or from about 90% to 99.9%, from about 80% to 99.5%, from about 85% to 99.5%, from about 90% to 99.5%, from about 80% to 99%, or from about 85% to 99%. The *ee* of *cis*-diol(s) in the product can be calculated using the method described above for styrene, such as using chiral HPLC.

In some forms, the above-described yield and *ee* of *cis*-diol(s) in the product formed from the AD reaction of conjugated diene is obtained using the disclosed methods catalyzed with the Fe(II) complex of Formula VI'.

Specific exemplary *cis*-diols and their corresponding yields and *ee* values obtained using conjugated diene as substrates are described in the Examples below.

### c. Conjugated Diene Substrate and Tetraol Product

In some forms, the conjugated diene in the reaction mixture can have the structure of Formula VII and the tetraol converted therefrom has a structure of Formula VIII' or VIII": 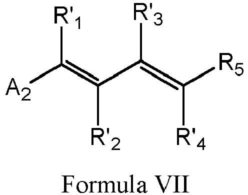 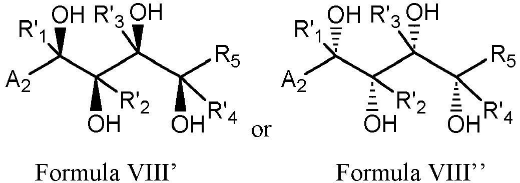 where: (a) A₂ can be a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl; (b) R₅ can be - C(=O)OR₆, R₆ can be hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl; (c) R'₁-R'₄ can be independently a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and (d) the substituents can be independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

As described above, in some forms, one may choose the iron-based catalyst in the AD reaction to achieve a desired stereochemistry. For example, when the disclosed methods convert a conjugated diene to a tetraol via AD reaction catalyzed by Fe(*S*,*S-*PQCN-6)(OTf)₂ described above, the tetraol formed from the conjugated diene can have a (2*S*,3*R*,*4R,5S*) stereochemistry, such as the structure of Formula VIII'. For example, when the disclosed methods convert a conjugated diene to a tetraol via AD reaction catalyzed by Fe(*S*,*S*-2-Me₂-BQCN)(OTf)₂ described above, the tetraol formed from the conjugated diene can have a (2*R*,3*S,4S,5R*) stereochemistry, such as the structure of Formula VIII".

### d. Characterization of Tetraol Product

The disclosed methods for asymmetric *cis*-dihydroxylation of conjugated diene to form tetraol can be characterized by *cis*-diol yield and enantiomeric excess ("*ee*") of tetraol in the product.

Generally, the tetraol(s) in the product formed from the AD reaction of conjugated diene using the disclosed methods can have a total yield of at least 10%, at least 14%, at least 30%, at least 40%, in a range from about 14% to about 99%, from about 30% to about 99%, from about 40% to about 99%, from about 50% to about 99%, from about 60% to about 99%, or from about 70% to about 99%. The yield of tetraol(s) in the product can be calculated using the method described above for styrene.

Generally, the tetraol or each tetraol of two or more tetraols in the product formed from the AD reaction of conjugated diene using the disclosed methods can have an enantiomeric excess ("*ee*") of at least 90%, at least 95%, at least 99.9%, >99.9%, up to 100%, in a range from about 90% to 100%, from about 95% to 100%, from about 90% to 99.9%, from about 95% to 99.9%, or from about 99.9% to 100%. The *ee* of tetraol(s) in the product can be calculated using the method described above for styrene, such as using chiral HPLC.

In some forms, the above-described yield and *ee* of tetraol(s) in the product formed from the AD reaction of conjugated diene are obtained using the disclosed methods catalyzed with the Fe(II) complex of Formula VI'.

Specific exemplary *cis*-diols and their corresponding yields and *ee* values obtained using conjugated diene as substrates are described in the Examples below.

### e. Structure of Conjugated Diene, cis-Diol, and Tetraol

In some forms, the conjugated diene in the reaction mixture can have the structure of Formula VII, the corresponding *cis*-diol in the product can have the structure of Formula VII' or VII", and the corresponding tetraol in the product can have a structure of Formula VIII' or VIII", as described above and shown below.

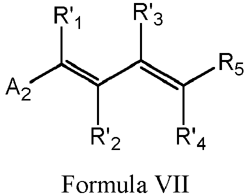

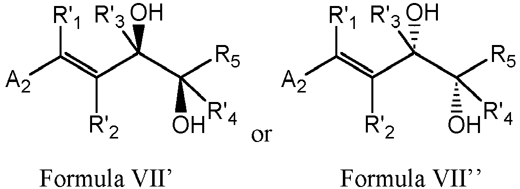

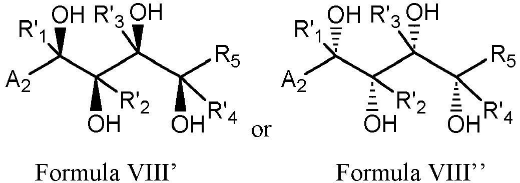

In some forms, A₂ can be: 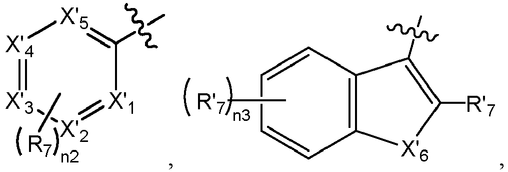 or - C(=O)OR₁₄, where: (a) X'₁-X'₆ can be independently carbon, nitrogen, oxygen, or sulfur; (b) n2 and n3 can be independently an integer from 0 to 5; (c) each occurrence of R₇ and R'₇ can be independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₇ groups or two neighboring R'₇ groups together with the carbon atoms to which they are attached can form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group; (d) R₁₄ can be hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl; and (e) the substituents can be independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

In some forms, each occurrence of R₇ and R'₇ can be independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, - C(=O)OR₄, or 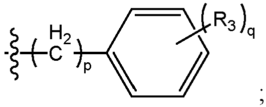 or two neighboring R₇ groups or two neighboring R'₇ together with the carbon atoms to which they are attached can form a substituted aryl or an unsubstituted aryl, and the substituents are independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, - C(=O)OR₄, or 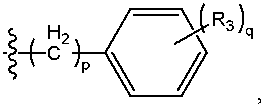 where p and q can be independently an integer from 0 to 5, and R₂-R₄ can be independently hydrogen, an unsubstituted C₁-C₁₀ alkyl, halide, or -O-(C₁-C₁₀ alkyl).

In some forms, each occurrence of R₇ and R'₇ can be independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), or a cyano.

In some forms, X'₂-X'₅ can be carbon, X'₁ can be carbon or nitrogen, and X'₆ can be carbon, nitrogen, oxygen, or sulfur.

In some forms, R₆ can be an unsubstituted C₁-C₁₀ alkyl or 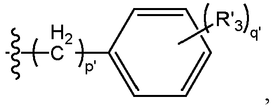 p' and q' can be independently an integer from 0 to 5, and each occurrence of R'₃ can be independently hydrogen, an unsubstituted C₁-C₁₀ alkyl, halide, or -O-(C₁-C₁₀ alkyl). In some forms, R₆ can be an unsubstituted C₁-C₈ alkyl, an unsubstituted C₁-C₆ alkyl, an unsubstituted C₁-C₄ alkyl, or 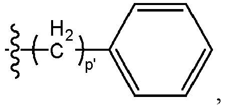 and p' can be an integer from 0 to 3, from 0 to 2, or 0 or 1.

In some forms, R'₁-R'₄ can be independently hydrogen, an unsubstituted C₁-C₁₀ alkyl, an unsubstituted aryl, or an unsubstituted alkylaryl.

In some forms, R₁₄ can be an unsubstituted C₁-C₈ alkyl, an unsubstituted C₁-C₆ alkyl, an unsubstituted C₁-C₄ alkyl, or 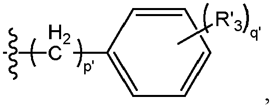 p' and q' can be independently an integer from 0 to 5, and each occurrence of R'₃ can be independently hydrogen, an unsubstituted C₁-C₁₀ alkyl, halide, or -O-(C₁-C₁₀ alkyl). In some forms, R₁₄ can be an unsubstituted C₁-C₄ alkyl or 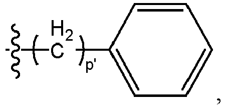 and p' can be an integer from 0 to 3, from 0 to 2, or 0 or 1.

For any of the formulae described herein, the alkyl (when present) can be a linear alkyl, a branched alkyl, or a cyclic alkyl (either monocyclic or polycyclic). The terms "cyclic alkyl" and "cycloalkyl" are used interchangeably herein. Exemplary alkyl include a linear C₁-C₃₀ alkyl, a branched C₄-C₃₀ alkyl, a cyclic C₃-C₃₀ alkyl, a linear C₁-C₂₀ alkyl, a branched C₄-C₂₀ alkyl, a cyclic C₃-C₂₀ alkyl, a linear C₁-C₁₀ alkyl, a branched C₄-C₁₀ alkyl, a cyclic C₃-C₁₀ alkyl, a linear C₁-C₆ alkyl, a branched C₄-C₆ alkyl, a cyclic C₃-C₆ alkyl, a linear C₁-C₄ alkyl, cyclic C₃-C₄ alkyl, such as a linear C₁-C₁₀, C₁-C₉, C₁-C₈, C₁-C₇, C₁-C₆, C₁-C₅, C₁-C₄, C₁-C₃, or C₁-C₂ alkyl group, a branched C₃-C₉, C₃-C₉, C₃-C₈, C₃-C₇, C₃-C₆, C₃-C₅, or C₃-C₄ alkyl group, or a cyclic C₃-C₉, C₃-C₉, C₃-C₈, C₃-C₇, C₃-C₆, C₃-C₅, or C₃-C₄ alkyl group. The cyclic alkyl can be a monocyclic or polycyclic alkyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkyl group.

For any of the formulae described herein, the alkenyl (when present) can be a linear alkenyl, a branched alkenyl, or a cyclic alkenyl (either monocyclic or polycyclic). The terms "cyclic alkenyl" and "cycloalkenyl" are used interchangeably herein. Exemplary alkenyl include a linear C₂-C₃₀ alkenyl, a branched C₄-C₃₀ alkenyl, a cyclic C₃-C₃₀ alkenyl, a linear C₂-C₂₀ alkenyl, a branched C₄-C₂₀ alkenyl, a cyclic C₃-C₂₀ alkenyl, a linear C₂-C₁₀ alkenyl, a branched C₄-C₁₀ alkenyl, a cyclic C₃-C₁₀ alkenyl, a linear C₂-C₆ alkenyl, a branched C₄-C₆ alkenyl, a cyclic C₃-C₆ alkenyl, a linear C₂-C₄ alkenyl, cyclic C₃-C₄ alkenyl, such as a linear C₂-C₁₀, C₂-C₉, C₂-C₈, C₂-C₇, C₂-C₆, C₂-C₅, C₂-C₄, C₂-C₃ alkenyl group, a branched C₃-C₉, C₃-C₉, C₃-C₈, C₃-C₇, C₃-C₆, C₃-C₅, C₃-C₄ alkenyl group, or a cyclic C₃-C₉, C₃-C₉, C₃-C₈, C₃-C₇, C₃-C₆, C₃-C₅, C₃-C₄ alkenyl group. The cyclic alkenyl can be a monocyclic or polycyclic alkenyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkenyl group.

For any of the formulae described herein, the alkynyl (when present) can be a linear alkynyl, a branched alkynyl, or a cyclic alkynyl (either monocyclic or polycyclic). The terms "cyclic alkynyl" and "cycloalkynyl" are used interchangeably herein. Exemplary alkynyl include a linear C₂-C₃₀ alkynyl, a branched C₄-C₃₀ alkynyl, a cyclic C₃-C₃₀ alkynyl, a linear C₂-C₂₀ alkynyl, a branched C₄-C₂₀ alkynyl, a cyclic C₃-C₂₀ alkynyl, a linear C₂-C₁₀ alkynyl, a branched C₄-C₁₀ alkynyl, a cyclic C₃-C₁₀ alkynyl, a linear C₂-C₆ alkynyl, a branched C₄-C₆ alkynyl, a cyclic C₃-C₆ alkynyl, a linear C₁-C₄ alkynyl, cyclic C₃-C₄ alkynyl, such as a linear C₂-C₁₀, C₂-C₉, C₂-C₈, C₂-C₇, C₂-C₆, C₂-C₅, C₂-C₄, C₂-C₃ alkynyl group, a branched C₃-C₉, C₃-C₉, C₃-C₈, C₃-C₇, C₃-C₆, C₃-C₅, C₃-C₄ alkynyl group, or a cyclic C₃-C₉, C₃-C₉, C₃-C₈, C₃-C₇, C₃-C₆, C₃-C₅, C₃-C₄ alkynyl group. The cyclic alkynyl can be a monocyclic or polycyclic alkynyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkynyl group.

Exemplary (2S,3R)-cis-diols converted from conjugated dienes are presented below.

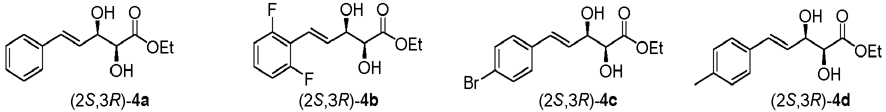

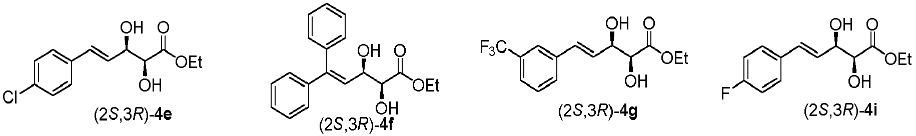

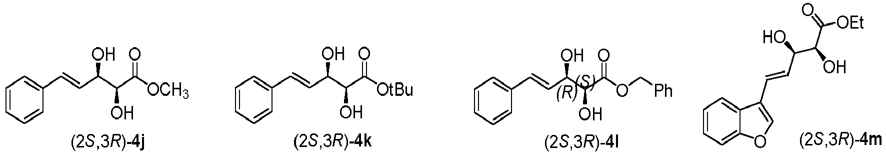

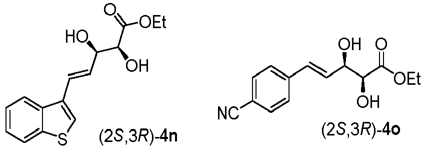

Exemplary (*2R*,*3S*)-*cis*-diols converted from conjugated dienes are presented below.

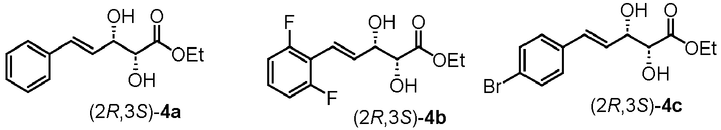

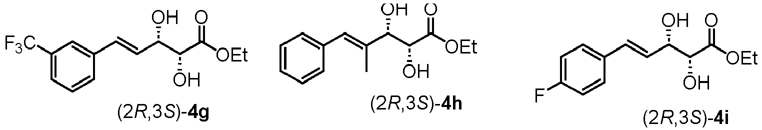

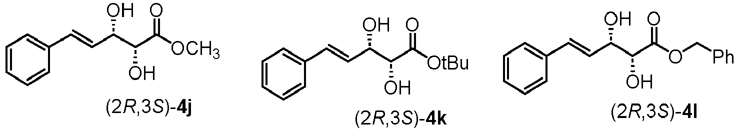

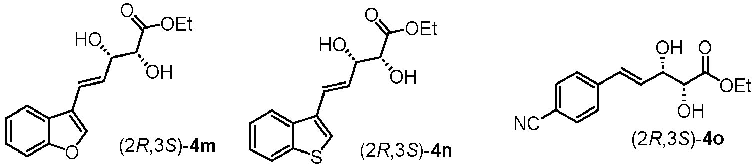

Exemplary (2S,3R,4R,5S)-tetraols converted from conjugated dienes are presented below.

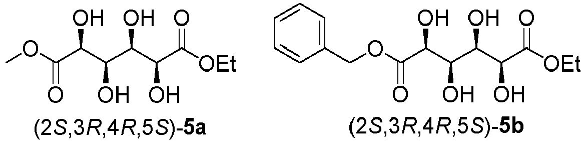

Exemplary (2R,3S,4S,5R)-tetraols converted from conjugated dienes are presented below.

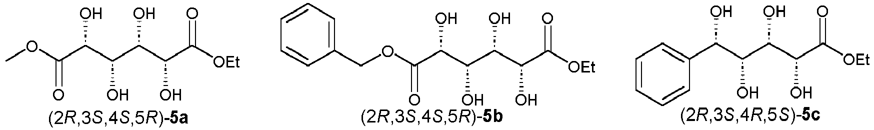

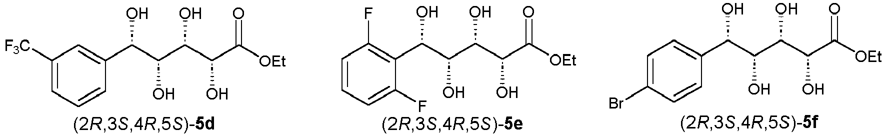

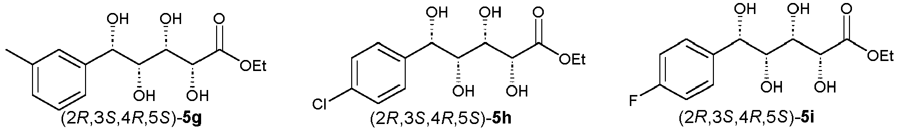

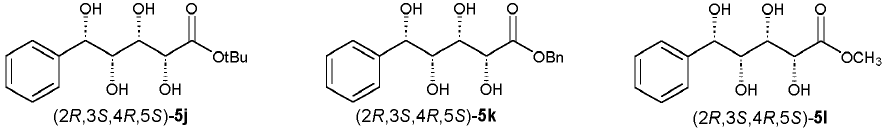

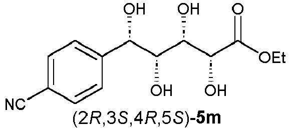

### B. Optional Steps

The disclosed methods can include one or more optional steps, such as adding an oxidant (e.g., hydrogen peroxide) into the reaction mixture prior to step (i); stirring the reaction mixture prior to and/or during step (i); and/or purifying the product, optionally by column chromatography, subsequent to step (i).

### 1. Adding an oxidant

Optionally, the disclosed method includes a step of adding an oxidant into a pre-mixture prior to step (i), maintaining the reaction mixture at a temperature for a period of time sufficient to form a product containing *cis*-diol(s) or tetraol(s). The pre-mixture contains the substrate, iron catalyst(s), and solvent for forming the reaction mixture.

An exemplary oxidant suitable for use in the disclosed methods is hydrogen peroxide. When the oxidant is added into the pre-mixture in the form of a solution, the solvent for forming the solution of oxidant can be the same or different from the solvent forming the reaction mixture. For example, a hydrogen peroxide solution is formed by mixing hydrogen peroxide with a solvent that is the same as the solvent forming the reaction mixture, such as an alcohol (e.g. methanol, ethanol, propanol, etc.), for example, methanol.

The oxidant, such as hydrogen peroxide, in the form of a solution, can be added into the reaction mixture by depressing a syringe filled with the oxidant in a solution form in a swift action (direct addition). Alternatively, the oxidant, such as a hydrogen peroxide, in the form of a solution, can be added into the reaction mixture by a syringe pump at a suitable flow rate over a period of time. For example, a hydrogen peroxide solution is added into the pre-mixture by a syringe pump over a time period ranging from 20 mins to 1 hour, such as about 30 mins, at room temperature.

Generally, following this step, the total mole amount of the oxidant, such as hydrogen peroxide, in the reaction mixture can be in a range from about 1-time to about 10-time, from about 1.5-time to about 6-time, or from about 2-time to about 5-time of the total mole amount of the substrate. For example, following addition of hydrogen peroxide into the pre-mixture (either by a swift injection or addition over a period of time), the total number of moles of the oxidant in the reaction mixture is in a range from about 1-time to about 10-time, from about 1.5-time to about 6-time, or from about 2-time to about 5-time, such as about 3-time, of the total mole amount of the substrate in the reaction mixture.

In some forms, the specific technique for adding the oxidant can be selected based on the desired product. For example, when the desired product is a cis-diol, the oxidant, in the form of a solution, may be added into the pre-mixture using a syringe pump over a suitable time period (such as a time period ranging from 20 mins to 1 hour, e.g., about 30 mins), at room temperature. For example, when the desired product is a tetraol, the oxidant, in the form of a solution, may be added into the pre-mixture directly at room temperature.

### 2. Stirring the reaction mixture

Optionally, the reaction mixture is under stirring prior to and/or during step (i), maintaining the reaction mixture at a temperature for a period of time sufficient to form a product containing cis-diol(s) or tetraol(s). For example, the reaction mixture is stirred at the reaction temperature during the entire reaction period to form the product containing *cis*-diol(s) or tetraol(s). Techniques for stirring the reaction mixture during reaction are known, such as by using a mechanical stirring bar, magnetic stirring beads, etc.

### 3. Purifying the product

Optionally, the disclosed method includes a step of purifying the product to remove impurities, such as unreacted substrates and/or the catalysts, in the product, and thereby obtain isolated *cis*-diol(s) or tetraol(s), subsequent to the AD reaction (i.e. step (i)). The product can be purified by known methods, such as using column chromatography on silica gel or recrystallization in a dichloromethane/hexane mixture.

The disclosed substrates, iron-based catalysts, products, and methods can be further understood through the following enumerated paragraphs.
1. An iron-based catalyst having the structure of: 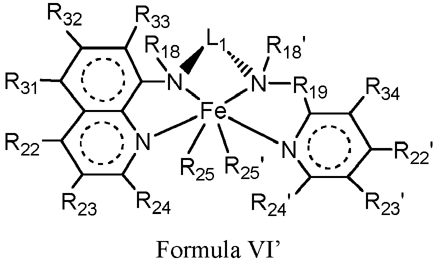 wherein:
   (a) L₁ is a bond or 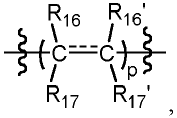 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' and/or R₁₇ and R₁₇' form a substituted or unsubstituted aryl, a substituted or unsubstituted polyaryl, or a substituted or unsubstituted cyclic group, and p is an integer from 1 to 10;
   (b) R₁₈, R₂₃, R₂₄, R₁₈', R₂₃', and R₂₄' are independently a hydrogen, a substituted or unsubstituted alkyl, a substituted or unsubstituted alkenyl, a substituted or unsubstituted aryl, a substituted or unsubstituted alkylaryl, or a substituted or unsubstituted polyaryl;
   (c) R₁₉ is a substituted or unsubstituted alkyl, a substituted or unsubstituted alkenyl, a substituted or unsubstituted aryl, a substituted or unsubstituted alkylaryl, or a substituted or unsubstituted polyaryl;
   (d) R₂₂, R₂₂', and R₃₁-R₃₄ are independently a hydrogen, a substituted or unsubstituted alkyl, a halogen, an amino, or an alkoxyl;
   (e) R₂₅ and R₂₅' are independently a leaving group; and
   (f) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.
2. The iron-based catalyst of paragraph 1, wherein R₂₂, R₂₂', and R₃₁-R₃₄ are independently a hydrogen, -NRR', a methoxy, or an ethoxy, wherein R and R' are independently hydrogen or an unsubstituted alkyl, such as an unsubstituted C₁-C₆ alkyl, for example, methyl.
3. The iron-based catalyst of paragraph 1 or 2, wherein R₃₂-R₃₄ are hydrogen.
4. The iron-based catalyst of paragraph 1, wherein the compound has a structure of: 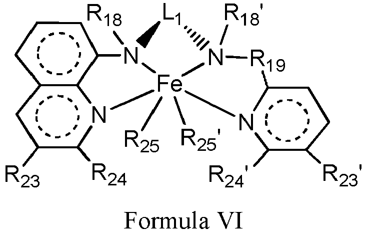
5. The iron-based catalyst of any one of paragraphs 1-4, wherein L₁ is 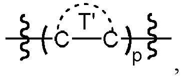 each occurrence of T' is a substituted or unsubstituted monocyclic group or a substituted or unsubstituted polycyclic group and p is an integer from 1 to 3, or 1 or 2.
6. The iron-based catalyst of paragraph 5, wherein T' is a substituted or unsubstituted C₃-C₆ monocycloalkyl group, and optionally wherein the substituent(s), when present, are independently an unsubstituted phenyl or a phenyl substituted with one or more unsubstituted alkyl(s), such as one or more unsubstituted C₁-C₆ alkyls.
7. The iron-based catalyst of any one of paragraphs 1-4, wherein L₁ is 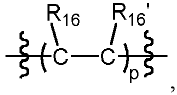 each occurrence of R₁₆ and R₁₆' are independently a substituted or unsubstituted phenyl, and p is an integer from 1 to 3, or 1 or 2.
8. The iron-based catalyst of paragraph 7, wherein R₁₆ and R₁₆' are unsubstituted phenyl.
9. The iron-based catalyst of any one of paragraphs 1-8, wherein R₂₅ and R₂₅' are independently a triflate, a tosylate, a mesylate, a halide, a nitrate, a phosphate, a thioether, an amino, a carboxylate, a phenoxide, an alkoxyl, or an amido.
10. The iron-based catalyst of any one of paragraphs 1-9, wherein R₂₅ and R₂₅' are triflate.
11. The iron-based catalyst of any one of paragraphs 1-10, wherein R₁₈ is an unsubstituted C₁-C₆ alkyl, such as methyl or ethyl.
12. The iron-based catalyst of any one of paragraphs 1-11, wherein R₁₈' is an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 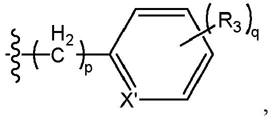 X' is carbon or nitrogen; p and q are independently an integer from 0 to 5, and each occurrence of R₃ is independently hydrogen, a halide (e.g., fluoride, chloride, bromide), an unsubstituted C₁-C₁₀ alkyl (e.g., a methyl, an ethyl, an isopropyl, a tert-butyl, etc.), or an unsubstituted phenyl, or two neighboring R₃ together with the carbon to which they are attached form an unsubstituted aryl or unsubstituted polyaryl.
13. The iron-based catalyst of any one of paragraphs 1-12, wherein R₁₈' is methyl or 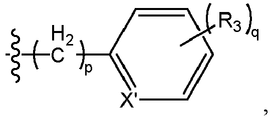 wherein X' is nitrogen or carbon, p is 1, q is an integer from 1 to 4, each occurrence of R₃ is independently hydrogen, a halide, an unsubstituted C₁-C₆ alkyl, an unsubstituted phenyl, or two neighboring R₃ together with the carbon to which they are attached form an unsubstituted aryl.
14. The iron-based catalyst of any one of paragraphs 1-13, wherein R₁₉ is a substituted or unsubstituted alkylene, optionally a substituted or unsubstituted methylene or ethylene group.
15. The iron-based catalyst of any one of paragraphs 1-14, wherein R₁₉ is an unsubstituted methylene group or a methylene group substituted with an unsubstituted C₁-C₆ alkyl, an unsubstituted phenyl, or an unsubstituted C₃-C₆ monocycloalkyl group.
16. The iron-based catalyst of any one of paragraphs 1-15, wherein R₂₃ and R₂₃' are hydrogen, and R₂₄ and R₂₄' are independently an unsubstituted C₁-C₁₀ alkyl, an unsubstituted C₁-C₈ alkyl, an unsubstituted C₁-C₆ alkyl, an unsubstituted C₁-C₄ alkyl, or an unsubstituted C₁-C₂ alkyl, such as a methyl.
17. The iron-based catalyst of any one of paragraphs 1-16, wherein the compound has a structure of: 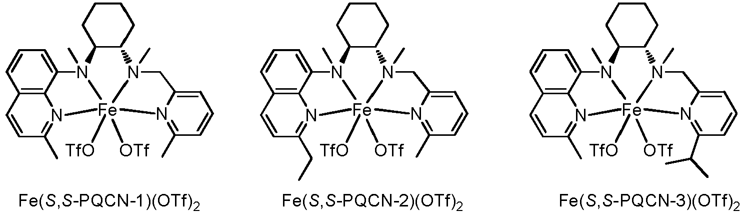 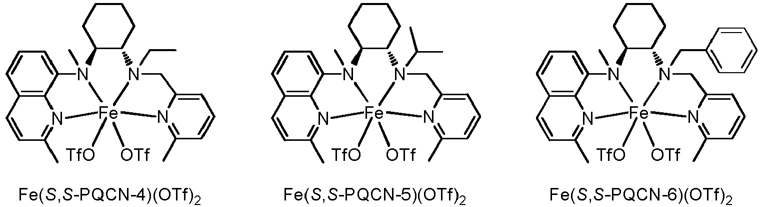 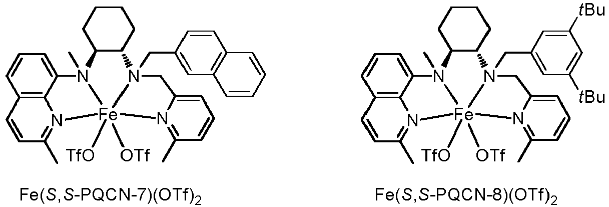 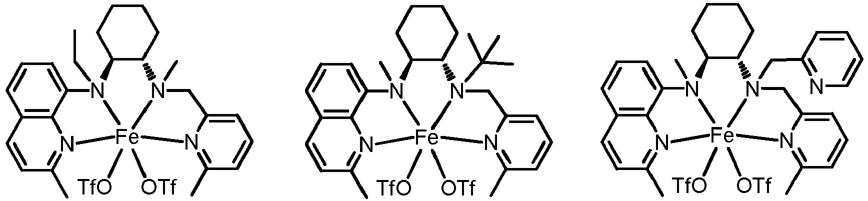 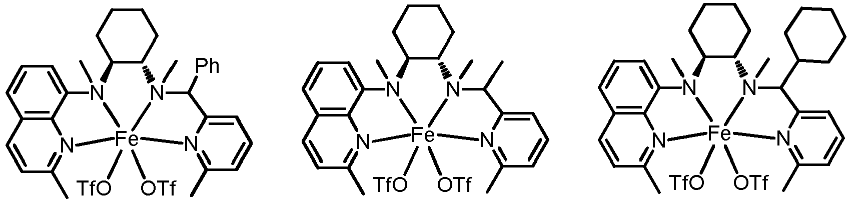 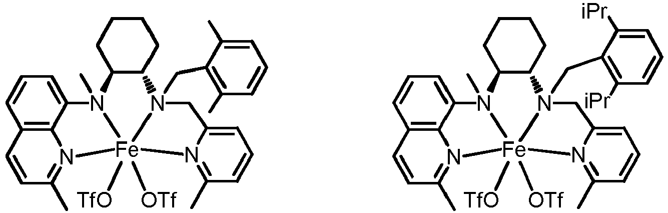 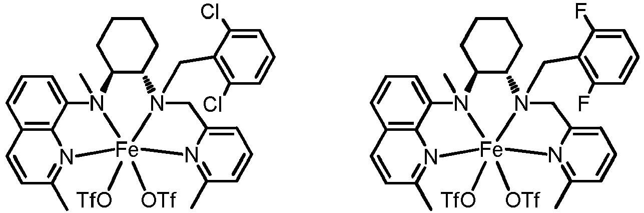 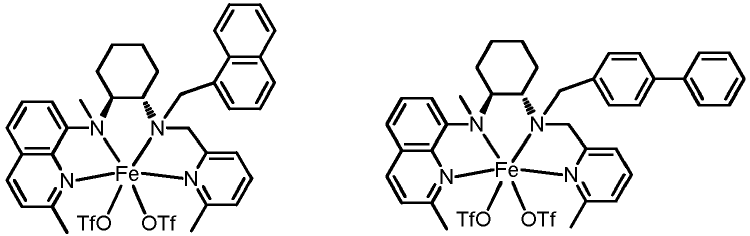 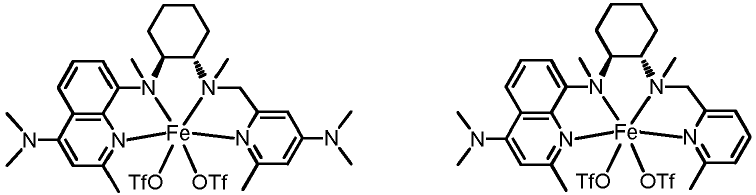 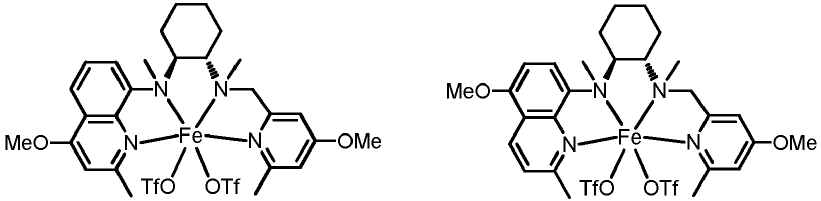 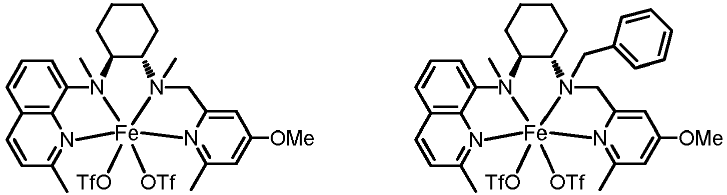 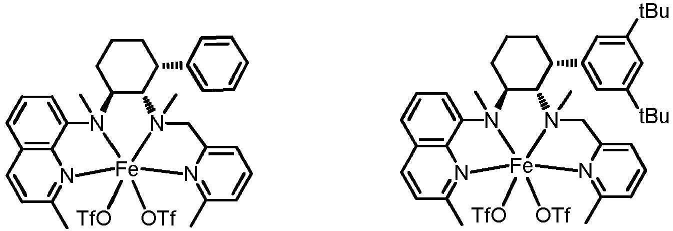 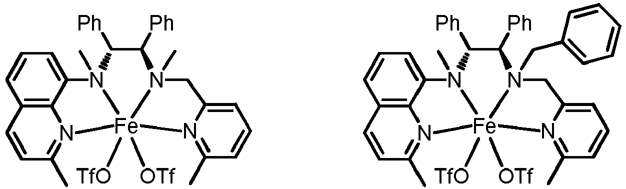 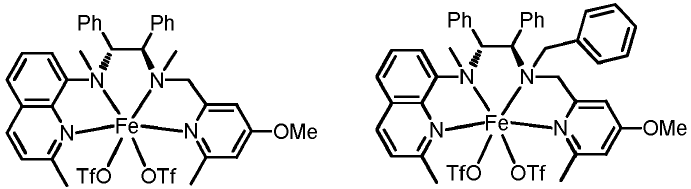 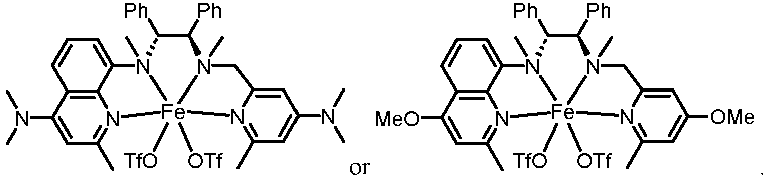
18. A method for asymmetric *cis*-dihydroxylation of a styrene or a derivative thereof catalyzed by the iron-based catalyst of any one of paragraphs 1-17, the method comprising:
   (i) maintaining a reaction mixture at a temperature for a period of time sufficient to form a product,
      wherein the reaction mixture comprises the styrene or derivative thereof, the iron-based catalyst, an oxidant, and a solvent,
      wherein the product comprises a cis-diol, and
      optionally wherein the oxidant is hydrogen peroxide.
19. The method of paragraph 18, wherein the styrene or derivative thereof has a structure of: 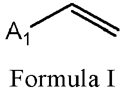 and the *cis*-diol has a structure of: 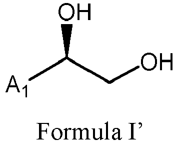 wherein A₁ is a substituted aryl, an unsubstituted aryl, a substituted polyaryl, an unsubstituted polyaryl, a substituted heteroaryl, an unsubstituted heteroaryl, a substituted heteropolyaryl, or an unsubstituted heteropolyaryl.
20. The method of paragraph 19, wherein the styrene or derivative thereof has a structure of: 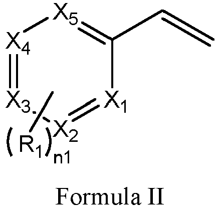 and the *cis*-diol has a structure of: 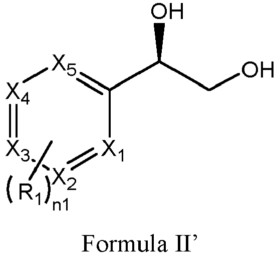 wherein:
   (a) X₁-X₅ are independently carbon, nitrogen, oxygen, or sulfur;
   (b) n1 is an integer from 0 to 5;
   (c) each occurrence of R₁ is independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₁ groups together with the carbon atoms to which they are attached form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
21. A method for asymmetric cis-dihydroxylation of a conjugated diene catalyzed by the iron-based catalyst of any one of paragraphs 1-17, the method comprising:
   (i) maintaining a reaction mixture at a temperature for a period of time sufficient to form a product,
      wherein the reaction mixture comprises the conjugated diene, the iron-based catalyst(s), an oxidant, and a solvent,
      wherein the product comprises a cis-diol or a tetraol, and
      optionally wherein the oxidant is hydrogen peroxide.
22. The method of paragraph 21, wherein the amount of the iron-based catalyst in the reaction mixture is up to 6 mol%, up to 5 mol%, up to 3 mol%, at least 0.1 mol%, at least 0.5 mol%, in a range from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%.
23. The method of paragraph 22, wherein the conjugated diene has a structure of: 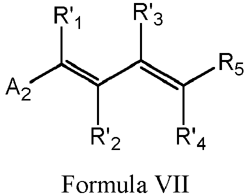 and the product comprises a cis-diol having a structure of: 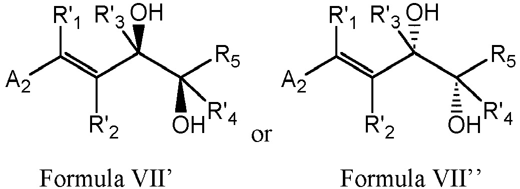 wherein:
   (a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
   (b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
   (c) R'₁-R'₄ are independently hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
24. The method of paragraph 21, wherein the amount of the iron-based catalyst in the reaction mixture is > 6 mol%, at least 7 mol%, at least 8 mol%, in a range from about 6.5 mol% to about 20 mol%, from about 7 mol% to about 20 mol%, from about 7 mol% to about 15 mol%, from about 6.5 mol% to about 15 mol%, from about 8 mol% to about 20 mol%, from about 8 mol% to about 15 mol%, from about 7 mol% to about 12 mol%, or from about 8 mol% to about 12 mol%, such as about 10 mol%.
25. The method of paragraph 24, wherein the conjugated diene has a structure of: 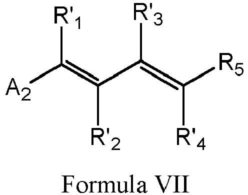 and the product comprises a tetraol having a structure of: 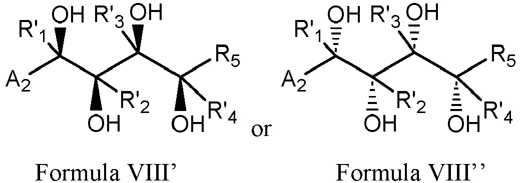 wherein
   (a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
   (b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
   (c) R'₁-R'₄ are independently a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

The disclosed substrates, iron-based catalysts, products, and methods can be further understood through the following enumerated paragraphs.
1. A method for asymmetric *cis*-dihydroxylation of a styrene or a derivative thereof comprising:
   (i) maintaining a reaction mixture at a temperature for a period of time sufficient to form a product,
      wherein the reaction mixture comprises the styrene or derivative thereof, one or more iron-based catalyst(s), an oxidant, and a solvent, and
      wherein the product comprises a cis-diol.
2. The method of paragraph 1, further comprising prior to step (i), adding the oxidant into a pre-mixture comprising the styrene or derivative thereof, one or more iron-based catalyst, and solvent to form the reaction mixture, and optionally wherein:
   the oxidant is hydrogen peroxide,
   the oxidant is added in the form of a solution comprising the oxidant and an oxidant solvent, or
   the oxidant is added using a syringe pump over a time period ranging from 20 mins to 1 hour, such as about 30 mins, at room temperature, or
   a combination thereof.
3. The method of paragraph 1 or 2, wherein the total mole amount of the oxidant in the reaction mixture is in a range from about 1-time to about 10-time, from about 1.5-time to about 6-time, or from about 1-time to about 5-time of the total mole amount of the styrene or derivative thereof.
4. The method of any one of paragraphs 1-3, wherein each of the one or more iron-based catalyst(s) has a structure of: 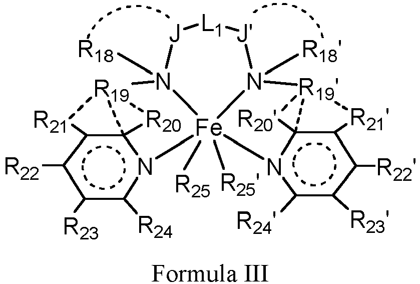 wherein:
   (a) J and J' are independently a bond (single, double, or triple), a substituted or unsubstituted alkyl, a substituted or unsubstituted alkenyl, a substituted or unsubstituted cyclic group, a substituted or unsubstituted aryl, or a substituted or unsubstituted polyaryl;
   (b) L₁ is a bond or 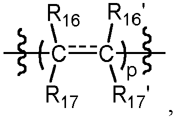 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' together and/or R₁₇ and R₁₇' together, with the carbon atoms to which they are attached, form a substituted or unsubstituted aryl, a substituted or unsubstituted polyaryl, or a substituted or unsubstituted cyclic group, and p is an integer from 1 to 10;
   (c) R₁₈-R₂₄ and R₁₈'-R₂₄' are independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl;
   (d) R₂₅ and R₂₅' are independently a leaving group;
   (e) ------ is absent or a bond (single, double, or triple); and
   (f) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.
5. The method of paragraph 4, wherein R₁₉, R₂₀, and R₂₁ together and/or R₁₉', R₂₀', and R₂₁' together with the carbon atoms to which they are attached form a substituted or unsubstituted aryl, a substituted or unsubstituted polyaryl, or a substituted or unsubstituted cyclic group.
6. The method of any one of paragraphs 1-5, wherein each of the one or more iron-based catalyst(s) has a structure of: 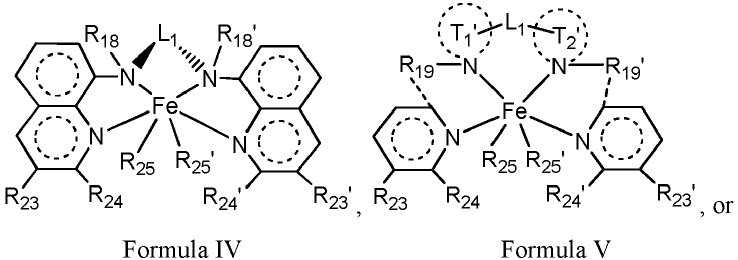 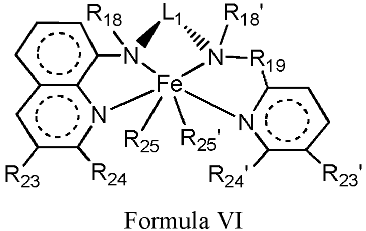 wherein:
   (a) L₁ is a bond or 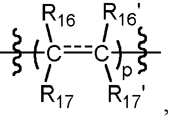 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' and/or R₁₇ and R₁₇' form a substituted or unsubstituted aryl, a substituted or unsubstituted polyaryl, or a substituted or unsubstituted cyclic group, and p is an integer from 1 to 10;
   (b) T₁' and T₂' are independently a substituted or unsubstituted heteroaryl, a substituted or unsubstituted heteropolyaryl, or a substituted or unsubstituted heterocyclic group;
   (c) R₁₈, R₂₃, R₂₄, R₁₈', R₂₃', and R₂₄' are independently a hydrogen, a substituted or unsubstituted alkyl, a substituted or unsubstituted alkenyl, a substituted or unsubstituted aryl, a substituted or unsubstituted alkylaryl, or a substituted or unsubstituted polyaryl;
   (d) R₁₉ and R₁₉' are independently a substituted or unsubstituted alkyl, a substituted or unsubstituted alkenyl, a substituted or unsubstituted aryl, a substituted or unsubstituted alkylaryl, or a substituted or unsubstituted polyaryl;
   (e) R₂₅ and R₂₅' are independently a leaving group; and
   (f) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.
7. The method of paragraph 6, wherein each of the one or more iron-based catalyst(s) has a structure of: 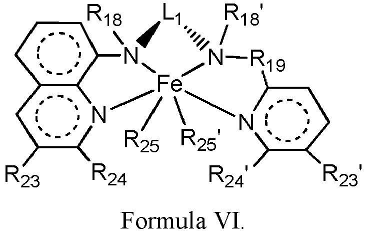
8. The method of paragraph 6, (a) wherein L₁ is 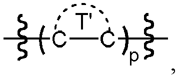 each occurrence of T' is a substituted or unsubstituted monocyclic group or a substituted or unsubstituted polycyclic group and p is an integer from 1 to 3; (b) wherein R₂₅ and R₂₅' are independently a triflate, a tosylate, a mesylate, a halide, a nitrate, a phosphate, a thioether, an amino, a carboxylate, a phenoxide, an alkoxyl, or an amido; or (c) or a combination thereof.
9. The method of any one of paragraphs 1-8, wherein the catalyst has a structure of: 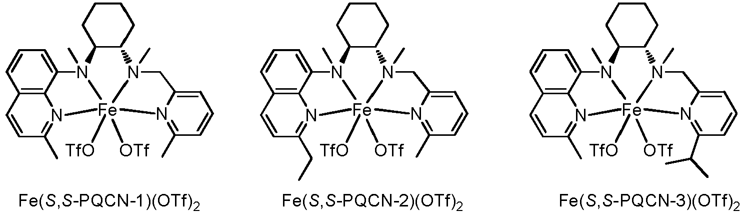 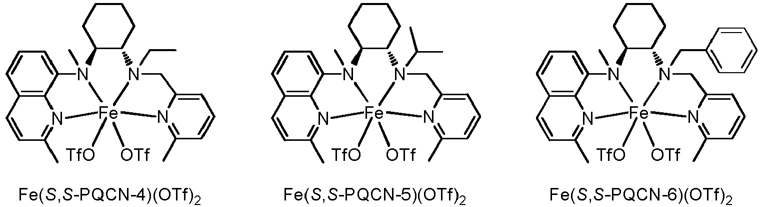 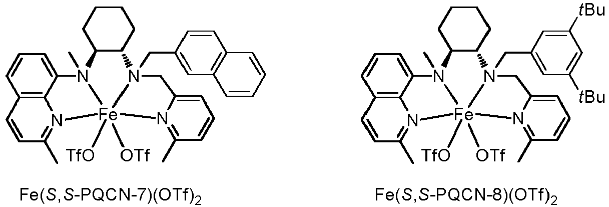 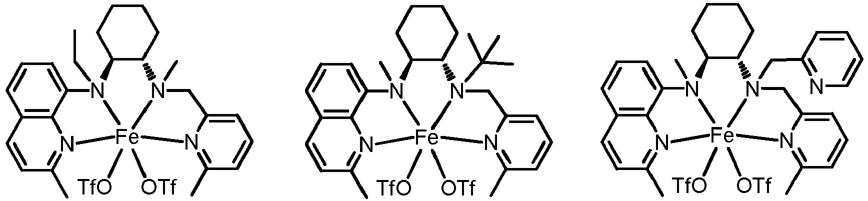 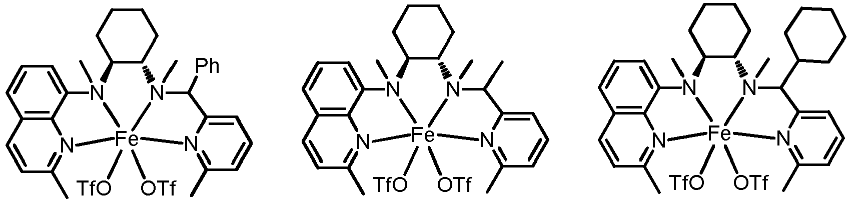 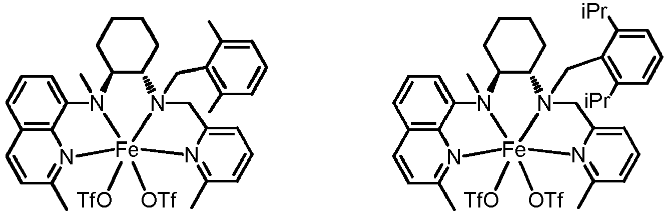 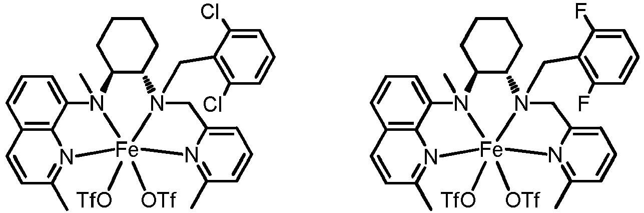 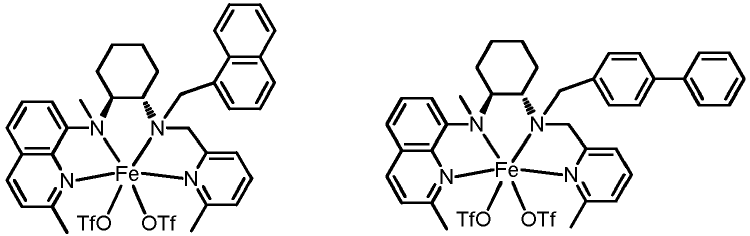 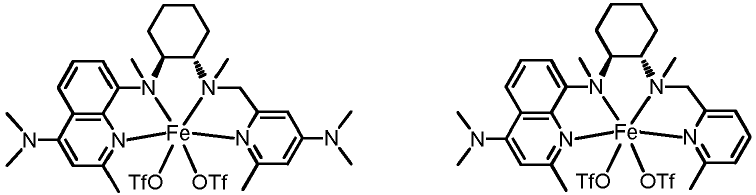 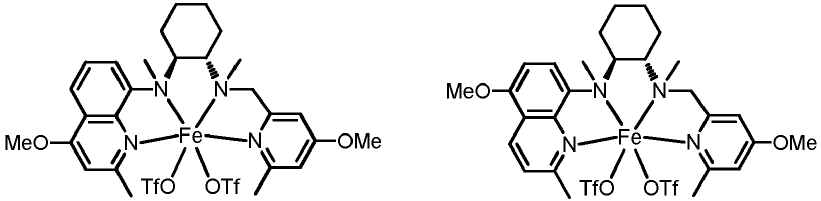 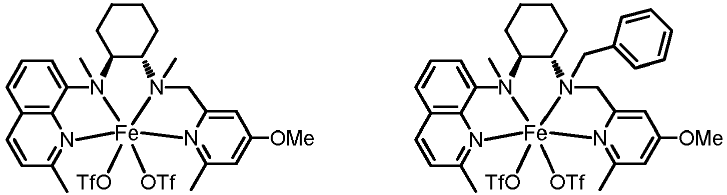 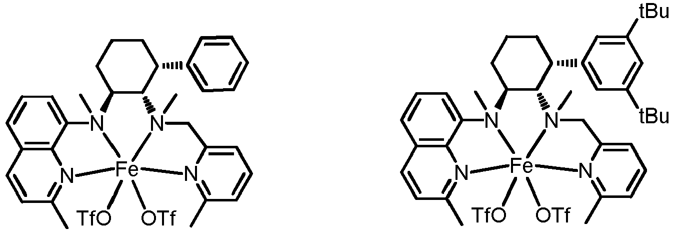 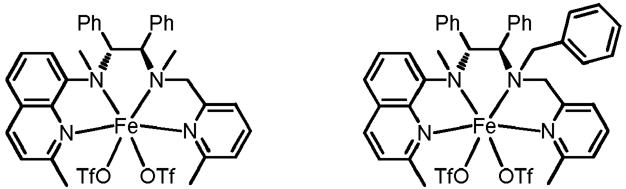 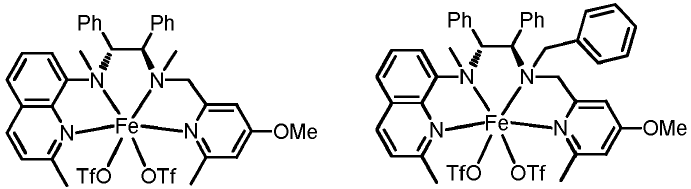 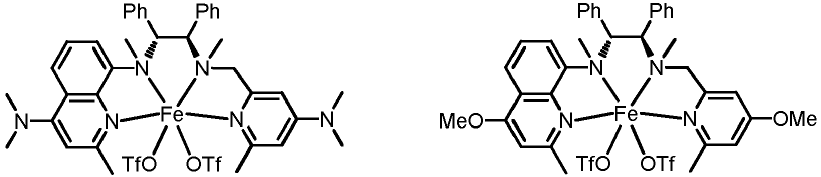 or 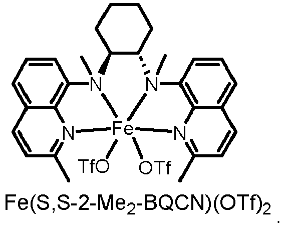
10. The method of any one of paragraphs 1-3, wherein each of the one or more iron-based catalyst(s) has a structure of: 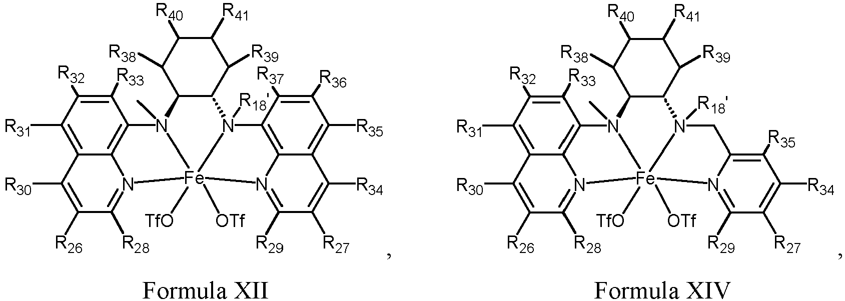 or 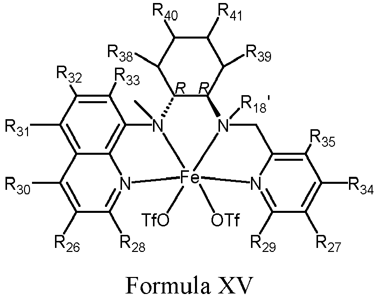 wherein:
   (a) R₂₆ and R₂₇ are independently hydrogen, a substituted or unsubstituted cyclic group, a substituted or unsubstituted aryl, or a substituted or unsubstituted polyaryl;
   (b) R₂₈-R₄₁ are independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl;
   (c) R₁₈' are an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 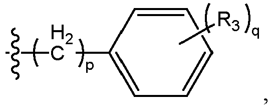 p and q are independently an integer from 0 to 5, and each occurrence of R₃ is independently hydrogen or an unsubstituted C₁-C₁₀ alkyl, or two neighboring R3 together with the carbon to which they are attached form an unsubstituted aryl or unsubstituted polyaryl; and
   (d) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof. In some forms of Formula XII, R₂₆ and R₂₇ are not hydrogen.
11. The method of paragraph 10, wherein (a) R₂₆ and R₂₇ are independently hydrogen, a substituted or unsubstituted alkyl, or a substituted or unsubstituted aryl; (b) R₂₈ and R₂₉ are independently a hydrogen, a substituted or unsubstituted alkyl, or a substituted or unsubstituted alkenyl; (c) R₃₀-R₄₁ are independently hydrogen or a substituted or unsubstituted alkyl; and (d) the substituents are independently a substituted or unsubstituted alkyl, a substituted or unsubstituted alkenyl, or a substituted or unsubstituted alkynyl, or a combination thereof.
12. The method of paragraph 10 or 11, wherein (a) R₂₆ and R₂₇ is hydrogen, (b) R₂₈ and R₂₉ are independently a substituted or unsubstituted alkyl, such as an unsubstituted C₁-C₈ alkyl, and (c) R₃₀-R₄₀ is hydrogen.
13. The method of any one of paragraphs 10-12, wherein each of the one or more iron-based catalyst(s) has a structure of: 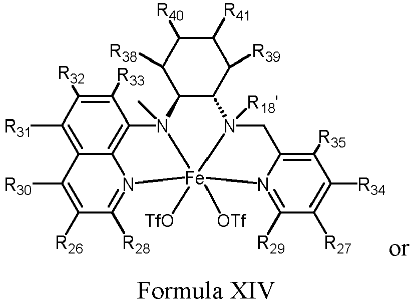 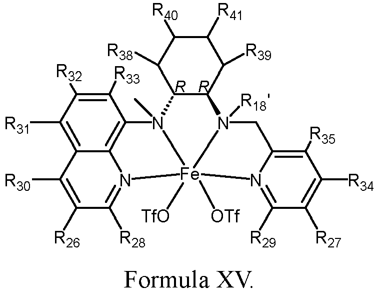
14. The method of any one of paragraphs 1-13,
   (a) wherein the total amount of the one or more iron-based catalyst(s) in the reaction mixture is up to 10 mol%, up to 8 mol%, up to 6 mol%, up to 5 mol%, up to 3 mol%, at least 0.1 mol%, at least 0.5 mol%, in a range from about 0.1 mol% to about 10 mol%, from about 0.1 mol% to about 8 mol%, from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%;
   (b) wherein the solvent is an alcohol, optionally a C₁-C₆ alcohol, such as methanol; or
   (c) wherein the reaction mixture is maintained at a temperature below 0°C (e.g., about -30°C) for a period of time in a range from about 20 minutes to about 3 hours, from about 20 minutes to about 2 hours, or from about 20 minutes to 1 hour (e.g., about 30 mins); or
   (d) a combination thereof.
15. The method of any one of paragraphs 1-14, wherein the styrene or derivative thereof has a structure of: 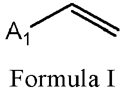 and the *cis*-diol has a structure of: 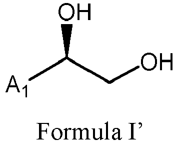 wherein A₁ is a substituted aryl, an unsubstituted aryl, a substituted polyaryl, an unsubstituted polyaryl, a substituted heteroaryl, an unsubstituted heteroaryl, a substituted heteropolyaryl, or an unsubstituted heteropolyaryl.
16. The method of paragraph 15, wherein the styrene or derivative thereof has a structure of: 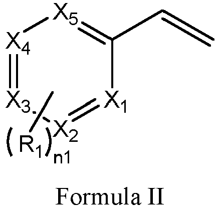 and the *cis*-diol has a structure of: 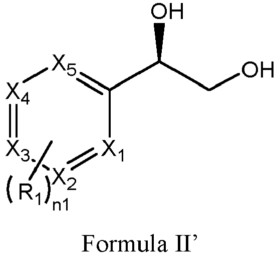 wherein:
   (a) X₁-X₅ are independently carbon, nitrogen, oxygen, or sulfur;
   (b) n1 is an integer from 0 to 5;
   (c) each occurrence of R₁ is independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₁ groups together with the carbon atoms to which they are attached form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
17. The method of paragraph 16, wherein each occurrence of R₁ is independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 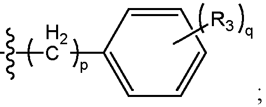 or wherein two neighboring R₁ groups together with the carbon atoms to which they are attached form a substituted aryl or an unsubstituted aryl, and the substituents are independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 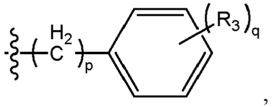 wherein p and q are independently an integer from 0 to 5, and R₂-R₄ are independently hydrogen, an unsubstituted C₁-C₁₀ alkyl, halide, or -O-(C₁-C₁₀ alkyl).
18. The method of paragraph 16 or 17, wherein n1 is not 0, and at least one R₁, at least two R₁, or at least three R₁ is/are electron-withdrawing group(s).
19. The method of paragraph 18, wherein n1 is 1, and R₁ is an electron-withdrawing group at the meta-position or para-position.
20. The method of paragraph 16 or 17, wherein n1 is 1 and R₁ is 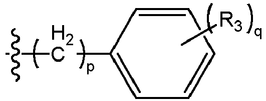 at the ortho-position, p and q are independently an integer from 0 to 5, and each occurrence of R₃ is independently hydrogen, an unsubstituted C₁-C₁₀ alkyl, halide, or -O-(C₁-C₁₀ alkyl).
21. The method of paragraph 16 or 17, wherein X₁ is nitrogen and X₂-X₅ are carbon.
22. The method of paragraph 21, wherein two neighboring R₁ groups together with the carbon atoms to which they are attached form a substituted aryl or an unsubstituted aryl, and the substituents are independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 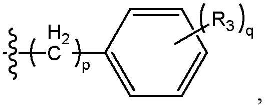 p and q are independently an integer from 0 to 5, and R₂-R₄ are independently hydrogen, an unsubstituted C₁-C₁₀ alkyl, halide, or -O-(C₁-C₁₀ alkyl).
23. The method of any one of paragraphs 15-22, wherein the *cis*-diol has a structure of: 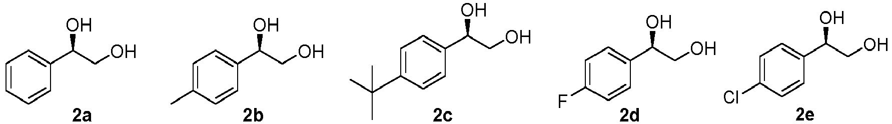 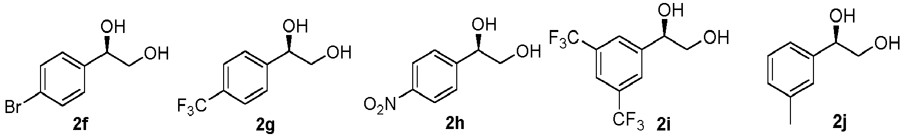 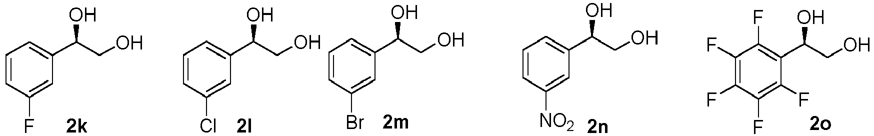 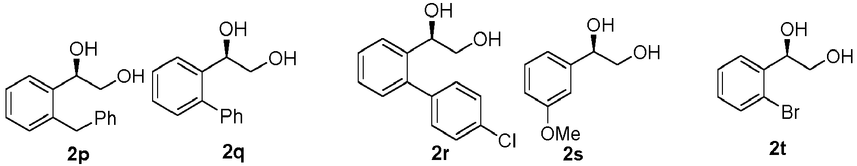 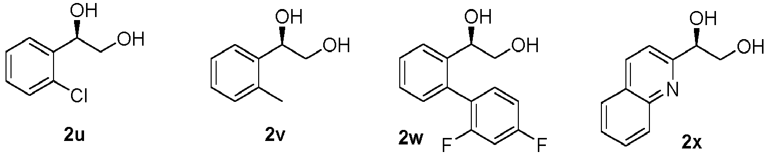 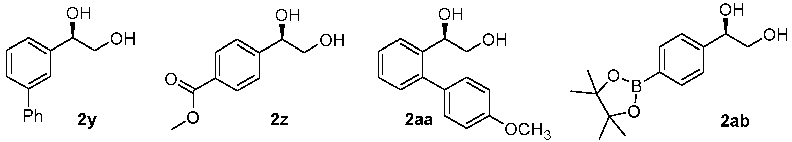
24. The method of any one of paragraphs 1-23, further comprising stirring the reaction mixture prior to and/or during step (i); or purifying the product, optionally by column chromatography, subsequent to step (i); or a combination thereof.
25. The method of any one of paragraphs 1-24,
   (a) wherein the cis-diol has a yield of at least 40%, at least 50%, in a range from about 40% to about 99%, from about 50% to about 99%, from about 60% to about 99%, from about 70% to about 99%, or from about 80% to about 99%; or
   (b) wherein the cis-diol has an enantiometric excess of at least 60%, at least 70%, at least 75%, at least 80%, up to 100%, in a range from about 60% to 100%, from about 60% to 100%, from about 70% to 100%, from about 80% to 100%, from about 90% to 100%, or from about 95% to 100%, from about 60% to 99%, from about 70% to 99%, from about 80% to 99%, from about 90% to 99%, or from about 95% to 99%, as determined by chiral HPLC; or
   (c) a combination thereof.
26. A method for asymmetric cis-dihydroxylation of a conjugated diene comprising:
   (i) maintaining a reaction mixture at a temperature for a period of time sufficient to form a product,
      wherein the reaction mixture comprises the conjugated diene, one or more iron-based catalyst(s), an oxidant, and a solvent, and
      wherein the product comprises a cis-diol or a tetraol.
27. The method of paragraph 26 further comprising prior to step (i), adding the oxidant into a pre-mixture comprising the conjugated diene, one or more iron-based catalyst, and solvent to form the reaction mixture, and optionally wherein:
   the oxidant is hydrogen peroxide, or
   the oxidant is added in the form of a solution comprising the oxidant and an oxidant solvent, or
   a combination thereof.
28. The method of paragraph 26 or 27, wherein the total mole amount of the oxidant in the reaction mixture is in a range from about 1-time to about 10-time, from about 1.5-time to about 6-time, or from about 1-time to about 5-time of the total mole amount of the conjugated diene.
29. The method of any one of paragraphs 26-28, wherein each of the one or more iron-based catalyst(s) has a structure of: 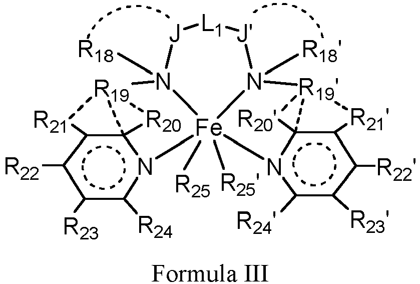 wherein:
   (a) J and J' are independently a bond (single, double, or triple), a substituted or unsubstituted alkyl, a substituted or unsubstituted alkenyl, a substituted or unsubstituted cyclic group, a substituted or unsubstituted aryl, or a substituted or unsubstituted polyaryl;
   (b) L₁ is a bond or 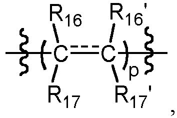 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' together and/or R₁₇ and R₁₇' together, with the carbon atoms to which they are attached, form a substituted or unsubstituted aryl, a substituted or unsubstituted polyaryl, or a substituted or unsubstituted cyclic group, and p is an integer from 1 to 10;
   (c) R₁₈-R₂₄ and R₁₈'-R₂₄' are independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl;
   (d) R₂₅ and R₂₅' are independently a leaving group;
   (e) ------ is absent or a bond (single, double, or triple); and
   (f) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.
30. The method of paragraph 29, wherein R₁₉, R₂₀, and R₂₁ together and/or R₁₉', R₂₀', and R₂₁' together with the carbon atoms to which they are attached form a substituted or unsubstituted aryl, a substituted or unsubstituted polyaryl, or a substituted or unsubstituted cyclic group.
31. The method of paragraph 29 or 30, wherein each of the one or more iron-based catalyst(s) has a structure of: 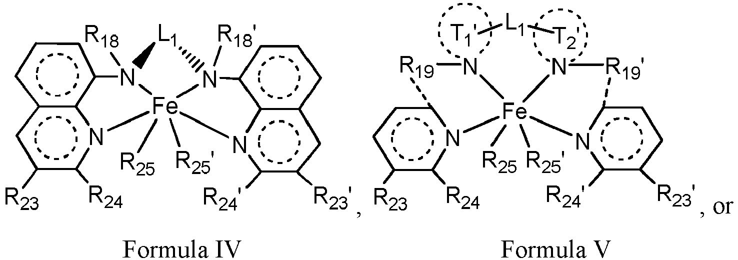 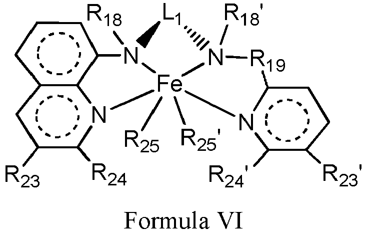 wherein:
   (a) L₁ is a bond or 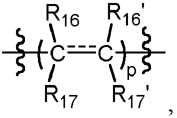 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' and/or R₁₇ and R₁₇' form a substituted or unsubstituted aryl, a substituted or unsubstituted polyaryl, or a substituted or unsubstituted cyclic group, and p is an integer from 1 to 10;
   (b) T₁' and T₂' are independently a substituted or unsubstituted heteroaryl, a substituted or unsubstituted heteropolyaryl, or a substituted or unsubstituted heterocyclic group;
   (c) R₁₈, R₁₉, R₂₃, R₂₄, R₁₈', R₁₉', R₂₃', and R₂₄' are independently a hydrogen, a substituted or unsubstituted alkyl, a substituted or unsubstituted alkenyl, a substituted or unsubstituted aryl, a substituted or unsubstituted alkylaryl, or a substituted or unsubstituted polyaryl;
   (d) R₂₅ and R₂₅' are independently a leaving group; and
   (e) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.
32. The method of paragraph 31, wherein each of the one or more iron-based catalyst(s) has a structure of: 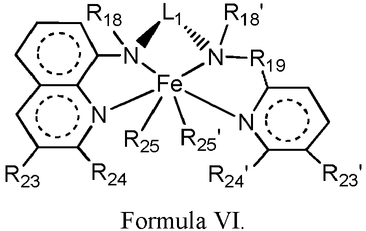
33. The method of paragraph 31, (a) wherein L₁ is 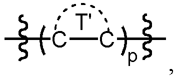 each occurrence of T' is a substituted or unsubstituted monocyclic group or a substituted or unsubstituted polycyclic group and p is an integer from 1 to 3; (b) wherein R₂₅ and R₂₅' are independently a triflate, a tosylate, a mesylate, a halide, a nitrate, a phosphate, a thioether, an amino, a carboxylate, a phenoxide, an alkoxyl, or an amido; or (c) or a combination thereof.
34. The method of any one of paragraphs 26-33, wherein the catalyst has a structure of: 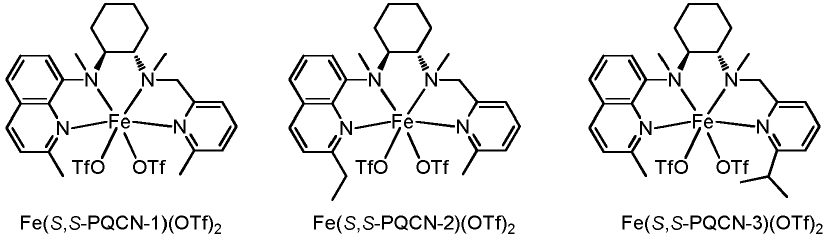 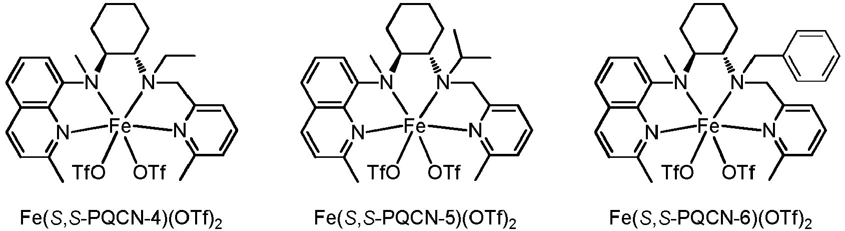 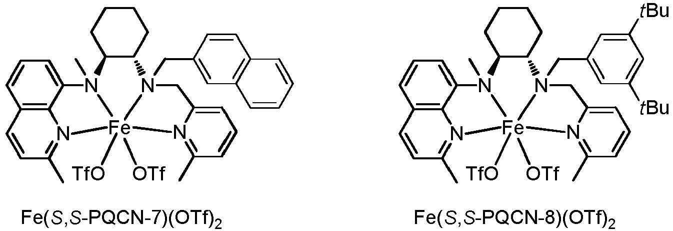 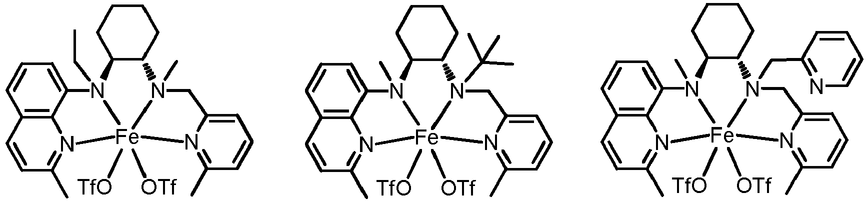 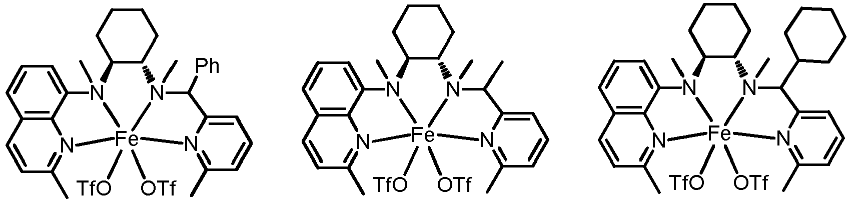 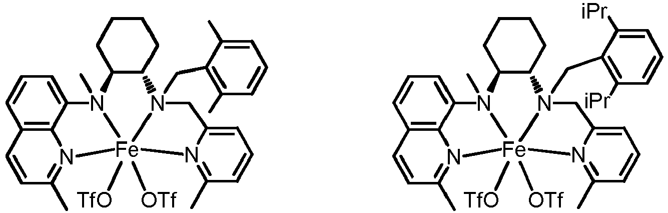 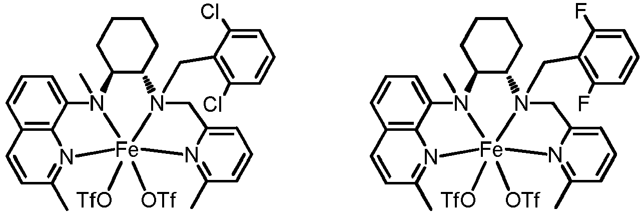 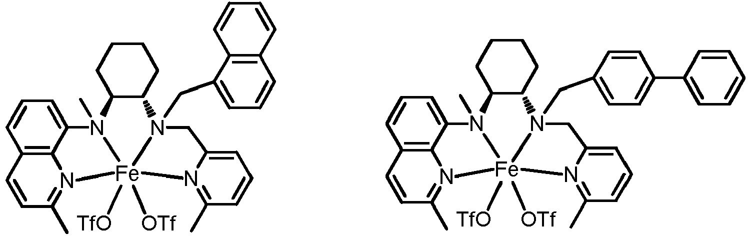 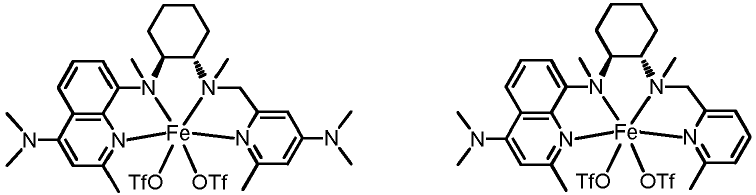 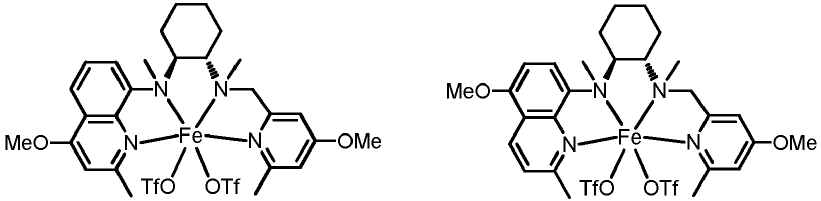 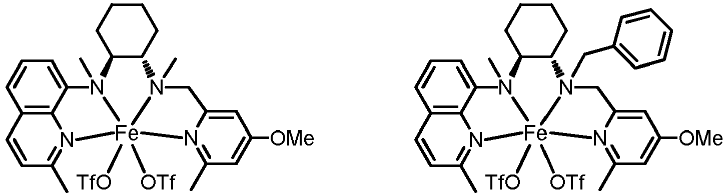 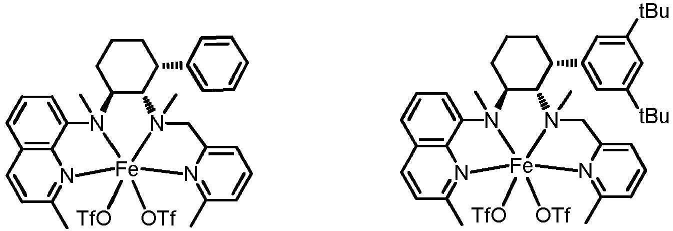 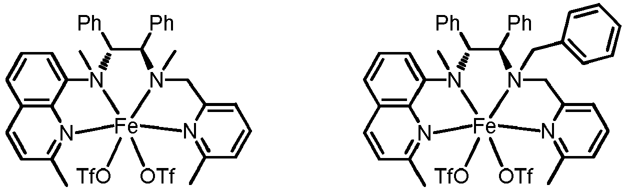 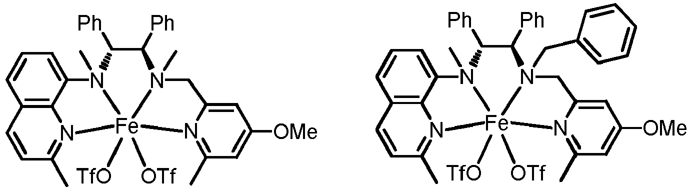 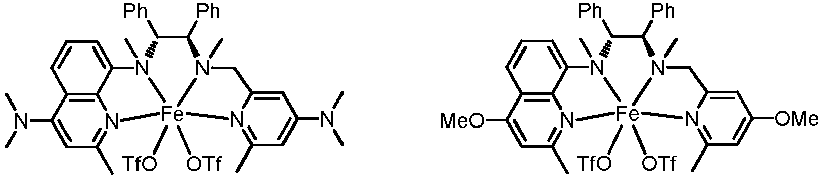 or 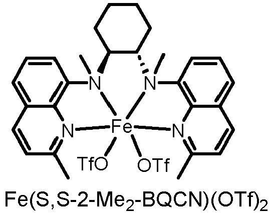
35. The method of any one of paragraphs 26-28, wherein each of the one or more iron-based catalyst(s) has a structure of: 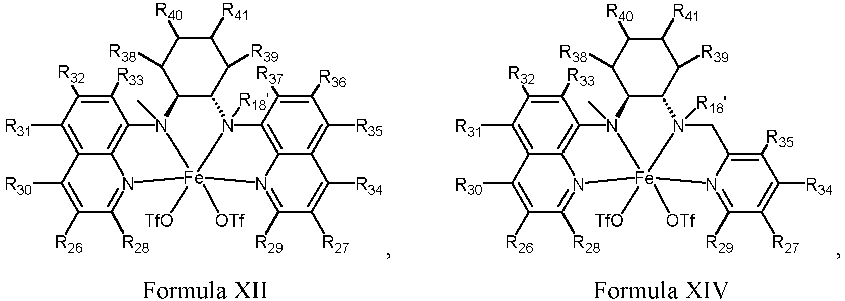 or 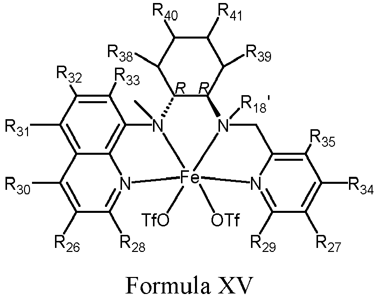 wherein:
   (a) R₂₆ and R₂₇ are independently hydrogen, a substituted or unsubstituted cyclic group, a substituted or unsubstituted aryl, or a substituted or unsubstituted polyaryl;
   (b) R₂₈-R₄₁ are independently a hydrogen, a substituted or unsubstituted alkyl, a substituted or unsubstituted alkenyl, a substituted or unsubstituted cyclic group, a substituted or unsubstituted aryl, or a substituted or unsubstituted polyaryl;
   (c) R₁₈' are an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 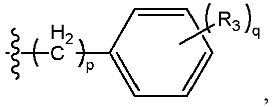 p and q are independently an integer from 0 to 5, and each occurrence of R₃ is independently hydrogen or an unsubstituted C₁-C₁₀ alkyl, or two neighboring R3 together with the carbon to which they are attached form an unsubstituted aryl or unsubstituted polyaryl; and
   (d) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof. In some forms of Formula XII, R₂₆ and R₂₇ are not hydrogen.
36. The method of paragraph 35, wherein (a) R₂₆ and R₂₇ are independently hydrogen, a substituted or unsubstituted alkyl, or a substituted or unsubstituted aryl; (b) R₂₈ and R₂₉ are independently a hydrogen, a substituted or unsubstituted alkyl, or a substituted or unsubstituted alkenyl; (c) R₃₀-R₄₁ are independently hydrogen or a substituted or unsubstituted alkyl; and (d) the substituents are independently a substituted or unsubstituted alkyl, a substituted or unsubstituted alkenyl, or a substituted or unsubstituted alkynyl, or a combination thereof.
37. The method of paragraph 35 or 36, wherein (a) R₂₆ and R₂₇ is hydrogen, (b) R₂₈ and R₂₉ are independently a substituted or unsubstituted alkyl, such as an unsubstituted C₁-C₈ alkyl, and (c) R₃₀-R₄₀ is hydrogen.
38. The method of any one of paragraphs 35-37, wherein each of the one or more iron-based catalyst(s) has a structure of: 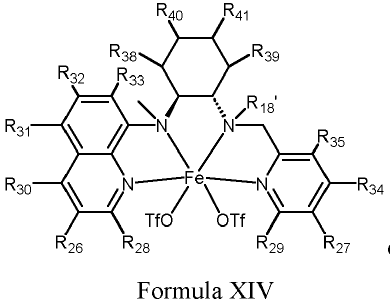 or 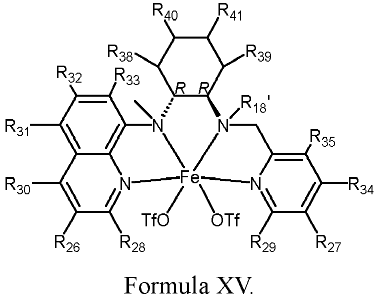
39. The method of any one of paragraphs 26-38, wherein the total amount of the one or more iron-based catalyst(s) in the reaction mixture is up to 6 mol%, up to 5 mol%, up to 3 mol%, at least 0.1 mol%, at least 0.5 mol%, in a range from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%.
40. The method of paragraph 39, wherein the oxidant is added into the pre-mixture comprising the conjugated diene, one or more iron-based catalyst, and solvent using a syringe pump over a time period ranging from 20 mins to 1 hour, such as about 30 mins, at room temperature.
41. The method of paragraph 39 or 40, wherein the reaction mixture is maintained at room temperature for a period of time in a range from about 20 minutes to about 3 hours, from about 20 minutes to about 2 hours, from about 20 minutes to 1 hour, or from about 20 mins to about 50 mins (e.g., about 30 mins).
42. The method of any one of paragraphs 39-41, wherein the conjugated diene has a structure of: 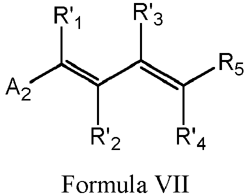 and the product comprises a cis-diol having a structure of: 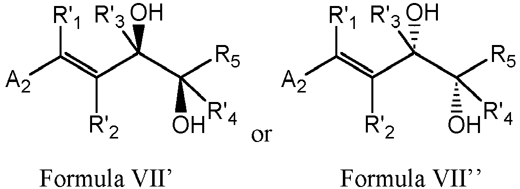 wherein:
   (a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
   (b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
   (c) R'₁-R'₄ are independently hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
43. The method of any one of paragraphs 39-42, wherein the catalyst has the structure of: 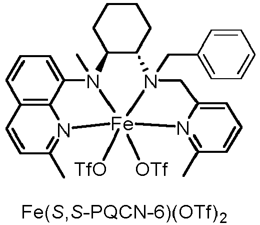 and the *cis*-diol has the structure of: 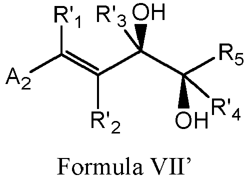
44. The method of any one of paragraphs 39-42, wherein the catalyst has the structure of: 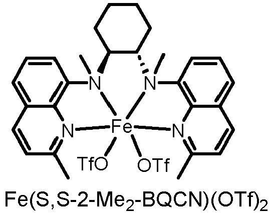 and the *cis*-diol has the structure of: 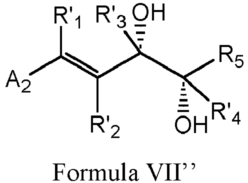
45. The method of any one of paragraphs 42-44, wherein:
   (a) the *cis*-diol has a yield of at least 30%, at least 40%, in a range from about 30% to about 99%, from about 40% to about 99%, from about 50% to about 99%, from about 60% to about 99%, or from about 70% to about 99%; or
   (b) the *cis*-diol has an enantiometric excess of at least 80%, at least 85%, up to 100%, in a range from about 80% to 100%, from about 85% to 100%, from about 90% to 100%, from about 80% to 99.9%, from about 85% to 99.9%, or from about 90% to 99.9%, from about 80% to 99.5%, from about 85% to 99.5%, from about 90% to 99.5%, from about 80% to 99%, or from about 85% to 99%,as determined by chiral HPLC; or
   (c) a combination thereof.
46. The method of any one of paragraphs 26-38, wherein the total amount of the one or more iron-based catalyst(s) in the reaction mixture is > 6 mol%, at least 7 mol%, at least 8 mol%, in a range from about 6.5 mol% to about 20 mol%, from about 7 mol% to about 20 mol%, from about 7 mol% to about 15 mol%, from about 6.5 mol% to about 15 mol%, from about 8 mol% to about 20 mol%, from about 8 mol% to about 15 mol%, from about 7 mol% to about 12 mol%, or from about 8 mol% to about 12 mol%, such as about 10 mol%.
47. The method of paragraph 46, wherein the oxidant is directly added into the pre-mixture comprising the conjugated diene, one or more iron-based catalyst, and solvent to form the reaction mixture, at room temperature.
48. The method of paragraph 46 or 47, wherein the reaction mixture is maintained at room temperatures for a period of time of at least 1 hour, in a range from 1 hour to about 5 hours, from 1 hour to about 4 hours, from 1 hour to about 3 hours, from 1 hour to about 2 hours (e.g., 1 hour).
49. The method of any one of paragraphs 46-48, wherein the conjugated diene has a structure of: 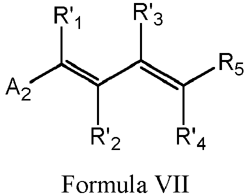 and the product comprises a tetraol having a structure of: 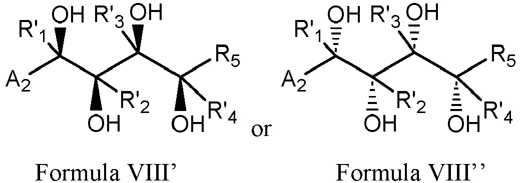 wherein
   (a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
   (b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
   (c) R' ₁-R'₄ are independently a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
   (d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
50. The method of any one of paragraphs 46-49, wherein the catalyst has the structure of: 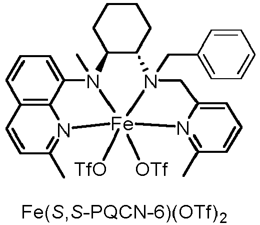 and the tetraol has the structure of: 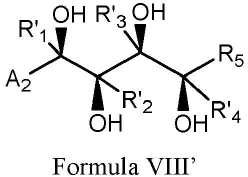
51. The method of any one of paragraphs 46-48, wherein the catalyst has the structure of: 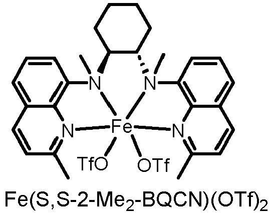 and the tetraol has the structure of: 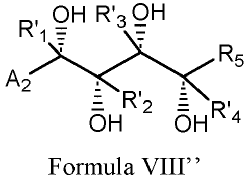
52. The method of any one of paragraphs 49-51, wherein:
   (a) the tetraol has a yield of at least 30%, at least 40%, in a range from about 30% to about 99%, from about 40% to about 99%, from about 50% to about 99%, from about 60% to about 99%, or from about 70% to about 99%; or
   (b) the tetraol has an enantiometric excess of at least 90%, at least 95%, at least 99.9%, >99.9%, up to 100%, in a range from about 90% to 100%, from about 95% to 100%, from about 90% to 99.9%, from about 95% to 99.9%, or from about 99.9% to 100%, as determined by chiral HPLC; or
   (c) a combination thereof.
53. The method of any one of paragraphs 42-52, wherein A₂ is 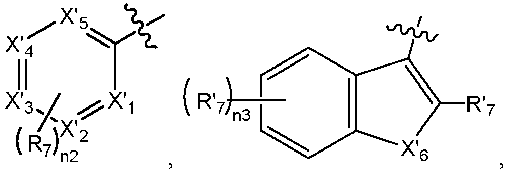 or -C(=O)OR₁₄, wherein:
   (a) X'₁-X'₆ are independently carbon, nitrogen, oxygen, or sulfur;
   (b) n2 and n3 are independently an integer from 0 to 5;
   (c) each occurrence of R₇ and R'₇ are independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₇ groups or two neighboring R'₇ groups together with the carbon atoms to which they are attached form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group;
   (d) R₁₄ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl; and
   (e) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
54. The method of paragraph 53, wherein each occurrence of R₇ and R'₇ are independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., - CF₃), a nitro, a cyano, -OR₂, -C(=O)OR₄, or 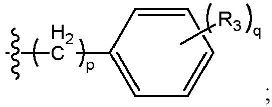 or two neighboring R₇ groups or two neighboring R'₇ together with the carbon atoms to which they are attached form a substituted aryl or an unsubstituted aryl, and the substituents are independently an unsubstituted C₁-C₁₀ alkyl, an unsubstituted heterocyclic, a heterocyclic substituted with an unsubstituted C₁-C₁₀ alkyl, a halide, a haloalkyl (e.g., -CF₃), a nitro, a cyano, -OR₂, -C(=O)OR₄, or 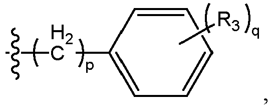 wherein p and q are independently an integer from 0 to 5, and R₂-R₄ are independently hydrogen, an unsubstituted C₁-C₁₀ alkyl, halide, or -O-(C₁-C₁₀ alkyl).
55. The method of paragraph 53 or 54, wherein each occurrence of R₇ and R'₇ are independently a hydrogen, an unsubstituted C₁-C₁₀ alkyl, an unsubstituted heterocyclic, a heterocyclic substituted with an unsubstituted C₁-C₁₀ alkyl, a halide, a haloalkyl (e.g., - CF₃), or a cyano.
56. The method of any one of paragraphs 53-55, wherein X'₂-X'₅ are carbon, X'₁ is carbon or nitrogen, and X'₆ is carbon, nitrogen, oxygen, or sulfur.
57. The method of any one of paragraphs 42-56, wherein R₆ is an unsubstituted C₁-C₁₀ alkyl or 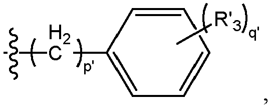 p' and q' are independently an integer from 0 to 5, and each occurrence of R'₃ is independently hydrogen, an unsubstituted C₁-C₁₀ alkyl, halide, or -O-(C₁-C₁₀ alkyl).
58. The method of paragraph 57, wherein R₆ is an unsubstituted C₁-C₈ alkyl, an unsubstituted C₁-C₆ alkyl, an unsubstituted C₁-C₄ alkyl, or 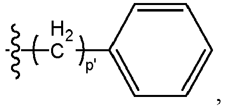 and p' is an integer from 0 to 3, from 0 to 2, or 0 or 1.
59. The method of any one of paragraphs 42-58, wherein R'₁-R'₄ are independently hydrogen, an unsubstituted C₁-C₁₀ alkyl, an unsubstituted aryl, or an unsubstituted alkylaryl.
60. The method of any one of paragraphs 53-59, wherein R₁₄ is an unsubstituted C₁-C₈ alkyl, an unsubstituted C₁-C₆ alkyl, an unsubstituted C₁-C₄ alkyl, or 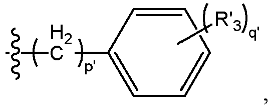 p' and q' are independently an integer from 0 to 5, and each occurrence of R'₃ is independently hydrogen, an unsubstituted C₁-C₁₀ alkyl, halide, or -O-(C₁-C₁₀ alkyl), optionally wherein R₁₄ is an unsubstituted C₁-C₄ alkyl or 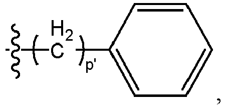 and p' is an integer from 0 to 3, from 0 to 2, or 0 or 1.
61. The method of any one of paragraphs 42, 43, 45, and 53-60, wherein the *cis-*diol has the structure of: 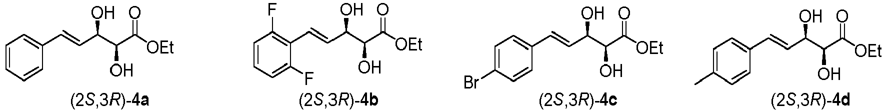 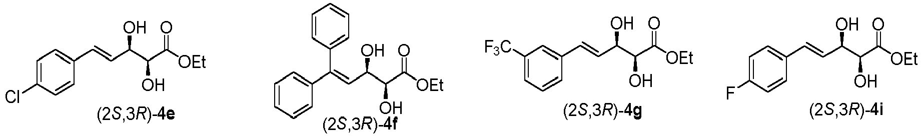 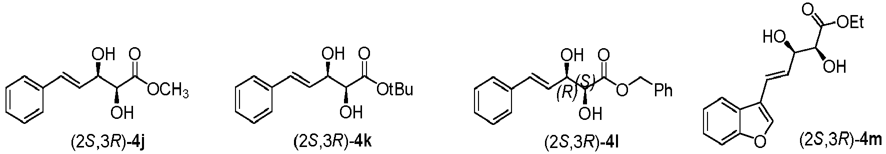 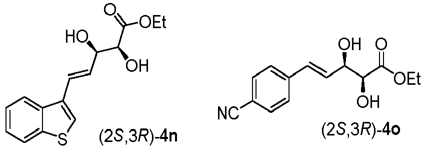
62. The method of any one of paragraphs 42, 44, 45, and 53-60, wherein the *cis-*diol has the structure of: 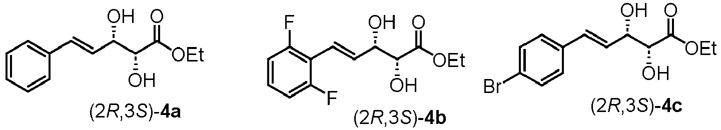 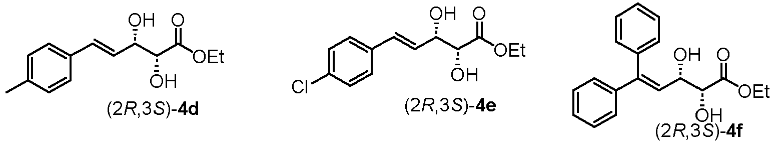 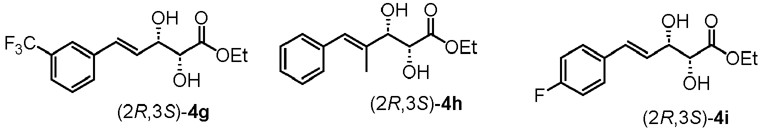 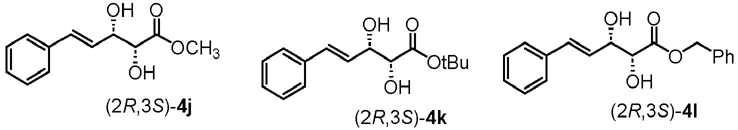 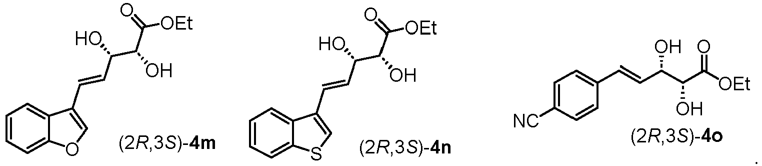
63. The method of any one of paragraphs 49, 50, and 52-60, wherein the tetraol has the structure of: 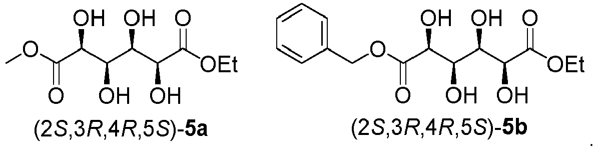
64. The method of any one of paragraphs 49, 51, and 52-60, wherein the tetraol has the structure of: 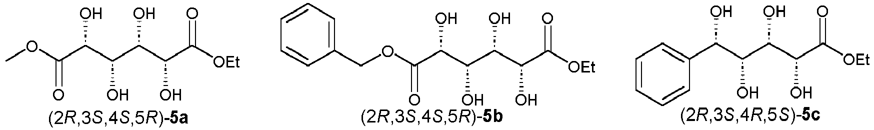 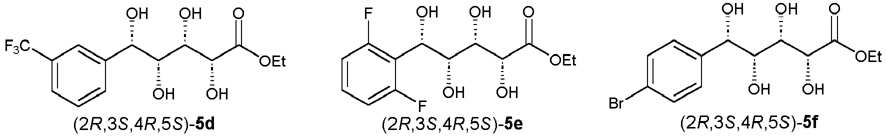 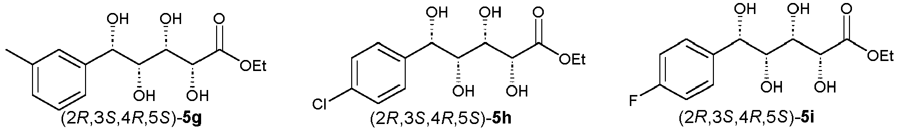 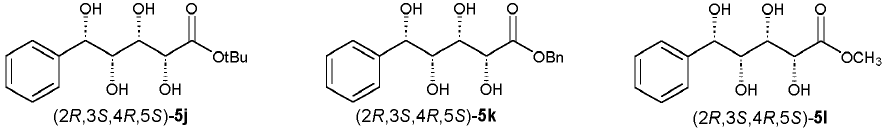 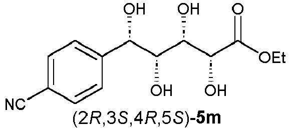
65. The method of any one of paragraphs 26-64, wherein the solvent is an alcohol, optionally a C₁-C₆ alcohol, such as methanol.
66. The method of any one of paragraphs 26-65 further comprising stirring the reaction mixture prior to and/or during step (i); or purifying the product, optionally by column chromatography, subsequent to step (i); or a combination thereof.

The present invention will be further understood by reference to the following non-limiting examples.

### Examples

### Example 1. Exemplary Fe(II) complexes catalyze asymmetric cis-dihydroxylation of styrenes and conjugated dienes

### Materials and Methods

Unsymmetrical tetradentate N₄ ligands including PQCN-n (n=1-8) were synthesized according to the synthesis of unsymmetric PDP ligands. Treatment of PQCN ligands with Fe(OTf)₂ in dry THF afforded the corresponding [Fe(*S*,*S*-PQCN-n)(OTf)₂] complexes in 60-70% yields. Crystals of [Fe(*S*,*S*-PQCN-1)(OTf)₂] were obtained by layering of hexane on the top of DCM.

### Synthesis of Iron Complexes

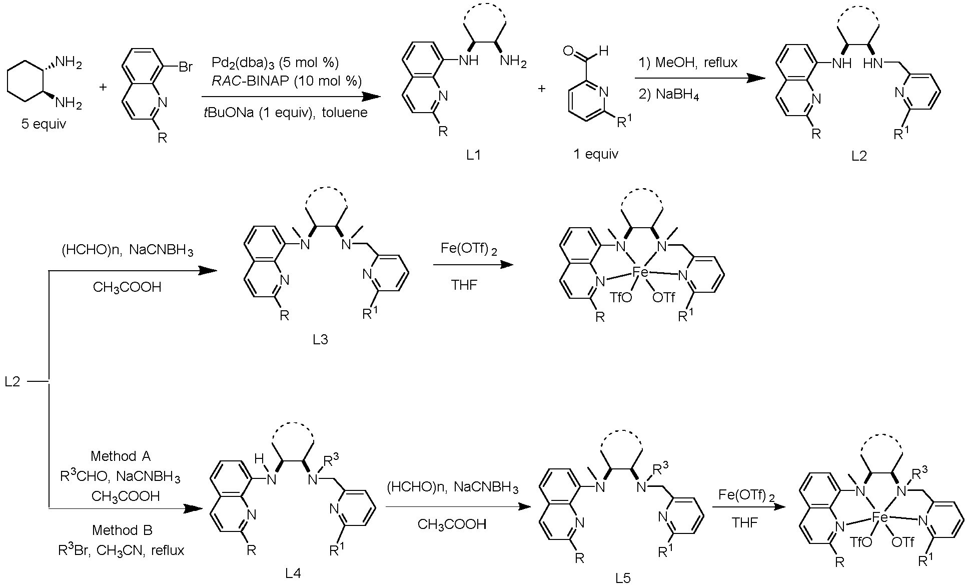

### General procedure for synthesis of L1

Under argon atmosphere, 8-bromo-2-R-quinoline (5 mmol), 1,2-diamine backbone (25 mmol), Pd₂(dba)₃ (5 mol %), RAC-BINAP (10 mol %) were dissolved in toluene, then sodium *t*-butoxide (5 mmol) was added, the mixture was stirred at 85 °C for 24 h. After being cooled to room temperature, the reaction mixture was filtered through a plug of silica and concentrated under reduced pressure. The crude residue was purified by flash chromatography on silica gel (EA/Methanol = 3:1 v/v) to afford L1.

### General procedure for synthesis of L2

Under argon atmosphere, a mixture of 6-methylpicolinaldehyde (1 equiv) and L1 (1 equiv) in methanol (0.2 M) was refluxed overnight. After being cooled to 0 °C, NaBH₄ (3 equiv) was added in portions. The reaction mixture was kept at 0°C for 3 h, after completion, the reaction was quenched by water. The aqueous layer was extracted with EtOAc. Finally, organic layers were concentrated and purified by flash chromatography on silica gel to afford L2.

### General procedure for synthesis of L3

0.5 mL CH₃COOH was added dropwise to s solution of L2 (2.5 mmol), 4.3 mL formaldehyde (37% wt. % in H₂O), NaCNBH₃ (1.75 g) in CH₃CN (25 mL) at 0 °C under air. After the mixture was stirred at room temperature overnight, the reaction was diluted with Et₂O (50 mL) and washed with aqueous NaHCO₃. (30 mL ×3). The organic fraction was combined and dried with NaSO₄, and concentrated under reduced pressure. The crude product was purified by flash chromatography on silica gel (Hexane/Et₃N= 100:1 v/v).

### General procedure for synthesis of L4 (Method A)

Under air, 0.5 mL CH₃COOH was added dropwise to a solution of L2 (2.5 mmol), aldehyde (2.5 mmol), NaCNBH₃ (1.75 g) in CH₃CN (25 mL) at 0 °C. After the mixture was stirred at room temperature overnight, the reaction was diluted with Et₂O (50 mL) and washed with aqueous NaHCO₃. (30 mL ×3). The organic fraction was combined and dried with NaSO₄, and concentrated under reduced pressure. The crude product was purified by flash chromatography on silica gel (hexane/Et₃N= 100:1 v/v).

### General procedure for synthesis of L4 (Method B)

K₂CO₃ (1.05 mmol) was added to a solution of L2 (1 mmol) in acetonitrile (60 mL), and the reaction was brought to refluxed. Then R³Br (1.05 mmol) was added dropwise. The mixture was refluxed overnight. When the reaction was complete as monitored by thin layer chromatography, the reaction was filtered through a short column of silica (4-5 cm), and the filtrate was evaporated to dryness. The crude product was purified by flash chromatography on silica gel (hexane/Et₃N=100:1 v/v).

The general procedure for synthesis of L5 was the same as synthesis of L3.

### General procedure for synthesis of C-1 symmetric Iron complexes

Under Argon, L3 (1 equiv) or L5 (1 equiv), and Fe(OTf)₂ (1 equiv) in THF (0.1 M) was stirred overnight. After reaction was completed, THF was removed under reduced pressure carefully under Argon. The crude residue was washed with dry Et₂O to give pure complex as yellow solids.

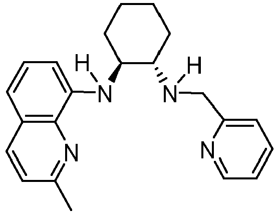

### (1S,2S)-N¹-(2-methylquinolin-8-yl)-N²-(pyridin-2-ylmethyl)cyclohexane-1,2-diamine

(Yellow oil, yield 86%) ¹H NMR (500 MHz, CD₂Cl₂) δ 8.54 (d, *J=* 4.7 Hz, 1H), 7.96 (d, *J=* 8.4 Hz, 1H), 7.60 (t, *J=* 7.6 Hz, 1H), 7.35 (t, *J=* 8.1 Hz, 2H), 7.26 (d, *J=* 8.4 Hz, 1H), 7.17 - 7.08 (m, 1H), 7.04 (d, *J* = 8.1 Hz, 1H), 6.87 (d, *J* = 7.7 Hz, 1H), 6.32 (d, *J* = 8.2 Hz, 1H), 4.06 (d, *J=* 14.5 Hz, 1H), 3.95 (d, *J=* 14.5 Hz, 1H), 3.48 (d, *J=* 6.9 Hz, 1H), 3.23 (s, 1H), 2.76 (s, 3H), 2.70 (td, *J* = 9.3, 3.8 Hz, 1H), 2.26 (d, *J* = 9.3 Hz, 2H), 1.90 - 1.69 (m, 2H), 1.50 - 1.24 (m, 4H). ¹³C NMR (125 MHz, CD₂Cl₂) δ 160.34, 155.52, 149.52, 144.76, 138.38, 136.65, 136.57, 127.34, 127.22, 122.54, 122.52, 122.10, 114.01, 105.95, 61.66, 57.42, 54.00, 32.47, 31.92, 26.10, 25.46, 25.07.HRMS (ESI): m/z ([M+H]⁺) calcd. for C₂₂H₂₇N₄ 347.2230, found 347.2230.

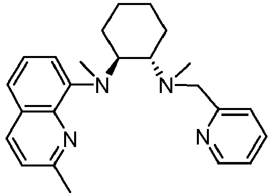

### (1S,2S)-N¹,N²-dimethyl-N¹-(2-methylquinolin-8-yl)-N²-(pyridin-2-ylmethyl)cyclohexane-1,2-diamine

(Yellow oil, yield 70%) ¹H NMR (400 MHz, CD₂Cl₂) δ 8.40 (d, *J=* 5.0 Hz, 1H), 7.92 (d, *J* = 8.4 Hz, 1H), 7.58 (td, *J* = 7.7, 1.8 Hz, 1H), 7.43 (d, *J* = 8.1 Hz, 1H), 7.32 (t, J = 7.8 Hz, 1H), 7.18 (d, *J* = 8.4 Hz, 1H), 7.15 (d, *J* = 8.1 Hz, 1H), 7.07 (d, *J* = 7.6 Hz, 1H), 4.71 (td, *J=* 11.0, 3.8 Hz, 1H), 3.87 (d, *J* = 14.1 Hz, 1H), 3.62 (d, *J* = 14.1 Hz, 1H), 3.01 (s, 3H), 2.84 (td, *J=* 10.8, 3.6 Hz, 1H), 2.64 (s, 3H), 2.39 - 2.26 (m, 1H), 2.00 (dt, *J* = 9.4, 2.9 Hz, 1H), 1.87 (s, 3H), 1.84 - 1.76 (m, 2H), 2.07-1.95 (m, 1H), 1.38 - 1.23 (m, 3H).¹³C NMR (101 MHz, CD₂Cl₂) δ 161.68, 155.31, 150.43, 148.87, 142.04, 136.82, 136.42, 128.32, 126.19, 123.42, 121.89, 121.55, 118.13, 115.70, 66.48, 62.73, 60.98, 36.38, 33.43, 30.47, 26.56, 26.34, 25.51, 24.21. HRMS (ESI): m/z ([M+H]⁺) calcd. for C₂₄H₃₁N₄ 375.2543, found 375.2543.

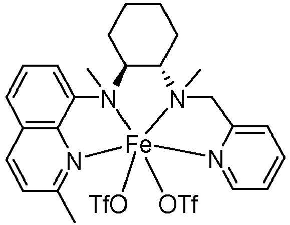

### (Yellow solid, yield 70%) HRMS (ESI): m/z ([M-2OTf]²⁺) calcd. for C₂₄H₃₀FeN₄ 215.0910, found 215.0906.

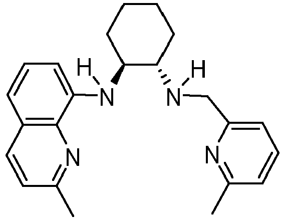

### (1S,2S)-N¹-((6-methylpyridin-2-yl)methyl)-N²-(2-methylquinolin-8-yl)cyclohexane-1,2-diamine

(Yellow oil, yield 80%) ¹H NMR (400 MHz, CDCl₃) δ 7.96 (d, *J* = 8.3 Hz, 1H), 7.51 (t, *J* = 7.7 Hz, 1H), 7.32 (t, *J* = 8.0 Hz, 1H), 7.26 (d, *J*= 8.4 Hz, 1H), 7.19 (d, *J*= 7.7 Hz, 1H), 7.00 (t, *J=* 8.9 Hz, 2H), 6.85 (d, *J* = 7.7 Hz, 1H), 6.19 (s, 1H), 4.06 (d, *J* = 14.4 Hz, 1H), 3.92 (d, *J=* 14.4 Hz, 1H), 3.55-3.41(m, 1H), 2.80-2.66 (m, 4H), 2.49 (s, 3H), 2.35 - 2.19 (m, 2H), 1.80 (d, *J* = 14.1 Hz, 2H), 1.53 - 1.26 (m, 4H). ¹³C NMR (101 MHz, CDCl₃) δ 159.39, 158.66, 155.49, 143.43, 137.97, 136.69, 136.24, 126.88, 125.65, 122.12, 120.51, 118.56, 111.54, 105.73, 60.83, 57.07, 52.56, 32.23, 31.51, 25.30, 24.65, 24.49.

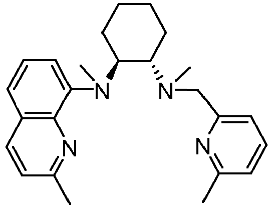

### (1S,2S)-N¹,N²-dimethyl-N¹-((6-methylpyridin-2-yl)methyl)-N²-(2-methylquinolin-8-yl)cyclohexane-1,2-diamine

(Yellow oil, yield 76%) ¹H NMR (500 MHz, CD₂Cl₂) δ 7.91 (d, *J=* 8.4 Hz, 1H), 7.46 (t, *J* = 7.7 Hz, 1H), 7.29 (t, *J* = 7.8 Hz, 1H), 7.19 (dd, *J* = 11.8, 8.0 Hz, 2H), 7.13 (d, *J* = 7.9 Hz, 1H), 7.05 (d, *J* = 7.7 Hz, 1H), 6.93 (d, *J* = 7.6 Hz, 1H), 4.66 (td, *J* = 11.1, 3.8 Hz, 1H), 3.76 (d, *J=* 14.2 Hz, 1H), 3.58 (d, *J=* 14.2 Hz, 1H), 2.99 (s, 3H), 2.82 (td, *J=* 10.8, 3.5 Hz, 1H), 2.63 (s, 3H), 2.43 (s, 3H), 2.36 - 2.28 (m, 1H), 2.06 - 1.91 (m, 1H), 1.86 - 1.74 (m, 5H), 1.68 (qd, *J* = 12.1, 3.6 Hz, 1H), 1.35 - 1.26 (m, 1H), 1.25 - 1.17 (m, 2H). HRMS (ESI): m/z ([M+H]⁺) calcd. for C₂₅H₃₃N₄ 389.2700, found389.2703.

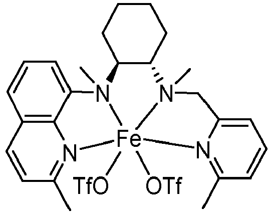

### (Yellow solid, yield 75%) Elemental analysis for C₂₇H₃₂F₆FeN₄O₆S₂ Calcd for C 43.67, H 4.34, N 7.55, S 8.64, found C, 45.20; H, 4.84; N, 7.53; S, 8.42

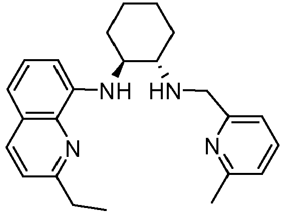

### (1S,2S)-N¹-(2-ethylquinolin-8-yl)-N²-((6-methylpyridin-2-yl)methyl)cyclohexane-1,2-diamine

(Yellow oil, yield 75%) ¹H NMR (500 MHz, CDCl₃) δ 7.98 (d, *J* = 8.3 Hz, 1H), 7.50 (t, *J=* 7.6 Hz, 1H), 7.37 - 7.24 (m, 2H), 7.17 (d, *J=* 7.6 Hz, 1H), 7.01 (dd, *J=* 18.9, 7.7 Hz, 2H), 6.86 (d, *J=* 7.6 Hz, 1H), 6.24 (d, *J=* 9.1 Hz, 1H), 4.06 (d, *J=* 14.3 Hz, 1H), 3.93 (d, *J=* 14.3 Hz, 1H), 3.49 (dd, *J=* 17.4, 10.7 Hz, 1H), 3.02 (q, *J=* 7.5 Hz, 2H), 2.78 - 2.68 (m, 1H), 2.48 (s, 3H), 2.25 (d, *J* = 14.6 Hz, 2H), 1.89 - 1.75 (m, 2H), 1.38 (ddd, *J*= 35.5, 20.2, 8.7 Hz, 7H).

¹³C NMR (125 MHz, CDCl₃) δ 160.38, 159.72, 157.82, 144.47, 137.88, 136.71, 136.34, 127.12, 126.85, 121.35, 121.09, 119.04, 113.74, 105.77, 61.61, 57.10, 52.58, 32.23, 31.93, 31.41, 25.24, 24.61, 24.44, 13.88. HRMS (ESI): m/z ([M+H]+) calcd. for C₂₄H₃₁N₄ 375.2543, found 375.2543.

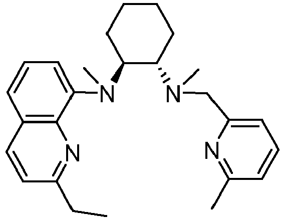

### (1S,2S)-N1-(2-ethylquinolin-8-yl)-N1,N2-dimethyl-N2-((6-methylpyridin-2-yl)methyl)cyclohexane-1,2-diamine

(Yellow oil, yield 69%)1H NMR (400 MHz, CD2Cl2) δ 7.96 (d, J = 8.4 Hz, 1H), 7.50 (t, J = 7.7 Hz, 1H), 7.38 (t, J = 7.8 Hz, 1H), 7.28 - 7.11 (m, 4H), 6.96 (d, J = 7.6 Hz, 1H), 4.88 (td, J = 11.0, 3.7 Hz, 1H), 3.83 (d, J = 14.2 Hz, 1H), 3.67 (d, J = 14.2 Hz, 1H), 3.07 (s, 3H), 2.99 (q, J = 7.6 Hz, 2H), 2.88 (td, J = 10.7, 3.3 Hz, 1H), 2.50 (s, 3H), 2.44 (d, J = 12.3 Hz, 1H), 2.08 - 1.98 (m, 1H), 1.86 (s, 5H), 1.80 - 1.71 (m, 1H), 1.43 (t, J = 7.6 Hz, 3H), 1.28 (t, J = 10.5 Hz, 1H). ¹³C NMR (100 MHz, CD₂Cl₂) δ 160.76, 160.20, 157.40, 150.46, 142.04, 137.20, 136.71, 128.59, 126.23, 121.23, 120.65, 120.17, 118.12, 115.58, 66.48, 62.50, 61.26, 36.09, 33.48, 32.73, 30.76, 26.68, 26.32, 24.60, 24.23, 14.31.

HRMS (ESI): m/z ([M+H]⁺) calcd. for C₂₆H₃₅N₄ 403.2856, found 403.2853.

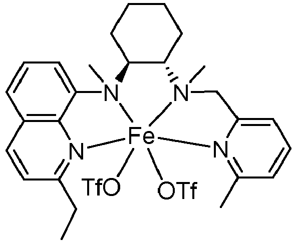

### (Yellow solid, yield 73%) HRMS (ESI): m/z ([M-2OTf]2+) calcd. for C26H34FeN4 229.1061, found 229.1061. Elemental analysis for C28H34F6FeN4O6S2 Calcd for C 44.45, H 4.53, N 7.41, S 8.48, found C 45.20, H 4.84, N 7.53, S 8.42.

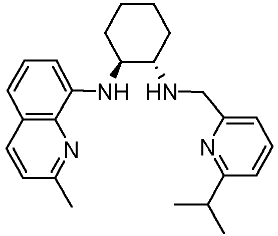

### (1S,2S)-N1-((6-isopropylpyridin-2-yl)methyl)-N2-(2-methylquinolin-8-yl)cyclohexane-1,2-diamine

(Yellow solid, yield 70%) 1HNMR (500 MHz, CD2Cl2) δ 7.95 (d, J = 8.4 Hz, 1H), 7.52 (t, J = 7.7 Hz, 1H), 7.31 (t, J = 7.9 Hz, 1H), 7.26 (d, J = 8.4 Hz, 1H), 7.13 (d, J = 7.6 Hz, 1H), 6.99 (t, J = 7.9 Hz, 2H), 6.85 (d, J = 7.6 Hz, 1H), 6.25 (d, J = 8.4 Hz, 1H), 4.01 (d, J = 14.2 Hz, 1H), 3.86 (d, J = 14.2 Hz, 1H), 3.46 (d, J = 6.9 Hz, 1H), 2.97 (s, 1H), 2.92 (dt, J = 13.8, 6.9 Hz, 1H), 2.72 (s, 3H), 2.70 - 2.65 (m, 1H), 2.29 - 2.14 (m, 2H), 1.79 (d, J = 15.6 Hz, 2H), 1.51 - 1.40 (m, 1H), 1.39 - 1.27 (m, 3H), 1.15 (d, J = 7.0 Hz, 6H). 13C NMR (125 MHz, CD2Cl2) δ 167.06, 160.02, 155.95, 144.88, 138.30, 137.06, 136.57, 127.39, 127.25, 122.53, 119.71, 118.85, 113.85, 105.75, 62.30, 57.53, 52.93, 36.66, 32.78, 32.04, 25.78, 25.47, 25.15, 22.82, 22.76. HRMS (ESI): m/z ([M+H]+) calcd. for C25H33N4 389.2700, found 389.2699.

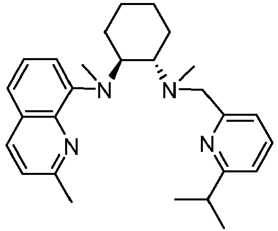

### (1S,2S)-N1-((6-isopropylpyridin-2-yl)methyl)-N1,N2-dimethyl-N2-(2-methylquinolin-8-yl)cyclohexane-1,2-diamine

(Yellow solid, yield 75%) 1HNMR (500 MHz, CD2Cl2) δ 7.93 (d, J = 8.3 Hz, 1H), 7.54 (t, J = 7.7 Hz, 1H), 7.34 (t, J = 7.8 Hz, 1H), 7.26 (d, J = 7.7 Hz, 1H), 7.18 (dd, J = 13.5, 8.2 Hz, 2H), 7.09 (d, J = 7.7 Hz, 1H), 6.99 (d, J = 7.6 Hz, 1H), 4.72 (td, J = 11.0, 3.4 Hz, 1H), 3.85 (d, J = 14.0 Hz, 1H), 3.67 (d, J = 14.0 Hz, 1H), 3.10 - 2.93 (m, 4H), 2.87 (t, J = 10.3 Hz, 1H), 2.69 (s, 3H), 2.39 (d, J = 12.0 Hz, 1H), 1.90-1.78 (d, J = 7.2 Hz, 1H), 1.91 (s, 3H), 1.83 (s, 2H), 1.78 - 1.66 (m, 1H), 1.43 - 1.16 (m, 9H). 13C NMR (126 MHz, CD2Cl2) δ 166.51, 160.58, 155.26, 150.57, 142.12, 136.81, 128.34, 126.26, 121.54, 120.64, 118.46, 118.10, 115.78, 66.32, 62.82, 61.40, 36.82, 36.27, 33.44, 30.66, 26.66, 26.41, 25.60, 24.21, 23.05, 23.04. HRMS (ESI): m/z ([M+H]+) calcd. for C27H37N4 417.3013, found 417.3013.

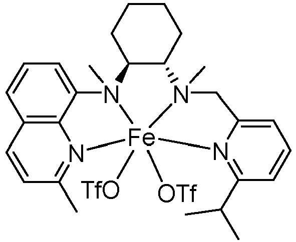

### (Yellow solid, yield 76%) HRMS (ESI): m/z ([M-2OTf]2+) calcd. for C27H36FeN4 236.1139, found 236.1134. Elemental analysis for C39H36F6FeN4O6S2 Calcd for C 45.20, H 4.71, N 7.27, S 8.32, found C 45.16, H 4.98, N 7.34, S 8.25.

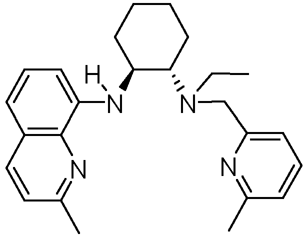

### (1S,2S)-N1-ethyl-N1-((6-methylpyridin-2-yl)methyl)-N2-(2-methylquinolin-8-yl)cyclohexane-1,2-diamine

(Yellow oil, yield 80%) 1H NMR (400 MHz,CD2Cl2) δ 7.95 (d, J = 8.3 Hz, 1H), 7.48 (d, J = 7.8 Hz, 1H), 7.35 - 7.18 (m, 3H), 7.11 (t, J = 7.7 Hz, 1H), 6.96 (d, J = 8.0 Hz, 1H), 6.82 (d, J = 7.5 Hz, 1H), 6.65 (d, J = 7.6 Hz, 1H), 3.84 (d, J = 15.2 Hz, 1H), 3.59 (d, J = 15.2 Hz, 1H), 3.21 (s, 1H), 2.94 - 2.81 (m, 4H), 2.77 (s, 3H), 2.57 (s, 3H), 2.40 (s, 3H), 2.14 (d, J = 12.4 Hz, 1H), 1.92 (d, J = 9.4 Hz, 2H), 1.78 (d, J = 14.7 Hz, 1H), 1.57 - 1.34 (m, 1H), 1.33 - 1.15 (m, 2H), 1.06 (t, J = 7.0 Hz, 3H). 13C NMR (101 MHz, CD2Cl2) δ 160.68, 156.90, 155.26, 144.97, 137.89, 136.57, 135.91, 127.30, 126.79, 122.33, 121.16, 119.84, 113.66, 104.65, 64.15, 56.46, 55.62, 44.75, 34.01, 26.57, 25.46, 25.29, 24.56, 24.53, 14.88. HRMS (ESI): m/z ([M+H]+) calcd. for C25H33N4 389.2700, found 389.2692.

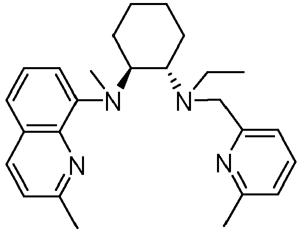

### (1S,2S)-N1-ethyl-N2-methyl-N1-((6-methylpyridin-2-yl)methyl)-N2-(2-methylquinolin-8-yl)cyclohexane-1,2-diamine

(Yellow oil, yield 70%) 1HNMR (500 MHz, CD2Cl2) δ 7.88 (d, J = 8.3 Hz, 1H), 7.37 (t, J = 7.6 Hz, 1H), 7.28 (t, J = 7.8 Hz, 1H), 7.16 (dd, J = 16.3, 8.0 Hz, 2H), 7.09 (d, J = 8.0 Hz, 1H), 6.99 (d, J = 6.4 Hz, 1H), 6.88 (d, J = 7.5 Hz, 1H), 4.92 (td, J = 10.9, 3.7 Hz, 1H), 3.96 (d, J = 14.4 Hz, 1H), 3.41 (d, J = 14.2 Hz, 1H), 2.98-2.84 (m, 4H), 2.60 (s, 3H), 2.42 (s, 3H), 2.37 (dd, J = 12.9, 6.7 Hz, 1H), 2.33 - 2.21 (m, 2H), 2.04 (dd, J = 10.1, 3.8 Hz, 1H), 1.83 - 1.75 (m, 2H), 1.69 (qd, J = 12.4, 3.6 Hz, 1H), 1.38 - 1.28 (m, 1H), 1.26 - 1.12 (m, 2H), 0.70 (t, J = 7.1 Hz, 3H). 13C NMR (125 MHz, CD2Cl2) δ 160.08, 156.59, 154.08, 148.73, 140.69, 128.87, 126.27, 121.43, 120.92, 119.93, 117.72, 115.42, 62.26, 57.15, 43.76, 32.94, 31.69, 26.71, 26.58, 25.45, 24.78, 22.93, 13.50. HRMS (ESI): m/z ([M+H]+) calcd. for C26H35N4 403.2856, found 403.2852.

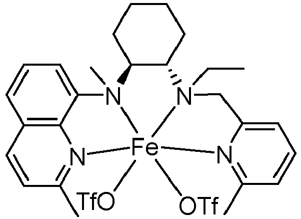

### (Yellow solid, yield 70%) HRMS (ESI): m/z ([M-2OTf]2+) calcd. for [C26H34FeN4]2+ 229.1061, found 229.1079.

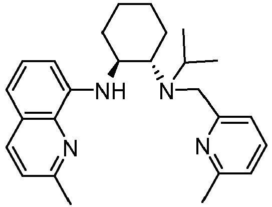

### (1S,2S)-N1-isopropyl-N1-((6-methylpyridin-2-yl)methyl)-N2-(2-methylquinolin-8-yl)cyclohexane-1,2-diamine

(Yellow oil, yield 65%) 1H NMR (400 MHz, CD2Cl2) δ 7.97 (d, J = 8.3 Hz, 1H), 7.47 (d, J = 7.8 Hz, 1H), 7.36 - 7.20 (m, 2H), 7.12 (s, 1H), 6.99 (d, J = 6.3 Hz, 2H), 6.80 (d, J = 7.5 Hz, 1H), 6.62 (d, J = 7.7 Hz, 1H), 4.04 - 3.67 (m, 2H), 3.19 - 2.98 (m, 2H), 2.87 (td, J = 10.8, 10.3, 2.9 Hz, 1H), 2.77 (s, 3H), 2.58 (d, J = 12.9 Hz, 1H), 2.42 (s, 3H), 2.17 (d, J = 17.7 Hz, 1H), 1.89 (d, J = 9.3 Hz, 1H), 1.76 (d, J = 12.7 Hz,1H), 1.59 (q, J = 12.3 Hz, 1H), 1.50 - 1.33 (m, 2H), 1.22 (d, J = 6.7 Hz, 3H), 1.14 (d, J = 6.5 Hz, 3H). 13C NMR (100 MHz, CD2Cl2) δ 162.15, 157.32, 155.70, 145.52, 138.62, 136.54, 127.31, 127.25, 122.31, 121.06, 119.69, 113.65, 105.16, 61.98, 56.15, 52.03, 49.79, 34.18, 29.44, 26.86, 25.37, 25.34, 24.56, 22.79, 19.74. HRMS (ESI): m/z ([M+H]+) calcd. for C26H35N4 403.2856, found 403.2854.

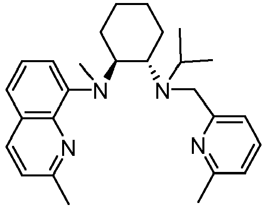

### (1S,2S)-N1-isopropyl-N2-methyl-N1-((6-methylpyridin-2-yl)methyl)-N2-(2-methylquinolin-8-yl)cyclohexane-1,2-diamine

(Yellow oil, yield 60%) 1H NMR (500 MHz, CD2Cl2) δ 7.87 (d, J = 8.3 Hz, 1H), 7.28 (t, J = 7.8 Hz, 1H), 7.22 (t, J = 7.5 Hz, 1H), 7.18 (d, J = 7.7 Hz, 1H), 7.13 (d, J = 8.4 Hz, 1H), 7.09 (d, J = 7.9 Hz, 1H), 6.89 (d, J = 7.8 Hz, 1H), 6.82 (d, J = 7.3 Hz, 1H), 5.03 - 4.84 (m, 1H), 4.01 (d, J = 14.7 Hz, 1H), 3.58 (d, J = 14.6 Hz, 1H), 3.04-2.94 (m, 1H), 2.88 (td, J = 10.8, 3.3 Hz, 1H), 2.78 (s, 3H), 2.58 (s, 3H), 2.43 (s, 3H), 2.14 (d, J = 9.6 Hz, 2H), 1.82 - 1.69 (m, 2H), 1.60 (qd, J = 12.2, 3.7 Hz, 1H), 1.36 - 1.12 (m, 4H), 0.96 (d, J = 6.5 Hz, 3H), 0.73 (d, J = 6.6 Hz, 3H). 13C NMR (125MHz, CD2Cl2) δ 162.94, 156.76, 154.66, 150.01, 141.91, 136.70, 136.11, 128.54, 126.31, 121.46, 121.38, 120.71, 117.47, 115.14, 62.47, 62.06, 52.21, 49.53, 33.60, 30.43, 29.13, 26.91, 26.62, 25.38, 24.62, 23.12, 21.03. HRMS (ESI): m/z ([M+H]+) calcd. for C27H37N4 417.3013, found 417.3015.

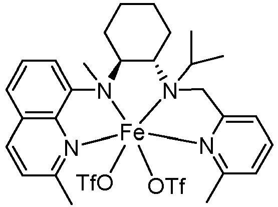

HRMS (ESI): m/z ([M-2OTf]2+) calcd. for [C27H36FeN4]2+ 236.1144, found 236.1151.

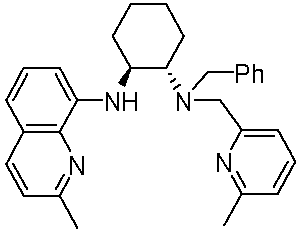

### (1S,2S)-N1-benzyl-N1-((6-methylpyridin-2-yl)methyl)-N2-(2-methylquinolin-8-yl)cyclohexane-1,2-diamine

(Yellow oil, yield 67%) 1HNMR (400 MHz, CD2Cl2) δ 7.99 (d, J = 8.3 Hz, 1H), 7.57 (d, J = 7.8 Hz, 1H), 7.51 (d, J = 6.7 Hz, 2H), 7.35 (d, J = 8.4 Hz, 1H), 7.21 (td, J = 10.8, 10.4, 7.4 Hz, 5H), 6.98 (d, J = 9.3 Hz, 1H), 6.86 (d, J = 7.6 Hz, 1H), 6.64 (d, J = 7.6 Hz, 1H), 3.82 (dd, J = 20.3, 14.0 Hz, 2H), 3.59 (dd, J = 31.8, 14.0 Hz, 2H), 3.33 (tt, J = 10.4, 3.3 Hz, 1H), 2.89 (s, 3H), 2.81 - 2.69 (m, 1H), 2.53 (dt, J = 13.0, 2.6 Hz, 1H), 2.41 (s, 3H), 2.28 (d, J = 11.0 Hz, 1H), 1.91 (d, J = 13.0 Hz, 1H), 1.73 (d, J = 13.5 Hz, 1H), 1.57 - 1.38 (m, 2H), 1.33 - 1.23 (m, 1H), 1.18 - 1.02 (m, 1H). 13C NMR (100 MHz, CD2Cl2) δ 160.62, 157.88, 155.78, 144.67, 141.40, 138.50, 136.77, 136.69, 129.59, 128.67, 127.43, 127.37, 127.29, 122.45, 121.08, 119.72, 113.30, 106.39, 62.72, 55.79, 55.46, 54.46, 34.16, 26.38, 25.48, 25.25, 24.56, 23.79.

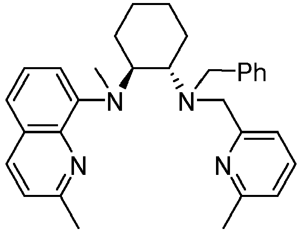

1H NMR (400 MHz, Methylene Chloride-d2) δ 7.90 (d, J = 8.4 Hz, 1H), 7.35 (t, J = 7.9 Hz, 2H), 7.31 - 7.19 (m, 5H), 7.16 (t, J = 7.4 Hz, 3H), 7.00 (d, J = 7.8 Hz, 1H), 6.89 (d, J = 7.5 Hz, 1H), 5.10 (td, J = 10.9, 3.6 Hz, 1H), 4.20 (d, J = 13.7 Hz, 1H), 3.90 (d, J = 12.9 Hz, 1H), 3.51 (dd, J = 17.4, 13.4 Hz, 2H), 2.83 (td, J = 10.9, 3.2 Hz, 1H), 2.69 (s, 3H), 2.60 (s, 3H), 2.46 (s, 3H), 2.29 (d, J = 15.3 Hz, 1H), 2.05 (d, J = 15.4 Hz, 1H), 1.84 (d, J = 18.1 Hz, 1H), 1.75 (d, J = 15.2 Hz, 1H), 1.54 (q, J = 14.0, 12.2 Hz, 1H), 1.46 - 1.18 (m, 3H). 13C NMR (100 MHz, CD2Cl2) δ 161.46, 156.99, 155.03, 149.94, 142.01, 140.97, 136.84, 136.46, 130.40, 128.55, 128.29, 127.12, 126.34, 121.55, 121.17, 121.09, 118.10, 115.83, 62.07, 60.81, 56.39, 54.76, 33.47, 29.63, 26.57, 26.48, 25.31, 24.74, 24.61. HRMS (ESI): m/z ([M+H]+) calcd. for C31H37N4 465.3013, found 465.3018.

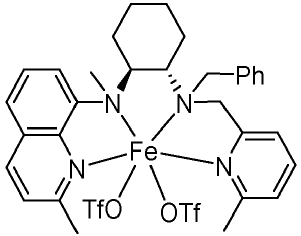

(Yellow solid , yield 75%) HRMS (ESI): m/z ([M-2OTf]2+) calcd. for [C31H36FeN4]2+ 260.1145, found 260.1141. Elemental Analysis for C33H36F6FeN4O6S2 [Fe(S,S-PQCN-7)(OTf)2]•H2O, Calcd. C, 47.38; N, 6.70 H, 4.58; S, 7.66. Found: C, 47.80; N,6.36; H,4.89; S, 7.90.

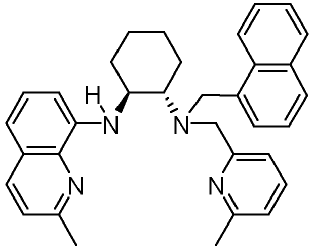

### (1S,2S)-N1-((6-methylpyridin-2-yl)methyl)-N2-(2-methylquinolin-8-yl)-N1-(naphthalen-1-ylmethyl)cyclohexane-1,2-diamine

(Yellow oil, yield 80%) ¹H NMR (500 MHz, Methylene Chloride-*d*₂) δ 8.24 (d, *J* = 8.5 Hz, 1H), 8.03 (d, *J* = 8.4 Hz, 1H), 7.80 (dd, *J* = 14.2, 8.3 Hz, 2H), 7.71 (d, *J* = 8.2 Hz, 1H), 7.53 (d, *J* = 7.8 Hz, 1H), 7.34 (dt, *J* = 24.0, 8.2 Hz, 5H), 7.11 (d, *J* = 4.2 Hz, 1H), 7.03 (d, *J* = 9.3 Hz, 1H), 6.98 (t, *J* = 7.8 Hz, 1H), 6.89 (d, *J* = 7.6 Hz, 1H), 6.71 (d, *J* = 7.9 Hz, 1H), 4.27 (d, *J* = 13.6 Hz, 1H), 4.18 (d, *J* = 13.6 Hz, 1H), 3.95 (d, *J* = 14.3 Hz, 1H), 3.82 (d, *J* = 14.3 Hz, 1H), 3.57 (ddd, *J* = 14.4, 9.1, 3.9 Hz, 1H), 2.96 - 2.87 (m, 1H), 2.84 (s, 3H), 2.57 (d, *J* = 14.0 Hz, 1H), 2.46 (s, 3H), 2.42 (d, *J* = 12.4 Hz, 1H), 1.96 (d, *J* = 13.2 Hz, 1H), 1.76 (d, *J* = 12.4 Hz, 1H), 1.73 - 1.60 (m, 1H), 1.57 - 1.45(m, 1H), 1.36 - 1.24 (m, 1H), 1.20 - 1.07 (m, 1H). ¹³C NMR (125 MHz, CD₂Cl₂) δ 160.58, 157.49, 155.72, 144.65, 138.51, 136.74, 136.64, 135.67, 134.23, 133.06, 128.76, 128.24, 128.02, 127.49, 127.34, 126.10, 125.86, 125.71, 124.70, 122.49, 121.41, 120.43, 113.61, 104.86, 63.70, 56.74, 54.89, 51.85, 33.64, 26.43, 25.63, 25.35, 24.60, 24.32. HRMS (ESI): m/z ([M+H]⁺) calcd. for C₃₄H₃₇N₄ 501.3013, found 501.3030.

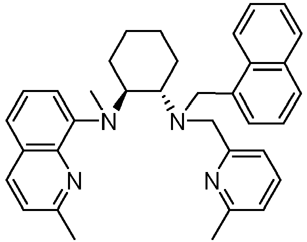

### (1S,2S)-N'-methyl-N²-((6-methylpyridin-2-yl)methyl)-N¹-(2-methylquinolin-8-yl)-N²-(naphthalen-1-ylmethyl)cyclohexane-1,2-diamine

(Yellow oil, yield 70%) ¹H NMR (400 MHz, Methylene Chloride-*d*₂) δ 8.06 (d, *J* = 8.6 Hz, 1H), 7.89 (d, *J* = 8.3 Hz, 1H), 7.78 (d, *J* = 8.2 Hz, 1H), 7.73 (d, *J =* 8.2 Hz, 1H), 7.49 (d, *J* = 6.9 Hz, 1H), 7.39 (td, *J* = 7.3, 3.9 Hz, 2H), 7.27 (tt, *J* = 11.7, 7.7 Hz, 3H), 7.17 (d, *J* = 7.9 Hz, 1H), 7.12 (d, *J* = 8.4 Hz, 1H), 6.88 (d, *J* = 7.5 Hz, 1H), 6.82 (d, *J* = 7.7 Hz, 1H), 6.77 (d, *J* = 7.7 Hz, 1H), 5.17 (td, *J* = 10.9, 3.6 Hz, 1H), 4.59 (d, *J* = 13.0 Hz, 1H), 4.30 (d, *J* = 12.9 Hz, 1H), 3.92 (d, *J* = 13.0 Hz, 1H), 3.49 (d, *J* = 12.8 Hz, 1H), 2.83 (td, *J* = 10.9, 3.2 Hz, 1H), 2.52 (s, 3H), 2.46 - 2.37 (m, 4H), 2.27 (s, 3H), 1.93 - 1.75 (m, 2H), 1.68 (d, *J* = 15.5 Hz, 1H), 1.50 - 1.31 (m, 2H), 1.29 - 1.02 (m, 2H).¹³C NMR (100 MHz, CD₂Cl₂) δ 161.07, 156.89, 155.10, 149.73, 142.02, 136.86, 136.39, 136.27, 134.28, 133.62, 128.99, 128.50, 128.41, 128.03, 126.61, 126.30, 125.86, 125.56, 125.50, 122.04, 121.58, 121.20, 118.31, 116.38, 62.02, 60.79, 56.72, 52.74, 32.97, 29.31, 26.53, 26.47, 25.21, 24.61, 24.23. HRMS (ESI): m/z ([M+H]⁺) calcd. for C₃₅H₃₉N₄ 515.3169, found 515.3172.

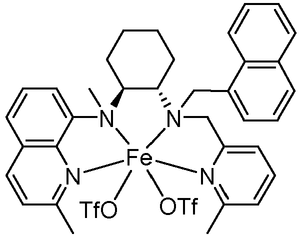

### (Yellow oil, yield 74%) HRMS (ESI): m/z ([M-2OTf]2+) calcd. for [C35H38FeN4]2+ 285.1217, found 285.1201.

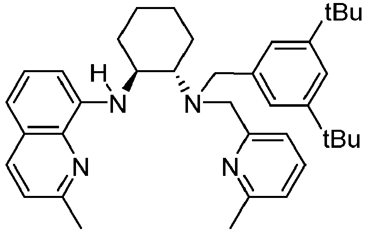

### (1S,2S)-N¹-(3,5-di-tert-butylbenzyl)-N¹-((6-methylpyridin-2-yl)methyl)-N²-(2-methylquinolin-8-yl)cyclohexane-1,2-diamine

(Yellow oil, yield 65%) ¹H NMR (400 MHz, Methylene Chloride-*d*₂) δ 7.97 (d, *J* = 8.4 Hz, 1H), 7.71 (d, *J* = 7.7 Hz, 1H), 7.53 (t, *J* = 7.7 Hz, 1H), 7.29 (dd, *J* = 19.1, 8.1 Hz, 2H), 7.14 (d, *J* = 9.8 Hz, 3H), 6.95 (dd, *J* = 12.9, 6.1 Hz, 3H), 6.66 (d, *J* = 7.6 Hz, 1H), 3.96 (d, *J* = 13.1 Hz, 1H), 3.78 (q, *J* = 14.7 Hz, 2H), 3.56 (td, *J* = 10.4, 5.2 Hz, 1H), 3.46 (d, *J* = 13.1 Hz, 1H), 2.89 - 2.73 (m, 4H), 2.59 - 2.41 (m, 4H), 2.30 (d, *J* = 12.6 Hz, 1H), 1.92 (d, *J* = 9.9 Hz, 1H), 1.74 (d, *J* = 12.5 Hz, 1H), 1.59 - 1.40 (m, 1H), 1.34 - 1.21 (m, 1H), 1.18 - 0.09 (s, 19H). ¹³C NMR (100 MHz, CD₂Cl₂) δ 160.88, 157.57, 155.77, 150.94, 144.51, 139.51, 138.49, 137.00, 136.66, 127.67, 127.30, 123.94, 122.57, 121.46, 120.82, 120.13, 113.35, 104.47, 63.21, 56.61, 54.54, 54.27, 54.12, 54.00, 53.73, 53.46, 46.82, 34.96, 33.36, 31.59, 26.40, 25.96, 25.39, 24.66, 24.10, 12.18. HRMS (ESI): m/z ([M+H]⁺) calcd. for C₃₈H₅₁N₄ 563.4108, found 563.4115.

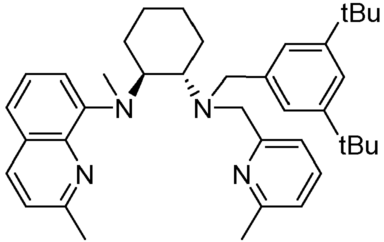

### (1S,2S)-N¹-(3,5-di-tert-butylbenzyl)-N²-methyl-N¹-((6-methylpyridin-2-yl)methyl)-N²-(2-methylquinolin-8-yl)cyclohexane-1,2-diamine

(Yellow oil, yield 50%) ¹H NMR (500 MHz, Methylene Chloride-*d*₂) δ 7.85 (d, *J* = 8.3 Hz, 1H), 7.35 - 7.22 (m, 3H), 7.18 - 7.06 (m, 5H), 6.92 (d, *J* = 9.1 Hz, 1H), 6.84 (d, *J* = 7.4 Hz, 1H), 5.10 (td, *J* = 11.0, 3.7 Hz, 1H), 4.28 (d, *J* = 13.7 Hz, 1H), 3.82 (d, *J* = 12.7 Hz, 1H), 3.47 (dd, *J* = 13.3, 6.2 Hz, 2H), 2.79 (td, *J* = 11.0, 3.2 Hz, 1H), 2.60 (s, 3H), 2.52 (s, 3H), 2.40 (s, 3H), 2.31 - 2.21 (m, 1H), 1.94 (d, *J* = 12.1 Hz, 1H), 1.81 (d, *J* = 10.1 Hz, 1H), 1.70 (d, *J* = 12.9 Hz, 1H), 1.48 (dd, *J* = 24.1, 3.7 Hz, 1H), 1.34 - 1.25 (m, 21H). ¹³C NMR (125 MHz, CD₂Cl₂) δ 161.51, 156.84, 155.04, 150.55, 149.83, 142.01, 139.73, 136.81, 136.31, 128.49, 126.27, 124.85, 121.55, 121.14, 120.93, 120.85, 118.08, 115.87, 62.12, 60.95, 56.40, 55.55, 35.15, 33.34, 31.82, 29.56, 26.63, 26.59, 25.21, 24.84, 24.61. HRMS (ESI): m/z ([M+H]⁺) calcd. for C₃₉H₅₃N₄ 577.4265, found 577.4287.

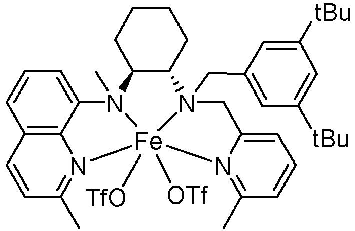

### (Yellow solid, yield 30%) HRMS (ESI): m/z ([M-2OTf]²⁺) calcd. for [C₃₉H₅₂FeN₄]²⁺ 316.1766, found 316.1798.

### Characterization of Diols of Styrenes

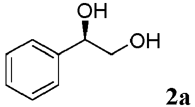

### (R)-1-phenylethane-1,2-diol

(colourless oil, 47.0, 85% yield) ¹H NMR (400 MHz, CDCl₃)δ 7.41 - 7.26 (m, 5H), 4.78 (d, *J* = 5.0 Hz, 1H), 3.77 - 3.54 (m, 2H), 3.36 (brs, 1H), 3.05 (brs, 1H). ¹³C NMR (100 MHz, CDCl₃) δ 140.55, 128.64, 128.09, 126.18, 74.82, 68.15. HPLC (Chiralcel OD-3, n-hexane/2-propanol = 93/7, flow rate: 1 ml/min, detection at 210 nm) retention time: t = 16.2 min (majo) and 25.7 min (minor), 82.9% ee. [α]²⁰_{D}=-41.0 (c=1.4, CHCl₃)

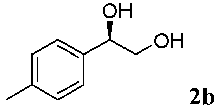

### (R)-1-(p-tolyl)ethane-1,2-diol

(white solid, 33 mg, 54% yield) ¹H NMR (500 MHz,CDCl₃) δ 7.22 (d, *J* = 8.1 Hz, 2H), 7.15 (d, *J* = 7.9 Hz, 2H), 4.75 (dd, *J* = 8.3, 3.5 Hz, 1H), 3.80 - 3.51 (m, 2H), 2.33 (s, 3H). ¹³C NMR (125 MHz, CDCl₃) δ 137.82, 129.32, 126.15, 74.69, 68.17, 21.24. HRMS (ESI): m/z ([M+Na]¹⁺) calcd. for C₉H₁₂NaO₂ 175.0730, found 175.0734. HPLC (Chiralcel OD-3, n-hexane/2-propanol = 90/10, flow rate: 1 ml/min, detection at 210 nm) retention time: t = 8.7 min (major) and 9.7 min (minor), 84.1% ee. [α]²⁰_{D}=-44.4 (c=1.0, CHCl₃)

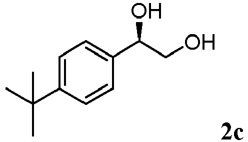

### (R)-1-(4-(tert-butyl)phenyl)ethane-1,2-diol

(white solid, 52.1 mg, 60% yield) ¹H NMR (500 MHz, MeOD) δ 7.37 (s, 2H), 7.29 (d, *J* = 7.9 Hz, 2H), 4.66 (t, *J* = 5.9 Hz, 1H), 3.60 (d, *J* = 5.9 Hz, 2H), 1.31 (s, 9H). ¹³C NMR (125 MHz, MeOD) δ 151.53, 140.22, 127.16, 126.12, 75.76, 68.73, 35.30, 31.80. HRMS (ESI): m/z ([M+Na]¹⁺) calcd. for C₁₂H₁₈NaO₂ 217.1199, found 2217.1202. HPLC (Chiralcel OD-3, n-hexane/2-propanol = 95/5, flow rate: 1 ml/min, detection at 230 nm) retention time: t = 15.8 min (major) and 17.3min (minor), 82.7% ee. [α]²⁰_{D}=-36.2 (c=1.0, CHCl₃)

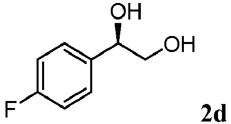

### (R)-1-(4-fluorophenyl)ethane-1,2-diol

(white solid, 40.6 mg, 65% yield) ¹H NMR (400 MHz, CDCl₃) δ 7.37 - 7.29 (m, 2H), 7.10 - 6.99 (m, 2H), 4.81 (dd, *J* = 8.2, 3.4 Hz, 1H), 3.74 (dd, *J* = 11.3, 3.4 Hz, 1H), 3.62 (dd, *J* = 11.3, 8.3 Hz, 1H), 2.83 (s, 1H), 2.35 (s, 1H).

¹³C NMR (100 MHz, CDCl₃) δ 163.82, 161.37, 136.34, 127.93, 127.85, 115.67, 115.45, 74.16, 68.18. HRMS (ESI): m/z ([M+Na]¹⁺) calcd. for C₈H₉FNaO₂ 179.0479, found 179.0482. HPLC (Chiralcel OD-3, n-hexane/2-propanol = 95/5, flow rate: 1 ml/min, detection at 210 nm) retention time: t = 22.7 min (major) and 25.8 min (minor), 80.1% ee. [α]¹⁹_{D}=-38.6 (c=3.9, CHCl₃)

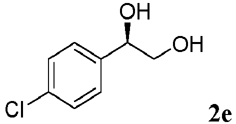

### (R)-1-(4-chlorophenyl)ethane-1,2-diol

(white solid, 46 mg, 67% yield)¹H NMR (400 MHz, CDCl₃) δ 7.36 - 7.24 (m, 4H), 4.79 (dd, *J* = 8.1, 3.3 Hz, 1H), 3.73 (dd, *J* = 11.3, 3.3 Hz, 1H), 3.60 (dd, *J* = 11.3, 8.2 Hz, 1H). ¹³C NMR (100 MHz, CDCl₃) δ 139.02, 133.87, 128.83, 127.58, 74.13, 68.04. HRMS (ESI): m/z ([M+Na]¹⁺) calcd. for C₈H₉ClNaO₂ 195.0183, found 195.0183. HPLC (Chiralcel OD-3, n-hexane/2-propanol = 90/10, flow rate: 1 ml/min, detection at 210 nm) retention time: t = 9.5 min (major) and 10.8 min (minor), 84.1% ee. [α]²⁰_{D}=-38.3 (c=1, CHCl₃)

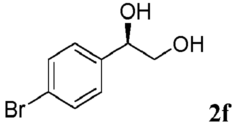

### (R)-1-(4-bromophenyl)ethane-1,2-diol

(white solid, 53.5 mg, 62% yield) ¹H NMR (500 MHz, MeOD) δ 7.48 (d, *J* = 7.7 Hz, 2H), 7.30 (d, *J* = 7.7 Hz, 2H), 4.66 (t, *J* = 5.8 Hz, 1H), 3.67 - 3.55 (m, 2H). ³C NMR (125 MHz, MeOD) δ 142.71, 132.26, 129.37, 122.07, 75.14, 68.42. HRMS (ESI): m/z ([M+Na]¹⁺) calcd. for C₈H₉BrNaO₂ 238.9678, found 238.9676. HPLC (Chiralcel OD-H, n-hexane/2-propanol = 90/10, flow rate: 1 ml/min, detection at 210 nm) retention time: t = 10.2 min (major) and 11.1 min (minor), 86.3% ee. [α]¹⁹_{D}=-30.5 (c=3.3, CHCl₃)

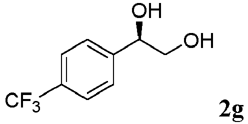

### (R)-1-(4-(trifluoromethyl)phenyl)ethane-1,2-diol

(white solid, 62.6 mg, 76% yield) ¹H NMR (400 MHz, Chloroform-*d*) δ 7.63 (d, *J* = 8.0 Hz, 2H), 7.50 (d, *J* = 8.0 Hz, 2H), 4.90 (dd, *J* = 8.1, 3.4 Hz, 1H), 3.81 (dd, *J* = 11.3, 3.5 Hz, 1H), 3.65 (dd, *J* = 11.2, 8.0 Hz, 1H), 2.72 (s, 1H), 2.10 (s, 1H). ¹³C NMR (125 MHz, MeOD) δ 148.12, 130.57 (q, *J* = 32.0 Hz), 129.01, 126.04 (q, *J* = 3.8 Hz), 125.78 (q, *J* = 270.8 Hz), 75.21, 68.45. ¹⁹F NMR (471 MHz, MeOD) δ -63.92. HRMS (ESI): m/z ([M+Na]¹⁺) calcd. for C₉H₉F₃O₂Na 229.0447, found 229.0451. HPLC (Chiralcel OD-3, n-hexane/2-propanol = 95/5, flow rate: 1 ml/min, detection at 210 nm) retention time: t = 48.5 min (major) and 52.9 min (minor), 88.5% ee. [α]²⁰_{D}=-40.0 (c=1.4, CHCl₃)

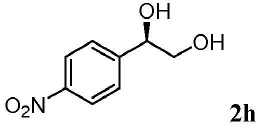

### (R)-1-(4-nitrophenyl)ethane-1,2-diol

(white solid, 61.6 mg, 72% yield) ¹H NMR (500 MHz, MeOD) δ 8.21 (d, *J* = 7.8 Hz, 2H), 7.63 (d, *J* = 8.0 Hz, 2H), 4.81 (t, *J* = 5.7 Hz, 1H), 3.71 - 3.59 (m, 2H). ¹³C NMR (100 MHz, MeOD) δ 151.43, 148.68, 128.49, 124.24, 74.94, 68.27. HRMS (ESI): m/z ([M+H]¹⁺) calcd. for C₈H₁₀NO₄ 184.0604, found 184.0609. HPLC (Chiralcel OD-3, n-hexane/2-propanol = 90/10, flow rate: 1 ml/min, detection at 210 nm) retention time: t = 20.1 min (major) and 23.3 min (minor), 89.9% ee. [α]²⁰_{D}=-30.4 (c=0.4, CHCl₃)

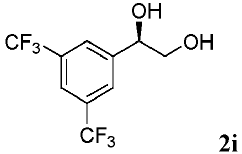

### (R)-1-(3,5-bis(trifluoromethyl)phenyl)ethane-1,2-diol

(white solid, 107.5 mg, 98% yield) ¹H NMR (400 MHz,CDCl₃) δ 7.86 (s, 2H), 7.83 (s, 1H), 4.97 (dd, *J* = 8.1, 3.4 Hz, 1H), 3.88 (dd, *J* = 11.1, 3.4 Hz, 1H), 3.66 (dd, *J* = 11.1, 7.8 Hz, 1H), 2.84 (s, 1H), 2.05 (s, 1H).¹³C NMR (100 MHz,MeOD) δ 147.31, 132.43 (q, *J* = 33.1 Hz), 128.02, 124.95 (q, *J* = 271.7 Hz), δ 121.98 (t, *J* = 4.0 Hz), 73.36, 68.64. ¹⁹F NMR (377 MHz, MeOD) δ -64.33. HRMS (EI): m/z ([M-F]¹⁺) calcd. for 255.0444 found 255.0435. HPLC (Chiralcel OD-3, n-hexane/2-propanol = 95/5, flow rate: 1 ml/min, detection at 210 nm) retention time: t = 5.2 min (major) and 5.8 min (minor), 94.5% *ee.* [α]²⁰_{D}=-24.6 (c=0.4, CHCl₃)

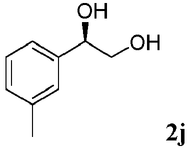

### (R)-1-(m-tolyl)ethane-1,2-diol

(white solid, 34.7 mg, 57% yield) ¹H NMR (500 MHz, CDCl₃) δ 7.24 (dd, *J* = 15.2, 7.6 Hz, 1H), 7.18 - 7.07 (m, 3H), 4.76 (dd, *J* = 8.3, 3.0 Hz, 1H), 3.71 (dd, *J* = 11.4, 3.1 Hz, 1H), 3.67 - 3.58 (m, 1H), 3.44 (brs, 1H), 3.08 (brs, 1H), 2.35 (s, 3H). ¹³C NMR (126 MHz, CDCl₃) δ 140.56, 138.31, 128.81, 128.54, 126.86, 123.24, 74.85, 68.18, 21.53. HRMS (ESI): m/z ([M+Na]1+) calcd. for C9H12NaO2 175.0730, found 175.0730. HPLC (Chiralcel AY-H, n-hexane/2-propanol = 95/5, flow rate: 1 ml/min, detection at 220 nm) retention time: t = 23.9 min (minor) and 25.7 min (major), 84.6% ee. [α]²⁰_{D}=-40.0 (c=1.4, CHCl₃)

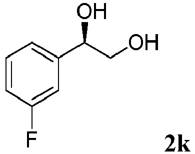

### (R)-1-(3-fluorophenyl)ethane-1,2-diol

(white solid, 40.6 mg, 65% yield) ¹H NMR (400 MHz, CDCl₃) δ 7.35 - 7.20 (m, 1H), 7.11 - 7.01 (m, 2H), 7.00 - 6.90 (m, 1H), 4.76 (dd, *J* = 8.3, 3.2 Hz, 1H), 3.92 - 3.63 (m, 2H), 3.58 (dd, *J* = 11.5, 8.3 Hz, 1H), 3.31 (s, 1H). ¹³C NMR (100 MHz, CDCl₃) δ 163.04 (d, *J* = 246.2 Hz), 143.20 (d, *J* = 6.9 Hz), 130.17 (d, *J* = 8.2 Hz), 121.71 (d, *J* = 2.9 Hz), 114.90 (d, *J* = 21.1 Hz), 113.16 (d, *J* = 22.0 Hz), 74.18 (d, *J* = 1.8 Hz), 67.91. HRMS (ESI): m/z ([M+Na]¹⁺) calcd. for C₈H₉FNaO₂ 179.0479, found 179.0475. HPLC (Chiralcel OD-3, n-hexane/2-propanol = 90/10, flow rate: 1 ml/min, detection at 210 nm) retention time: t = 8.8 min (major) and 9.9 min (minor), 85.1% ee. [α]²⁰_{D}=-36.5 (c=1.0, CHCl₃)

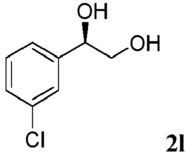

### (R)-1-(3-chlorophenyl)ethane-1,2-diol

(white solid, 55 mg, 80% yield) ¹H NMR (500 MHz, CDCl₃) δ 7.30 (s, 1H), 7.27 - 7.19 (m, 2H), 7.14 (d, *J* = 4.3 Hz, 1H), 4.71 (dd, *J* = 8.2, 2.3 Hz, 1H), 4.36 (s, 1H), 3.91 (s, 1H), 3.65 (dd, *J* = 11.5, 2.6 Hz, 1H), 3.53 (dd, *J* = 11.2, 8.8 Hz, 1H). 13C NMR (125 MHz, CDCl₃) δ 142.60, 134.48, 129.90, 128.12, 126.33, 124.30, 74.14, 67.80. HRMS (ESI): m/z ([M+Na]¹⁺) calcd. for C₈H₉ClNaO₂ 195.0183, found 195.0183. HPLC (Chiralcel OD-3, n-hexane/2-propanol = 90/10, flow rate: 1 ml/min, detection at 220 nm) retention time: t = 8.9 min (major) and 10.0 min (minor), 85.8% ee. [α]²⁰_{D}=-34.6 (c=2.7, CHCl₃)

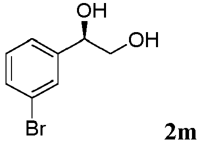

### (R)-1-(3-bromophenyl)ethane-1,2-diol

(white solid, 62.2 mg, 72% yield) ¹H NMR (500 MHz, CDCl₃) δ 7.46 (s, 1H), 7.38 (d, *J* = 7.4 Hz, 1H), 7.22 - 7.11 (m, 2H), 4.75 - 4.63 (m, 1H), 4.36 (s, 1H), 3.94 (s, 1H), 3.64 (dd, *J* = 11.5, 2.3 Hz, 1H), 3.58 - 3.46 (m, 1H). ¹³C NMR (125 MHz, CDCl₃) δ 142.85, 131.05, 130.20, 129.24, 124.79, 122.70, 74.06, 67.80. HRMS (ESI): m/z ([M+Na]¹⁺) calcd. for C₈H₉BrNaO₂ 238.9678, found 238.9682. HPLC (Chiralcel OD-3, n-hexane/2-propanol = 90/10, flow rate: 1 ml/min, detection at 210 nm) retention time: t = 9.2 min (major) and 10.7 min (minor), 84.1% ee. [α]²⁰_{D}=-24.6 (c=0.6, CHCl₃)

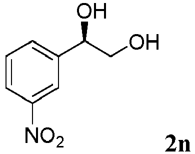

### (R)-1-(3-nitrophenyl)ethane-1,2-diol

(white solid, 58.6 mg, 80% yield)¹H NMR (500 MHz, MeOD) δ 8.29 (s, 1H), 8.13 (d, *J* = 8.1 Hz, 1H), 7.78 (d, *J* = 7.6 Hz, 1H), 7.58 (t, *J* = 7.9 Hz, 1H), 4.81 (t, *J* = 5.6 Hz, 1H), 3.73 - 3.61 (m, 2H). ¹³C NMR (125 MHz, MeOD) δ 149.60, 146.15, 133.81, 130.35, 123.21, 122.22, 74.64, 68.23. HRMS (ESI): m/z ([M+Na]¹⁺) calcd. for C₈H₉NNaO₄ 206.0424, found 206.0421. HPLC (Chiralcel OD-3, n-hexane/2-propanol = 90/10, flow rate: 1 ml/min, detection at 210 nm) retention time: t = 16.1 min (major) and 18.5 min (minor), 91.7% ee. [α]¹⁸_{D}=-59.3 (c=0.6, CHCl₃)

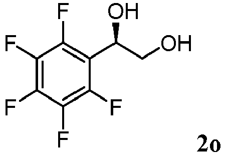

### (R)-1-(perfluorophenyl)ethane-1,2-diol

(white solid, 80.3 mg, 88% yield) ¹H NMR (500 MHz, CH₃OD) δ 5.08 (t, *J* = 6.9 Hz, 1H), 3.90 (dd, *J* = 11.1, 7.0 Hz, 1H), 3.76 (dd, *J* = 11.1, 6.8 Hz, 1H). ¹³C NMR(500 MHz, CH₃OD) δ 148.15-147.84 (m), 145.70-145.26 (m), 143.33-142.78 (m), 140.78-139.86 (m), 137.76-137.34(m), 67.65, 65.27. ¹⁹F NMR (377 MHz, CH₃OD) δ -144.69 - - 144.85 (m), -158.96 (t, *J* = 20.0 Hz), -165.82 (td, *J* = 21.2, 8.1 Hz). HRMS (EI): m/z ([M-CH₃OH]¹⁻) calcd. for C₇H₂F₅O 197.0026, found 197.0018. HPLC (Chiralcel AYH, n-hexane/2-propanol = 90/10, flow rate: 1 ml/min, detection at 210 nm) retention time: t = 4.3 min (major) and 4.7 min (major), 91.6% ee.

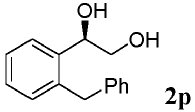

### (R)-1-(2-benzylphenyl)ethane-1,2-diol

(white solid, 72.0 mg, 79% yield) ¹H NMR (400 MHz, CDCl₃) δ 7.51 (d, *J* = 7.3 Hz, 1H), 7.33-7.23 (m, 4H), 7.21-7.12 (m, 2H), 7.10 (d, *J* = 7.6 Hz, 2H), 5.02 (dd, *J* = 8.4, 3.3 Hz, 2H), 4.19 - 3.88 (m, 2H), 3.60 - 3.34 (m, 1H), 2.51 (s, 1H), 2.19 (s, 1H). ¹³C NMR (100 MHz, CDCl₃) δ 140.69, 138.88, 137.61, 132.53, 128.72, 128.69, 128.15, 127.20, 126.68, 126.41, 71.70, 67.40, 39.23. HRMS (ESI): m/z ([M+Na]¹⁺) calcd. for C₁₅H₁₆O₂Na 251.1042, found 251.1032. HPLC (Chiralcel AD-H, n-hexane/2-propanol = 90/5, flow rate: 1 ml/min, detection at 210 nm) retention time: t = 30.9 min (major) and 33.0 min (minor), 79.9% ee. [α]²⁰_{D}=-47.3 (c=1.0, CHCl₃)

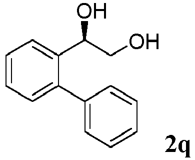

### (R)-1-([1,1'-biphenyl]-2-yl)ethane-1,2-diol

(white solid, 61.6 mg, 72% yield)¹H NMR (500 MHz, CD₃OD) δ 7.62 (d, *J* = 7.8 Hz, 1H), 7.41 (dd, *J* = 15.6, 8.0 Hz, 3H), 7.33 (td, *J* = 14.7, 7.7 Hz, 4H), 7.17 (d, *J* = 7.6 Hz, 1H), 4.84 (d, *J* = 6.0 Hz, 1H), 3.53 (d, *J* = 6.0 Hz, 2H). ¹³C NMR (125 MHz, CD₃OD) δ 142.65, 142.47, 140.24, 130.87, 130.41, 129.25, 128.63, 128.30, 128.13, 127.68, 72.04, 68.07. HRMS (ESI): m/z ([M+Na]¹⁺) calcd. for C₁₄H₁₄O₂Na 237.0886, found 237.0879. HPLC (Chiralcel AYH, n-hexane/2-propanol = 90/10, flow rate: 1 ml/min, detection at 210 nm) retention time: t = 9.9 min (minor) and 11,0 min (major),93.1% ee. [α]¹⁹_{D}=-32.4 (c=1, CHCl₃)

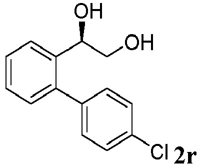

### (R)-1-(4'-chloro-[1,1'-biphenyl]-2-yl)ethane-1,2-diol

(white solid, 86.3 mg, 87% yield) ¹H NMR (500 MHz, CDCl₃) δ 7.61 (d, *J* = 7.6 Hz, 1H), 7.38 (tt, *J* = 14.6, 7.2 Hz, 4H), 7.28 - 7.13 (m, 3H), 4.91 - 4.77 (m, 1H), 3.59 (d, *J* = 5.3 Hz, 2H), 2.54 (s, 1H), 2.08 (s, 1H). ¹³C NMR (125 MHz, CDCl₃) δ 140.09, 139.16, 137.80, 133.63, 130.66, 130.18, 128.68, 128.33, 128.00, 126.59, 71.11, 67.48. HRMS (ESI): m/z ([M+Na]¹⁺) calcd. for C₁₄H₁₃ClNaO₂ 271.0496, 271.0499. HPLC (Chiralcel OD-3, n-hexane/2-propanol = 90/10, flow rate: 1 ml/min, detection at 210 nm) retention time: t = 8.5 min (major) and 9.5 min (minor), 94.9% ee. [α]²⁰_{D}=-24.0 (c=1.0, CHCl₃)

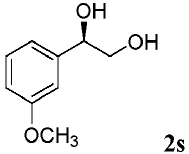

### (R)-1-(3-methoxyphenyl)ethane-1,2-diol

(white solid, 45.9 mg, 60% yield) ¹H NMR (400 MHz, CDCl₃) δ 7.26 (dd, *J* = 9.7, 6.0 Hz, 1H), 6.90 (d, *J* = 7.1 Hz, 2H), 6.82 (dd, *J* = 8.9, 2.0 Hz, 1H), 4.77 (dd, *J* = 8.2, 3.3 Hz, 1H), 3.79 (s, 3H), 3.72 (dd, *J* = 11.4, 3.3 Hz, 1H), 3.62 (dd, *J* = 11.4, 8.3 Hz, 1H), 3.38 (s, 1H), 2.96 (s, 1H). ¹³C NMR (100 MHz, CDCl₃) δ 159.84, 142.34, 129.68, 118.46, 113.43, 111.78, 74.72, 68.14, 55.35. HRMS (ESI): m/z ([M+Na]¹⁺) calcd. for C₉H₁₂NaO₃ 191.0679, found 191.0680. HPLC (Chiralcel OD-3, n-hexane/2-propanol = 92/8, flow rate: 1 ml/min, detection at 230 nm) retention time: t = 18.0 min (major) and 20.3 min (minor), 87.7% ee. [α]¹⁹_{D}=-39.4 (c=1.6, CHCl₃)

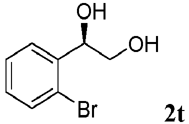

### (R)-1-(2-bromophenyl)ethane-1,2-diol

(white solid, 64.7 mg, 75% yield) ¹H NMR (400 MHz, MeOD) δ 7.60 (dd, *J* = 7.8, 1.5 Hz, 1H), 7.53 (dd, *J* = 8.0, 0.9 Hz, 1H), 7.36 (t, *J* = 7.5 Hz, 1H), 7.16 (td, *J* = 7.8, 1.7 Hz, 1H), 5.10 (dd, *J* = 7.8, 3.1 Hz, 1H), 3.73 (dd, *J* = 11.5, 3.2 Hz, 1H), 3.46 (dd, *J* = 11.5, 7.8 Hz, 1H). ¹³C NMR (100 MHz, MeOD) δ 142.15, 133.52, 130.08, 129.31, 128.65, 123.00, 74.67, 67.21. HRMS (ESI): m/z ([M+Na]¹⁺) calcd. for C₈H₉BrNaO₂ 238.9678, found 238.9676. HPLC (Chiralcel OD-3, n-hexane/2-propanol = 95/5, flow rate: 1 ml/min, detection at 210 nm) retention time: t = 8.3 min (major) and 10.4 min (minor), 68.3% ee.

### [α]²¹_{D}=-29.6 (c=0.8, CHCl₃)

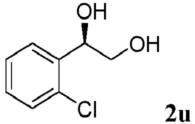

### (R)-1-(2-chlorophenyl)ethane-1,2-diol

(white solid, 46.1 mg, 67% yield) ¹H NMR (400 MHz,CDCl₃) δ 7.55 (d, *J* = 7.7 Hz, 1H), 7.35 - 7.10 (m, 2H), 5.22 (d, *J* = 7.2 Hz, 1H), 4.07 - 3.08 (m, 4H). ¹³C NMR (100 MHz, CDCl₃) δ 137.50, 132.83, 129.48, 129.00, 127.67, 127.19, 71.58, 66.35. HRMS (ESI): m/z ([M+Na]¹⁺) calcd. for C₈H₉ClO₂Na 195.0183, found 195.0182. HPLC (Chiralcel OD-3, n-hexane/2-propanol = 90/10, flow rate: 1 ml/min, detection at 210 nm) retention time: t = 17.4 min (major) and 24.0 min (minor), 75.5% ee. [α]²⁰_{D}=-38.4 (c=2.2, CHCl₃)

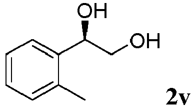

### (R)-1-(o-tolyl)ethane-1,2-diol

(white solid, 54.7 mg, 90% yield) ¹H NMR (500 MHz, CDCl₃) δ 7.47 (d, *J* = 6.7 Hz, 1H), 7.26 - 7.18 (m, 2H), 7.15 (d, *J* = 6.7 Hz, 1H), 5.04 (d, *J* = 8.0 Hz, 1H), 3.85-3.38 (m, 4H), 2.32 (s, 3H). ¹³C NMR (125 MHz, CDCl₃) δ 138.54, 134.79, 130.47, 127.75, 126.35, 125.73, 71.58, 66.98, 19.07. HRMS (ESI): m/z ([M+Na]¹⁺) calcd. for C₉H₁₂NaO₂ 175.0730, found 175.0729. HPLC (Chiralcel OD-H, n-hexane/2-propanol = 90/10, flow rate: 1 ml/min, detection at 220 nm) retention time: t = 8.0 min (major) and 9.7 min (minor), 76.3% ee. [α]²⁰_{D}=-51.85 (c=1.4, CHCl₃)

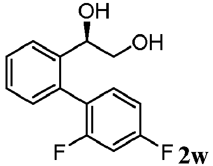

### (R)-1-(2',4'-difluoro-[1,1'-biphenyl]-2-yl)ethane-1,2-diol

(white solid, 85 mg, 85% yield) ¹H NMR (500 MHz, MeOD) δ 7.65 (s, 1H), 7.44 (t, *J* = 7.6 Hz, 1H), 7.34 (t, *J* = 7.5 Hz, 2H), 7.15 (d, *J* = 7.0 Hz, 1H), 7.04 (t, *J* = 8.5 Hz, 2H), 4.62 (s, 1H), 3.52 (s, 1H), 3.44 (s, 1H). ¹³C NMR (125 MHz, MeOD) δ 165.01 (d, *J* = 11.7 Hz), 163.05 (d, *J* = 11.7 Hz), 141.54 (d, *J* = 31.9 Hz), 134.00 (d, *J* = 67.8 Hz), 131.37, 129.56, 128.43, 127.69 (d, *J* = 37.5 Hz), 125.74 (d, *J* = 31.0 Hz), 112.29 (d, *J* = 21.9 Hz), 104.75 (t, *J* = 26.3 Hz), 72.68, 68.07 (d, *J* = 41.8 Hz). ¹⁹F NMR (471 MHz, MeOD) δ -111.50, -111.98, -112.92, -113.06. HRMS (ESI): m/z ([M+Na]¹⁺) calcd. for C₁₄H₁₂F₂NaO₂ 273.0698, found 273.0703. HPLC (Chiralcel OD-3, n-hexane/2-propanol = 90/10, flow rate: 1 ml/min, detection at 230 nm) retention time: t = 8.7 min (major) and 10.4 min (minor), 91.5% ee. [α]²⁰_{D}=-30.8 (c=2.5, CHCl₃)

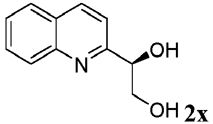

### (R)-1-(quinolin-2-yl)ethane-1,2-diol

(white solid, 34.0 mg, 45% yield) ¹H NMR (500 MHz, CDCl₃) δ 8.19 (d, *J=* 8.4 Hz, 1H), 8.07 (d, *J* = 8.4 Hz, 1H), 7.84 (d, *J* = 8.1 Hz, 1H), 7.74 (t, *J* = 7.0 Hz, 1H), 7.56 (t, *J* = 7.5 Hz, 1H), 7.43 (d, *J* = 8.5 Hz, 1H), 5.03 - 4.94 (m, 1H), 4.07 (dd, *J* = 11.4, 3.6 Hz, 1H), 3.93 - 3.79 (m, 1H). ¹³C NMR (100 MHz, CDCl₃) δ 191.55, 175.43, 166.26, 160.45, 157.30, 157.20, 157.15, 154.88, 149.52, 104.08, 95.78. HRMS (ESI): m/z ([M+H]¹⁺) calcd. for C₁₁H₁₂NO₂ 190.0863, found 190.0864. HPLC (Chiralcel OD-3, n-hexane/2-propanol = 95/5, flow rate: 1 ml/min, detection at 250 nm) retention time: t = 49.6 min (major) and 51.9 min (minor), 97.9% ee.

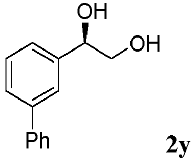

### (R)-1-([1,1'-biphenyl]-3-yl)ethane-1,2-diol

(white solid, 70.3 mg, 82% yield) ¹H NMR (400 MHz, CDCl₃) δ 7.58 (d, *J* = 7.1 Hz, 3H), 7.53 (d, *J* = 7.8 Hz, 1H), 7.48 - 7.39 (m, 3H), 7.38 - 7.30 (m, 2H), 4.89 (dd, *J* = 8.2, 3.5 Hz, 1H), 3.90 - 3.54 (m, 2H), 2.81 (s, 1H), 2.29 (s, 1H). ¹³C NMR (100 MHz, CDCl₃) δ 141.72, 141.16, 141.01, 129.15, 128.94, 127.60, 127.32, 127.00, 125.12, 125.03, 74.87, 68.27. HRMS (ESI): m/z ([M+Na]¹⁺) calcd. for C₁₄H₁₄NaO₂ 237.0886, found 237.1011. HPLC (Chiralcel OD-3, n-hexane/2-propanol = 90/10, flow rate: 1 ml/min, detection at 210 nm) retention time: t = 17.0 min (major) and 20.9 min (minor), 87.7% ee.

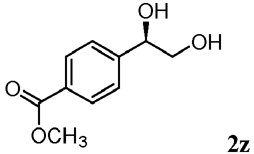

### methyl (R)-4-(1,2-dihydroxyethyl)benzoate

(white solid, 61.2 mg, 78% yield) ¹H NMR (400 MHz, CDCl₃) δ 7.98 (d, *J* = 8.0 Hz, 2H), 7.40 (d, *J* = 8.0 Hz, 2H), 4.84 (dd, *J* = 8.3, 3.3 Hz, 1H), 3.90 (s, 3H), 3.75 (dd, *J* = 11.4, 3.3 Hz, 1H), 3.60 (dd, *J* = 11.4, 8.2 Hz, 2H), 3.02 (s, 1H). ¹³C NMR (100 MHz, CDCl₃) δ 167.11, 145.77, 129.86, 129.65, 126.12, 74.38, 67.88, 52.32. HRMS (ESI): m/z ([M+H]¹⁺) calcd. for C₁₀H₁₃O₄ 197.0808, found 197.0806. HPLC (ChiralcelOJH, n-hexane/2-propanol = 95/5, flow rate: 1 ml/min, detection at 210 nm) retention time: t = 69.7 min (major) and 74.4 min (minor), 83.3% ee.

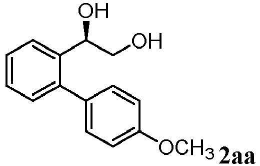

### (R)-1-(4'-methoxy-[1,1'-biphenyl]-2-yl)ethane-1,2-diol

(white solid, 39.1 mg, 40% yield)¹H NMR (400 MHz, CDCl₃) δ 7.57 (d, *J* = 7.6 Hz, 1H), 7.44-7.27 (m, 2H), 7.19 (d, *J* = 6.7 Hz, 3H), 6.92 (d, *J* = 7.9 Hz, 2H), 5.02 - 4.74 (m, 1H), 3.83 (s, 3H), 3.65-3.45(m, 2H), 2.77 (brs, 1H), 2.48 (brs, 1H). ¹³C NMR (100 MHz, CDCl₃) δ 158.93, 140.93, 138.01, 133.03, 130.43, 130.35, 127.76, 127.70, 126.42, 113.85, 71.22, 67.49, 55.42. HRMS (ESI): m/z ([M+Na]¹⁺) calcd. for C₁₅H₁₆NaO₃ 267.0992, found 267.0991. HPLC (Chiralcel OD-3, n-hexane/2-propanol = 90/10, flow rate: 1 ml/min, detection at 210 nm) retention time: t = 12.9 min (major) and 14.9 min (minor), 77.5% ee.

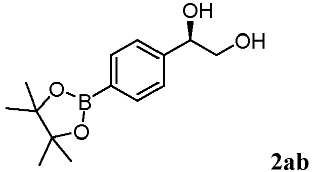

(R)-1-(4-(4,4,5,5-tetramethyl-1,3,2-dioxaborolan-2-yl)phenyl)ethane-1,2-diol (colourless, 59.2 mg, 56% yield) ¹H NMR (400 MHz, CDCl₃) δ 7.81 (s, 1H), 7.77 (d, *J* = 7.5 Hz, 1H), 7.51 (d, *J* = 7.7 Hz, 1H), 7.40 (t, *J* = 7.5 Hz, 1H), 4.87 (dd, *J* = 8.3, 3.5 Hz, 1H), 3.80 (d, *J=* 9.3 Hz, 1H), 3.76 - 3.65 (m, 1H), 2.59 (s, 1H), 2.15 (s, 1H), 1.37 (s, 12H). ¹³C NMR (100 MHz, CDCl₃) δ 140.09, 134.33, 132.49, 129.23, 127.96, 83.97, 75.23, 74.75, 68.08, 24.88, 24.84. HRMS (ESI): m/z ([M+H]¹⁺) calcd. for C₁₄H₂₁BNaO₄ 287.1425, found 287.1426. HPLC (Chiralcel OD-3, n-hexane/2-propanol = 90/10, flow rate: 1 ml/min, detection at 220 nm) retention time: t = 7.1 min (major) and 8.0 min (minor), 83.1% ee.

### Characterization of Diols of Conjugated Dienes

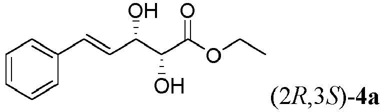

### ethyl (2R,3 S,E)-2,3-dihydroxy-5-phenylpent-4-enoate

(colourless oil, 60.5 mg, r.r.=511, 64% yield, majr product) ¹H NMR (400 MHz, Chloroform-*d*) δ 7.42 - 7.36 (m, 2H), 7.31 (t, *J* = 7.4 Hz, 2H), 7.28 - 7.22 (m, 1H), 6.70 (d, *J* = 15.9 Hz, 1H), 6.34 (dd, *J* = 16.0, 6.5 Hz, 1H), 4.59 (d, *J* = 4.0 Hz, 1H), 4.36 - 4.19 (m, 3H), 3.31 (brs, 1H), 2.60 (brs, 1H), 1.31 (t, *J* = 7.2 Hz, 3H). ¹³C NMR (100MHz, CDCl₃) δ 172.87, 136.33, 132.61, 128.70, 128.10, 127.54, 126.77, 73.96, 73.73, 62.38, 14.29. HPLC (Chiralcel AD-H, n-hexane/2-propanol = 90/10, flow rate: 1 ml/min, detection at 210 nm) retention time: t = 20.8 min (major) and 24.2 min (minor), 95.7% ee.

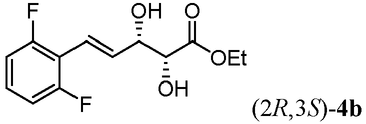

### ethyl (2R,3S,E)-5-(2,6-difluorophenyl)-2,3-dihydroxypent-4-enoate

(white solid, 45.7 mg, 42% yield, major product) ¹H NMR (500 MHz, CDCl₃) δ 7.19 - 7.10 (m, 1H), 6.86 (t, *J* = 8.2 Hz, 2H), 6.69 (dt, *J* = 16.4, 11.0 Hz, 2H), 4.61 (s, 1H), 4.31 (dd, *J* = 12.8, 5.8 Hz, 2H), 4.26 (s, 1H), 3.30 (d, *J* = 5.2 Hz, 1H), 2.68 (d, *J* = 6.6 Hz, 1H), 1.31 (t, *J* = 7.1 Hz, 3H). ¹³C NMR (125 MHz, MeOD) δ 173.60,δ 131.18 (t, *J* = 10.6 Hz), 118.22 (t, *J* = 17.4 Hz), 112.80 (d, *J* = 26.3 Hz), 112.80 (d, *J* = 16.5 Hz), 74.42, 74.05, 72.99, 68.16, 60.83, 13.95. HRMS (ESI): m/z ([M+Na]⁺) calcd. for C₁₃H₁₄F₂NaO₄ 295.0752, found 295.0753. HPLC (Chiralcel AD-H, n-hexane/2-propanol = 90/10, flow rate: 1 ml/min, detection at 220 nm) retention time: t = 20.9 min (minor) and 26.4 min (major), 98.5% ee. [α]²⁰_{D}=-21.0 (c=1.6, CHCl₃)

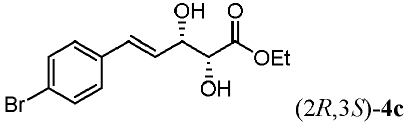

### ethyl (2R,3SE)-5-(4-bromophenyl)-2,3-dihydroxypent-4-enoate

(white solid, 48.2mg, 50% yield, r.r.=3.311, major product) ¹H NMR (400 MHz, CDCl₃) δ 7.44 (d, *J=* 8.4 Hz, 2H), 7.26 (d, *J* = 8.4 Hz, 2H), 6.65 (d, *J* = 16.0 Hz, 1H), 6.33 (dd, *J=* 16.0, 6.3 Hz, 1H), 4.59 (t, *J=* 5.0 Hz, 1H), 4.36 - 4.28 (m, 2H), 4.24 (dd, *J* = 5.1, 2.7 Hz, 1H), 3.18 (d, *J* = 5.4 Hz, 1H), 2.42 (d, *J* = 7.8 Hz, 1H), 1.32 (t, *J* = 7.1 Hz, 3H). ¹³C NMR (101 MHz, CDCl₃) δ 172.78, 135.30, 131.87, 131.39, 128.44, 128.31, 121.97, 73.75, 73.49, 62.53, 14.33. HRMS (ESI): m/z ([M+Na]⁺) calcd. for C₁₃H₁₅BrNaO₄ 337.0046, found 337.0046. HPLC (Chiralcel AD-H, n-hexane/2-propanol = 90/10, flow rate: 1 ml/min, detection at 210 nm) retention time: t = 32.3 min (minor) and 35.5min (major), 97.9% ee. [α]²⁰_{D}=-8.5 (c=1, CHCl₃)

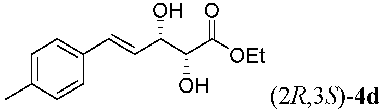

### ethyl (2R,3 S,E)-2,3-dihydroxy-5-(p-tolyl)pent-4-enoate

(colourless oil, 59 mg, r.r.=511, 59% yield, majr product) ¹H NMR (400 MHz,CDCl₃) δ 7.34 - 7.26 (m, 2H), 7.13 (d, *J* = 7.7 Hz, 2H), 6.67 (d, *J* = 15.9 Hz, 1H), 6.37 - 6.19 (m, 1H), 4.63-4.52 (m, 1H), 4.31 (q, *J* = 7.2 Hz, 2H), 4.24 (dt, *J* = 4.8, 2.2 Hz, 1H), 3.12 (d, *J* = 5.7 Hz, 1H), 2.34 (s, 3H), 2.31 (s, 1H), 1.32 (t, *J* = 8.0 Hz, 3H). ¹³C NMR (100 MHz, CDCl₃) δ 172.90, 138.30, 136.27, 132.81, 128.95, 128.63, 127.46, 127.30, 124.01, 73.91, 73.75, 62.44, 21.51, 14.33. HRMS (ESI): m/z ([M+Na]⁺) calcd. for C₁₄H₁₈NaO₄ 273.1097, found 273.1097. HPLC (Chiralcel AD-H, n-hexane/2-propanol = 90/10, flow rate: 1 ml/min, detection at 220 nm) retention time: t = 27.2 min (minor) and 32.2 min (major), 94.0% ee.

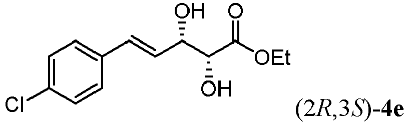

### ethyl (2R,3S,E)-5-(4-chlorophenyl)-2,3-dihydroxypent-4-enoate

(white solid, 43.8 mg, 59% yield, r.r.=511, major product) ¹H NMR (500 MHz, CDCl₃) δ 7.30 (dd, *J* = 18.4, 8.2 Hz, 4H), 6.66 (d, *J* = 15.9 Hz, 1H), 6.32 (dd, *J* = 16.0, 6.3 Hz, 1H), 4.58 (s, 1H), 4.31 (dd, *J* = 14.2, 7.1 Hz, 2H), 4.24 (s, 1H), 3.23 (s, 1H), 2.48 (s, 1H), 1.32 (t, *J* = 7.1 Hz, 3H). ¹³C NMR (125 MHz, CDCl₃) δ 172.79, 134.88, 133.79, 131.34, 128.91, 128.31, 127.99, 73.83, 73.52, 62.49, 14.32. HRMS (ESI): m/z ([M+Na]⁺) calcd. for C₁₄H₁₅ClNaO₄ 293.0551, found 293.0550. HPLC (Chiralcel AD-H, n-hexane/2-propanol = 90/10, flow rate: 1 ml/min, detection at 210 nm) retention time: t = 29.7 min (minor) and 31.9min (major), 96.2% ee.
[α]²⁰_{D}=-14.8 (c=1.3, CHCl₃)

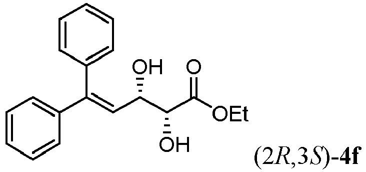

### ethyl (2R,3S)-2,3-dihydroxy-5,5-diphenylpent-4-enoate

(white solid, 60.6 mg, 97% yield, *r.r*.>20//1, major product) ¹H NMR (400 MHz,CDCl₃) δ 7.46-7.31 (m, 3H), 7.30-7.16 (m, 7H), 6.24 (d, *J* = 9.5 Hz, 1H), 4.46 (d, *J* = 9.5 Hz, 1H), 4.29 - 4.01 (m, 3H), 3.24 (s, 1H), 2.49 (s, 1H), 1.22 (t, *J* = 7.0 Hz, 3H). ¹³C NMR (125 MHz, CDCl₃) δ 172.81, 145.81, 141.67, 138.98, 129.81, 128.41, 128.27, 127.99, 127.82, 127.79, 125.95, 74.33, 70.67, 62.16, 14.14. HRMS (ESI): m/z ([M+Na]¹⁺) calcd. for C₁₉H₂₀NaO₄ 335.1254, found 335.1253. HPLC (Chiralcel AD-H, n-hexane/2-propanol = 90/10, flow rate: 1 ml/min, detection at 210 nm) retention time: t = 25.5 min (minor) and 34.0 min (major), 97.1% ee. [α]²⁰_{D}=-7.8 (c=2, CHCl₃)

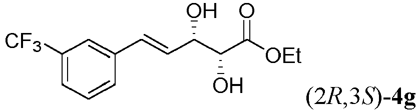

### ethyl (2R,3 S,E)-2,3-dihydroxy-5-(3-(trifluoromethyl)phenyl)pent-4-enoate

(white solid, 58.6 mg, 56% yield, *r.r*.=6.2511, major product) ¹H NMR (500 MHz, Chloroform-*d*) δ 7.63 (s, 1H), 7.55 (d, *J*= 7.6 Hz, 1H), 7.50 (d, *J*= 7.6 Hz, 1H), 7.43 (t, *J* = 7.7 Hz, 1H), 6.74 (d, *J* = 16.0 Hz, 1H), 6.42 (dd, *J* = 16.0, 6.1 Hz, 1H), 4.62 (s, 1H), 4.31 (q, *J* = 7.2 Hz, 2H), 4.26 (s, 1H), 3.31 (d, *J* = 5.6 Hz, 1H), 2.62 (d, *J* = 7.9 Hz, 1H), 1.32 (t, *J* = 7.2 Hz, 3H). ¹³C NMR (125 MHz, CDCl₃) δ 172.75, 137.19, 131.16 (q, *J* = 35.4 Hz), 131.02, 129.94, 129.77, 129.20, 124.62 (q, *J* = 3.7 Hz), 124.18 (q, *J* = 272.4 Hz), 123.39 (q, *J* = 3.8 Hz), 73.80, 73.34, 62.54, 14.28. HRMS (ESI): m/z ([M+Na]⁺) calcd. for C₁₄H₁₅F₃NaO₄ 327.0815, found 327.0815. HPLC (Chiralcel AD-H, n-hexane/2-propanol = 90/10, flow rate: 1 ml/min, detection at 210 nm) retention time: t = 17.1 min (minor) and 20.8 min (major), 97.7% ee. [α]²⁰_{D}=-11.5 (c=1.3, CHCl₃)

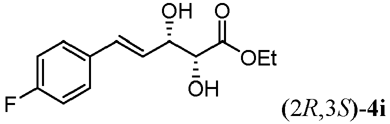

### ethyl (2R,3SE)-5-(4-fluorophenyl)-2,3-dihydroxypent-4-enoate

(white solid, 50 mg, 59% yield, r.r.=511, major product) ¹H NMR (400 MHz,CDCl₃) δ 7.37 (dd, *J* = 8.5, 5.4 Hz, 2H), 7.01 (t, *J* = 8.5 Hz, 2H), 6.67 (d, *J* = 15.9 Hz, 1H), 6.26 (dd, *J* = 15.9, 6.5 Hz, 1H), 4.59 (d, *J* = 6.9 Hz, 1H), 4.32 (q, *J* = 7.6, 7.0 Hz, 2H), 4.24 (s, 1H), 3.16 (s, 1H), 2.37 (d, *J* = 7.3 Hz, 1H), 1.32 (t, *J* = 7.1 Hz, 3H). ¹³C NMR (100 MHz, CDCl₃) δ 172.85, 163.88, 161.42, 132.51 (d, *J* = 3.3 Hz), ,131.02, 128.33 (d, *J* = 8.0 Hz), 127.33 (d, *J* = 2.2 Hz), 115.66 (d, *J* = 21.6 Hz), 73.75 (d, *J* = 29.2 Hz), 62.45, 14.31. ¹⁹F NMR (377 MHz, CDCl₃) δ -113.83. HRMS (ESI): m/z ([M+Na]¹⁺) calcd. for C₁₃H₁₅FNaO₄ 277.0847, found 277.0847. HPLC (Chiralcel AD-H, n-hexane/2-propanol = 90/10, flow rate: 1 ml/min, detection at 210 nm) retention time: t = 21.2 min (minor) and 29.8 min (major), 97.9% ee. [α]²⁰_{D}=-12.7 (c=2.3, CHCl₃)

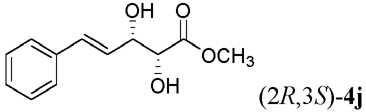

### methyl (2R,3SE)-2,3-dihydroxy-5-phenylpent-4-enoate

(white solid, 43.8 mg, 59% yield, r.r.=511, major product) ¹H NMR (500 MHz, CDCl₃) δ 7.39 (d, *J* = 7.7 Hz, 2H), 7.31 (t, *J* = 7.4 Hz, 2H), 7.24 (d, *J* = 6.9 Hz, 1H), 6.70 (d, *J* = 16.0 Hz, 1H), 6.34 (dd, *J* = 15.9, 6.5 Hz, 1H), 4.60 (d, *J* = 5.7 Hz, 1H), 4.27 (s, 1H), 3.84 (s, 3H), 3.29 (s, 1H), 2.57 (s, 1H). ¹³C NMR (125 MHz, CDCl₃) δ 173.31, 136.31, 132.73, 128.72, 128.15, 127.47, 126.80, 74.02, 73.68, 53.05. HRMS (ESI): m/z ([M+Na]¹⁺) calcd. for C₁₂H₁₄NaO₄ 245.0784, found 245.0781. HPLC (Chiralcel AD-H, n-hexane/2-propanol = 90/10, flow rate: 1 ml/min, detection at 210 nm) retention time: t = 34.2 min (minor) and 37.4 min (major), 95.3% ee. [α]²⁰_{D}=-19.6 (c=0.7, CHCl₃)

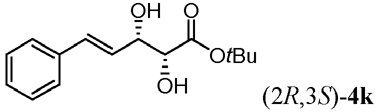

### tert-butyl (2R,3SE)-2,3-dihydroxy-5-phenylpent-4-enoate

(white solid, 57.6 mg, 60% yield, r.r.=10/1, major product) ¹H NMR (500 MHz, CDCl₃) δ 7.39 (d, *J* = 7.3 Hz, 1H), 7.31 (t, *J* = 7.5 Hz, 1H), 7.26 - 7.22 (m, 1H), 6.69 (d, *J* = 15.9 Hz, 1H), 6.33 (dd, *J* = 16.0, 6.4 Hz, 1H), 4.52 (dd, *J* = 5.4, 2.9 Hz, 1H), 4.13 (d, *J* = 3.0 Hz, 1H), 3.26 (s, 1H), 2.51 (s, 1H), 1.50 (s, 9H). ¹³C NMR (100 MHz, CDCl₃) δ 172.09, 136.45, 132.40, 128.69, 128.02, 127.82, 126.75, 83.66, 74.02, 73.88, 28.15. HRMS (ESI): m/z ([M+Na]¹⁺) calcd. for C₁₅H₂₀NaO₄ 287.1254, found 287.1253. HPLC (Chiralcel AD-H, n-hexane/2-propanol = 90/10, flow rate: 1 ml/min, detection at 210 nm) retention time: t = 25.5 min (minor) and 30.5min (major), 92.3% ee. [α]²⁰_{D}=-16.1 (c=1, CHCl₃)

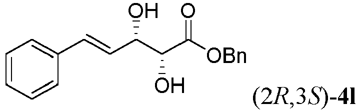

(white solid, 65.6 mg, 62% yield, r.r.=811, major product) ¹H NMR (400 MHz,CDCl₃) δ 7.40 - 7.20 (m, 9H), 6.66 (d, *J =* 15.9 Hz, 1H), 6.30 (dd, *J* = 15.9, 6.6 Hz, 1H), 5.25 (d, *J* = 2.9 Hz, 2H), 4.60 (dd, *J* = 6.8, 2.9 Hz, 1H), 4.28 (d, *J* = 3.0 Hz, 1H), 3.34 (brs, 1H), 2.70 (brs, 1H). ¹³C NMR (100 MHz, CDCl₃) δ 172.28, 136.26, 134.95, 132.77, 128.81, 128.78, 128.69, 128.59, 128.12, 127.29, 126.80, 74.11, 73.77, 67.97. HRMS (ESI): m/z ([M+Na]⁺) calcd. for C₁₈H₁₈NaO₄ 321.1097, 321.1099. HPLC (Chiralcel AD-H, n-hexane/2-propanol = 90/10, flow rate: 1 ml/min, detection at 210 nm) retention time: t = 43.4min (minor) and 46.6 min (major), 96.3% ee. [α]²⁰_{D}=-18.2 (c=1.0, CHCl₃)

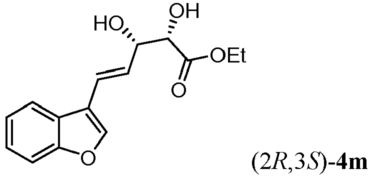

### ethyl (2S,3S,E)-5-(benzofuran-3-yl)-2,3-dihydroxypent-4-enoate

¹H NMR (400 MHz,CDCl₃) δ 7.87 (d, *J* = 7.0 Hz, 2H), 7.58 (s, 1H), 7.45-7.30 (m, 2H), 5.39 (d, *J* = 5.1 Hz, 1H), 4.56 (d, *J* = 3.0 Hz, 1H), 4.28 (p, *J* = 6.9 Hz, 3H), 3.30 (s, 1H), 2.79 (d, *J* = 7.9 Hz, 1H), 1.27 (t, *J* = 7.2 Hz, 2H). ¹³C NMR (100 MHz, CDCl₃) δ 169.65, 151.89, 143.51, 125.86, 124.41, 122.96, 119.00, 113.41, 72.45, 67.99, 62.22, 14.23. HRMS (ESI): m/z ([M+Na]⁺) calcd. for C₁₃H₁₄NaO₅ 273.0733, found 273.0727. HPLC (Chiralcel AD-H, n-hexane/2-propanol = 90/10, flow rate: 1 ml/min, detection at 210 nm) retention time: t = 23.9 min (minor) and 26.2 min (major), 98.5% ee. [α]²⁰_{D}=-5.7 (c=1, CHCl₃)

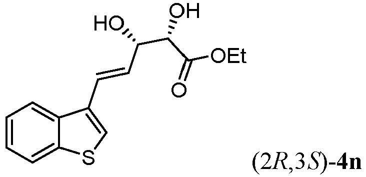

### ethyl (2S,3S,E)-5-(benzo[b]thiophen-3-yl)-2,3-dihydroxypent-4-enoate

(colourless oil, 39.8 mg, *r.r*.>2011, 34% yield, majr product)¹H NMR (400 MHz, CDCl₃) δ 7.87 (dt, *J* = 7.4, 1.9 Hz, 2H), 7.59 (s, 1H), 7.46 - 7.33 (m, 2H), 5.46 - 5.30 (m, 1H), 4.64 - 4.47 (m, 1H), 4.38 - 4.14 (m, 2H), 3.29 (d, *J* = 5.4 Hz, 1H), 2.77 (d, *J* = 7.8 Hz, 1H), 1.27 (t, *J* = 7.2 Hz, 3H). ¹³C NMR (100 MHz, CDCl₃) δ 170.66, 140.73, 136.68, 134.16, 124.70, 124.39, 123.11, 121.92, 72.91, 67.22, 60.68, 14.20. HRMS (ESI): m/z ([M+Na]⁺) calcd. for C₁₃H₁₄NaO₄S 289.0505, found 289.0505. HPLC (Chiralcel AD-H, n-hexane/2-propanol = 90/10, flow rate: 1 ml/min, detection at 220 nm) retention time: t = 24.7 min (minor) and 27.4 min (major), 98.1% ee. [α]²⁰_{D}=+10.5 (c=1, CHCl₃)

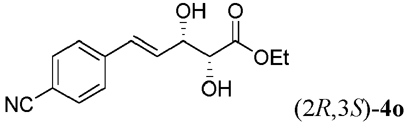

### ethyl (2R,3 S,E)-5-(4-cyanophenyl)-2,3-dihydroxypent-4-enoate

(white solid, 78.4 mg, 75% yield) ¹H NMR (400 MHz, CDCl₃) δ 7.61 (d, *J* = 8.0 Hz, 2H), 7.48 (d, *J* = 8.1 Hz, 2H), 6.74 (d, *J* = 16.0 Hz, 1H), 6.47 (dd, *J* = 16.0, 5.9 Hz, 1H), 4.64 (s, 1H), 4.33 (q, *J* = 7.1 Hz, 2H), 4.27 (d, *J* = 2.7 Hz, 1H), 3.19 (s, 1H), 2.50 (s, 1H), 1.33 (t, *J* = 7.1 Hz, 3H). ¹³C NMR (100 MHz, CDCl₃) δ 172.60, 140.90, 132.60, 131.83, 130.62, 127.26, 118.96, 112.09, 73.58, 73.11, 62.65, 14.33. HRMS (ESI): m/z ([M+Na]⁺) calcd. for C₁₄H₁₅NNaO₄ 284.0899, found 284.0885. HPLC (Chiralcel AD-H, n-hexane/2-propanol = 90/10, flow rate: 1 ml/min, detection at 220 nm) retention time: t = 119.7 min (major) and 150.2 min (minor), 98.3% ee

### Characterization of Tetraols of Conjugated Dienes

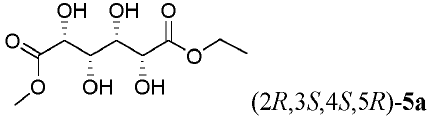

### 1-ethyl 6-methyl (2R,3S,4S,5R)-2,3,4,5-tetrahydroxyhexanedioate

(white solid, 73.6 mg, 73% yield) ¹H NMR (400 MHz, DMSO-*d*₆) δ 5.16 (dd, *J* = 19.6, 7.5 Hz, 2H), 4.75 - 4.57 (m, 2H), 4.19 (dd, *J* = 11.9, 7.5 Hz, 2H), 4.14 - 3.99 (m, 2H), 3.81 (d, *J* = 6.3 Hz, 2H), 3.63 (s, 3H), 1.19 (t, *J* = 7.1 Hz, 3H). HPLC (Chiralcel ADH, n-hexane/2-propanol = 85/15, flow rate: 1 ml/min, detection at 210 nm) retention time: t = 33.5 min (minor) and 38.0 min (major), >99.9% ee. [α]²⁰_{D}=-5.5 (c=1, CH₃OH) ¹³C NMR (100 MHz, DMSO) δ 173.19, 172.68, 72.90, 72.84, 71.18, 69.93, 60.23, 51.56, 14.25. HPLC (Chiralcel AD-H, n-hexane/2-propanol = 85/15, flow rate: 1 ml/min, detection at 210 nm) retention time: t = 33.5 min (minor) and 38.1min (major), >99.9% ee.

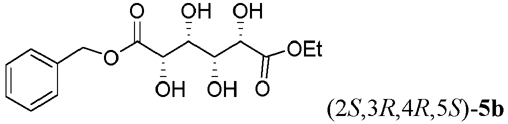

### 1-benzyl 6-ethyl (2S,3R,4R,5S)-2,3,4,5-tetrahydroxyhexanedioate

(white solid, 105 mg, 80% yield) ¹H NMR (400 MHz, Methanol-*d*₄) δ 7.46 - 7.23 (m, 5H), 5.29 - 5.14 (m, 2H), 4.43 (d, *J* = 2.1 Hz, 1H), 4.36 (d, *J* = 2.2 Hz, 1H), 4.22 (q, *J* = 7.1 Hz, 2H), 4.09 (qd, *J* = 7.5, 2.1 Hz, 2H), 1.28 (t, *J* = 7.1 Hz, 3H). ¹³C NMR (100 MHz, MeOD) δ 174.16, 174.03, 137.24, 129.49, 129.20, 129.18, 74.20, 72.57, 72.45, 67.84, 62.29, 14.47. HRMS (ESI): m/z ([M+Na]⁺) calcd. for C₁₅H₂₀NaO₈ 351.1050, found 351.1055. HPLC (Chiralcel AD-H, n-hexane/2-propanol = 85/15, flow rate: 1 ml/min, detection at 210 nm) retention time: t = 48.3 min (minor) and 58.4min (major), >99.9% ee.
[α]²⁰_{D}=+13.5 (c=1, CH₃OH)

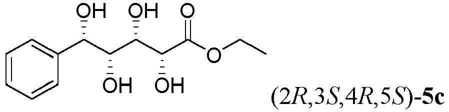

### ethyl (2R,3S,4R,5S)-2,3,4,5-tetrahydroxy-5-phenylpentanoate

¹H NMR (400 MHz, MeOD) δ 7.42 (d, *J* = 7.2 Hz, 2H), 7.34 (t, *J* = 7.5 Hz, 2H), 7.29 - 7.22 (m, 1H), 4.81 (d, *J* = 5.2 Hz, 1H), 4.31 (d, *J* = 3.7 Hz, 1H), 4.25 - 4.10 (m, 2H), 3.77 (dt, *J* = 18.7, 4.4 Hz, 2H), 1.25 (t, *J* = 7.1 Hz, 3H). ¹³C NMR (100 MHz, MeOD) δ 174.11, 143.38, 129.19, 128.50, 128.00, 76.96, 75.07, 73.46, 73.18, 62.22, 14.44. HPLC (Chiralcel ADH, n-hexane/2-propanol = 85/15, flow rate: 1 ml/min, detection at 210 nm) retention time: t = 25.0 min (minor) and 27.3 min (major), >99.9% ee

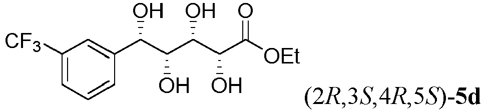

### ethyl (2R,3S,4R,5S)-2,3,4,5-tetrahydroxy-5-(3-(trifluoromethyl)phenyl)pentanoate

(white solid, 67.6 mg, 50% yield) ¹H NMR (400 MHz, MeOD) δ 6.88 (s, 1H), 6.79 (d, *J* = 7.0 Hz, 1H), 6.71 - 6.60 (m, 2H), 4.06 (d, *J* = 3.7 Hz, 1H), 3.52 (d, *J* = 3.2 Hz, 1H), 3.32 (q, *J* = 7.1 Hz, 2H), 2.98 (dd, *J* = 5.3, 3.2 Hz, 1H), 2.91 (t, *J* = 4.6 Hz, 1H), 0.38 (t, *J* = 7.1 Hz, 3H). ¹³C NMR (100 MHz, MeOD) δ 171.78, 140.12, 131.64, 131.35 (d, *J* = 31.8 Hz), 125.81 (d, *J* = 271.3 Hz) 124.98 (q, *J* = 3.9 Hz), 124.60 (q, *J* = 3.9 Hz). δ129.60, 76.66, 74.04, 73.66, 73.05, 62.68, 14.43. HRMS (ESI): m/z ([M+Na]⁺) calcd. for C₁₄H₁₇F₃NaO₆ 361.0875, found 361.0866. HPLC (Chiralcel AD-H, n-hexane/2-propanol = 90/10, flow rate: 1 ml/min, detection at 210 nm) retention time: t = 23.7 min (minor) and 27.5 min (major), >99.9% ee. [α]²⁰_{D}=+10.2 (c=1, CH₃OH)

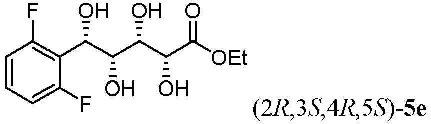

### ethyl (2R,3S,4R,5S)-5-(2,6-difluorophenyl)-2,3,4,5-tetrahydroxypentanoate (white solid, 51.4 mg, 42% yield)

¹H NMR (400 MHz, CD₃OD) δ 7.42 - 7.26 (m, 1H), 6.95 (t, *J* = 8.5 Hz, 2H), 5.19 (d, *J* = 7.8 Hz, 1H), 4.31 - 4.07 (m, 4H), 3.50 (dd, *J* = 5.2, 2.5 Hz, 1H), 1.25 (t, *J* = 7.1 Hz, 3H). ¹³C NMR (100 MHz, CD₃OD) δ δ 173.95, 164.06 (d, *J* = 8.5 Hz), 161.59 (d, *J* = 8.6 Hz), 131.17 (t, *J* = 10.6 Hz), 118.23 (t, *J* = 17.3 Hz), 113.13 - 112.55 (m), 74.45 (t, *J* = 2.7 Hz) , 74.04, 71.52, 67.04, 61.48, 14.38. ¹⁹F NMR (377 MHz,CD₃OD) δ -114.21 (t, *J* = 7.3 Hz). HPLC (Chiralcel AD-H, n-hexane/2-propanol = 85/15, flow rate: 1 ml/min, detection at 210 nm) retention time: t = 27.4 min (major) and 30.9 min (minor), >99.9% ee. [α]¹⁹_{D}=+6.3 (c=1, CH₃OH)

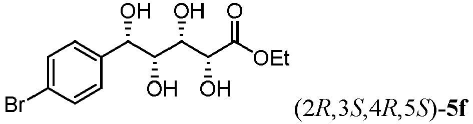

### ethyl (2R,3S,4R,5S)-5-(4-bromophenyl)-2,3,4,5-tetrahydroxypentanoate

(white solid, 69.8 mg, 50% yield) ¹H NMR (500 MHz, MeOD) δ 7.49 (d, *J* = 8.4 Hz, 2H), 7.35 (d, *J* = 8.5 Hz, 2H), 4.81 (d, *J* = 4.5 Hz, 1H), 4.37 (d, *J* = 3.2 Hz, 1H), 4.21 (qd, *J* = 7.2, 2.7 Hz, 2H), 3.84 - 3.73 (m, 2H), δ 1.26 (t, *J* = 7.1 Hz, 3H). ¹³C NMR (125 MHz, MeOD) δ 173.68, 142.85, 131.18, 128.76, 121.97, 76.16, 74.19, 73.31, 73.18, 62.86, 15.18. HRMS (ESI): m/z ([M+Na]⁺) calcd. for C₁₃H₁₇BrNaO₆ 371.0101, found 371.0096. HPLC (Chiralcel AD-H, n-hexane/2-propanol = 90/10, flow rate: 1 ml/min, detection at 210 nm) retention time: t = 46.9 min (minor) and 60.8 min (major), 99.3% ee. [α]¹⁹_{D}=+9.0 (c=1, CH₃OH)

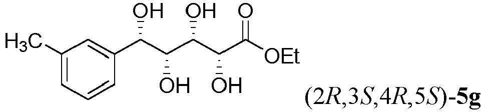

### ethyl (2R,3S,4R,5S)-2,3,4,5-tetrahydroxy-5-(m-tolyl)pentanoate

(white solid, 64.4 mg, 55% yield) ¹H NMR (400 MHz,MeOD) δ 7.27 - 7.16 (m, 3H), 7.08 (d, *J* = 6.2 Hz, 1H), 4.76 (d, *J* = 5.4 Hz, 1H), 4.29 (d, *J* = 3.8 Hz, 1H), 4.22 - 4.13 (m, 2H), 3.82 - 3.75 (m, 1H), 3.73 (t, *J=* 4.1 Hz,1H), 2.34 (s, 3H), 1.25 (t, *J=* 7.1 Hz, 3H).. ¹³C NMR (100 MHz, MeOD) δ 174.08, 142.39, 138.83, 129.17, 129.11, 128.62, 125.09, 76.48, 75.15, 73.50, 72.38, 61.30, 21.52, 14.88. HRMS (ESI): m/z ([M+Na]⁺) calcd. for C₁₄H₂₀NaO₆ 307.1152, found 307.1155. HPLC (Chiralcel AD-H, n-hexane/2-propanol = 85/15, flow rate: 1 ml/min, detection at 210 nm) retention time: t = 23.7 min (minor) and 27.5 min (major), >99.9% ee. [α]¹⁹_{D}=+10.8 (c=1, CH₃OH)

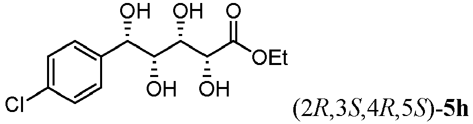

### ethyl (2R,3S,4R,5S)-5-(4-chlorophenyl)-2,3,4,5-tetrahydroxypentanoate

(white solid, 61 mg, 51% yield) ¹H NMR (500 MHz, MeOD) δ 7.40 (d, *J* = 8.1 Hz, 2H), 7.33 (d, *J* = 8.0 Hz, 2H), 4.83 - 4.75 (m, 1H), 4.34 (d, *J* = 3.3 Hz, 1H), 4.25-4.13 (m, 2H), 3.78 (d, *J* = 3.7 Hz, 1H), 3.74 (s, 1H), 1.25 (t, *J* = 7.1 Hz, 3H). ¹³C NMR (125 MHz, MeOD) δ 173.72, 142.47, 134.07, 129.57, 129.18, 76.79, 74.24, 73.35, 73.27, 62.27, 14.44. HRMS (ESI): m/z ([M+Na]⁺) calcd. for C₁₃H₁₇FNaO₆ 327.0606, found 327.0603. HPLC (Chiralcel IA, n-hexane/2-propanol = 90/10, flow rate: 1 ml/min, detection at 210 nm) retention time: t = 40.4 min (minor) and 74.9 min (major), >99.9% ee. [α]²⁰_{D}=+10.4 (c=1, CH₃OH)

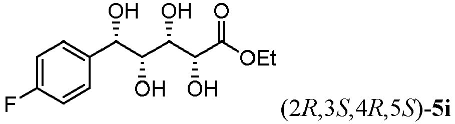

### ethyl (2R,3S,4R,5S)-5-(4-fluorophenyl)-2,3,4,5-tetrahydroxypentanoate

(white solid, 57.6 mg, 50% yield) ¹H NMR (500 MHz, Methanol-*d*₄) δ 7.43 (dd, *J* = 8.6, 5.6 Hz, 2H), 7.06 (t, *J* = 8.8 Hz, 2H), 4.82 (d, *J* = 3.9 Hz, 1H), 4.33 (d, *J* = 2.7 Hz, 1H), 4.18 (ddt, *J* = 10.8, 6.9, 3.8 Hz, 2H), 3.75 (d, *J* = 2.3 Hz, 2H), 1.26 (t, *J* = 7.1 Hz, 3H). ¹³C NMR (101 MHz, CD₃OD_SPE) δ 174.12, 164.81, 161.98, 139.49, 129.84, 129.76, 115.85, 115.63, 76.89, 74.34, 73.37, 73.27, 62.25, 14.44. ¹⁹F NMR (471 MHz, MeOD) δ -117.64. HRMS (ESI): m/z ([M+Na]⁺) calcd. for C₁₃H₁₇FNaO₆ 311.0901, found 311.0903.

HPLC (Chiralcel AD-H, n-hexane/2-propanol = 90/10, flow rate: 1 ml/min, detection at 210 nm) retention time: t = 42.0 min (minor) and 46.9 min (major), 95.9% ee.
[α]¹⁸_{D}=+10.2 (c=1, CH₃OH)

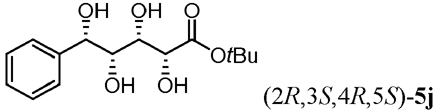

### tert-butyl (2R,3S,4R,5S)-2,3,4,5-tetrahydroxy-5-phenylpentanoate

(white solid, 60.8 mg, 51% yield) ¹H NMR (500 MHz, MeOD) δ 7.42 (d, *J* = 7.2 Hz, 2H), 7.33 (t, *J* = 7.5 Hz, 2H), 7.26 (t, *J* = 7.3 Hz, 1H), 4.80 (d, *J* = 5.5 Hz, 1H), 4.17 (d, *J* = 4.1 Hz, 1H), 3.79 - 3.74 (m, 1H), 3.68 (t, *J* = 4.2 Hz, 1H), 1.44 (s, 9H). HRMS (ESI): m/z ([M+Na]⁺) calcd. for C₁₅H₂₂NaO₆ 321.1309, found 321.1301. HPLC (Chiralcel AD-H, n-hexane/2-propanol = 85/15, flow rate: 1 ml/min, detection at 210 nm) retention time: t = 20.7min (minor) and 27.9 min (major), >99.9% ee. [α]¹⁹_{D}=+10.6 (c=1, CH₃OH)

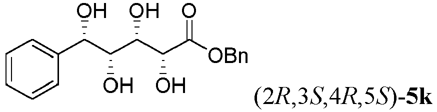

### benzyl (2R,3S,4R,5S)-2,3,4,5-tetrahydroxy-5-phenylpentanoate

(white solid, 71.8 mg, 54% yield) ¹H NMR (400 MHz, MeOD) δ 7.41 - 7.25 (m, 10H), 5.16 (s, 2H), 4.81 (d, *J* = 4.3 Hz, 1H), 4.38 (s, 1H), 3.79 (s, 2H). ¹³C NMR (100 MHz, MeOD) δ 173.43, 143.23, 137.15, 129.50, 129.21, 129.19, 129.17, 128.47, 127.39, 76.96, 75.12, 73.58, 73.16, 68.22. HRMS (ESI): m/z ([M+Na]⁺) calcd. for C₁₈H₂₀NaO₆ 355.1152, found 355.1148. HPLC (Chiralcel AD-H, n-hexane/2-propanol = 90/10, flow rate: 1 ml/min, detection at 210 nm) retention time: t = 74.4min (minor) and 78.4 min (major), >99.9% ee. [α]²⁰_{D}=+13.9 (c=1, CH₃OH)

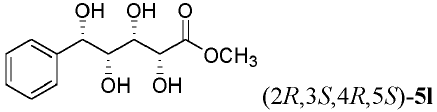

### methyl (2R,3S,4R,5S)-2,3,4,5-tetrahydroxy-5-phenylpentanoate

(white solid, 56.3 mg, 55% yield) ¹H NMR (400 MHz,MeOD) δ 7.42 (d, *J* = 7.6 Hz, 2H), 7.33 (t, *J* = 7.4 Hz, 2H), 7.26 (t, *J* = 7.3 Hz, 1H), 4.80 (d, *J* = 4.7 Hz, 1H), 4.34 (s, 1H), 3.80 (dt, *J* = 6.7, 4.2 Hz, 1H), 3.76 (s, 1H), 3.72 (s, 3H). ¹³C NMR (100 MHz, MeOD) δ 169.45, 142.67, 129.20, 128.51, 128.00, 76.88, 75.09, 73.49, 70.91, 52.54. HRMS (ESI): m/z ([M+Na]⁺) calcd. for C₁₂H₁₆NaO₆ 279.0839, found 295.0834. HPLC (Chiralcel IC-3, n-hexane/2-propanol = 85/15, flow rate: 1 ml/min, detection at 210 nm) retention time: t = 63.7 min (minor) and 67.6 min (major), >99.9% ee. [α]²⁰_{D}=+8.2 (c=1, CH₃OH)

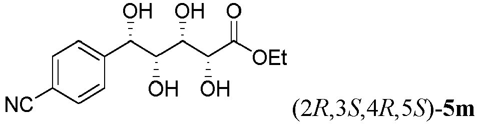

ethyl (2R,3S,4R,5S)-5-(4-cyanophenyl)-2,3,4,5-tetrahydroxypentanoate

(white solid, 41.3 mg, 35% yield) ¹H NMR (400 MHz, CDCl₃) δ 7.64 (d, *J* = 7.9 Hz, 2H), 7.54 (d, *J* = 8.0 Hz, 2H), 4.91 (d, *J* = 5.1 Hz, 1H), 4.38 - 4.17 (m, 3H), 4.14 - 3.13 (m, 6H), 1.26 (t, *J* = 7.1 Hz, 3H). ¹³C NMR (101 MHz, CDCl₃) δ 172.43, 145.88, 132.37, 127.68, 118.81, 111.84, 75.99, 74.08, 72.99, 71.24, 62.63, 14.20.HRMS (ESI): m/z ([M+Na]⁺) calcd. for C₁₄H₁₇NNaO₆ 318.0954, found 318.0944.

### Results

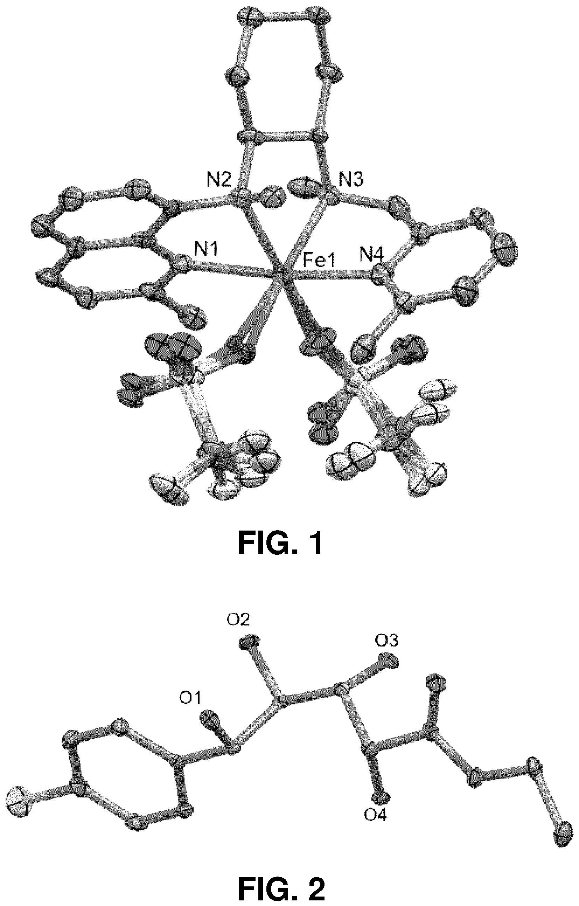

The structures of iron catalysts formed from PQCN-n (n=1-8) and BQCN ligands are shown below. X-ray diffraction analysis revealed a *cis*-α configuration for [Fe(S,S-PQCN-1)(OTf)₂] (Figure 1).

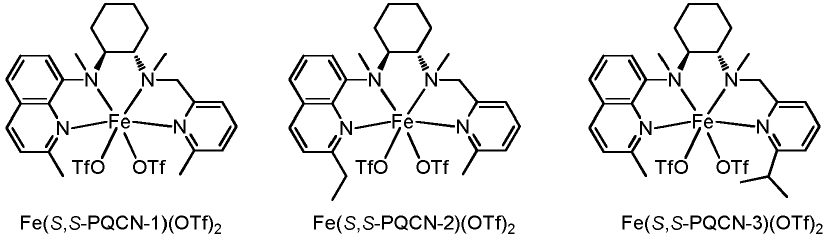

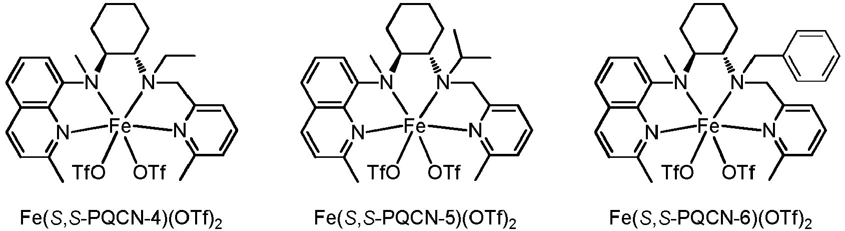

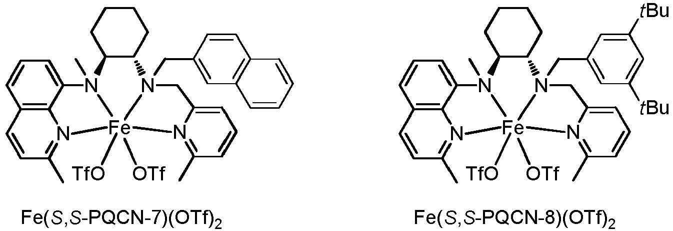

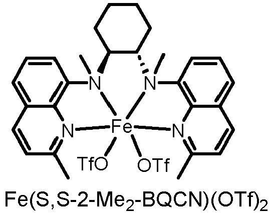

The unsymmetric iron catalysts were used for asymmetric *cis*-dihydroxylation of styrene. Under air, 3 equiv H₂O₂ dissolved in methanol was added into a methanol solution containing the styrene substrate and 6 mol % catalysts via syringe pump over 30 minutes at -30 °C. Then reactions were stirred for another 5 h. The results are shown in **Table** 1. As shown in **Table 1,** [Fe(*S,S*-PQCN-1)(OTf)₂] gave *cis*-diols of styrene in 66% yield with 75% *ee.* When compared to reported iron-catalyzed AD of styrenes ([Fe(6-Me₂-BPBPP)(OTf)₂] (Angew. Chem. Int. Ed. 2008, 47, 1887-1889), and [Fe(2-Me₂-BQCN)(OTf)₂] (Angew. Chem. Int. Ed. 2016, 55,10253-10257)), the enantioselectivity in the AD reaction of styrene was improved to a large extent (ee obtained using different catalysts: [Fe(*S,S*-PQCN-1)(OTf)₂] = 75%, [Fe(*S,S*-PQCN-6)(OTf)₂] = 84%; cf. ([Fe(6-Me₂-BPBPP)(OTf)₂] =15%, [Fe(2-Me₂-BQCN)(OTf)₂] = 17.5%). Further screening of catalyst revealed that 4 mol % [Fe(*S,S*-PQCN-6)(OTf)₂] gave 89% yield and 80% *ee,* and 6 mol% [Fe(*S,S*-PQCN-6)(OTf)₂] gave 85% product yield and 84% *ee.* When using 10 mol % [Fe(*S,S*-PQCN-6)(OTf)₂], the product yield decreased to 66% while the *ee* was maintained.

**Table 1. Screening of Iron complexes in asymmetric cis-dihydroxylation of styrene**

| | | | |
|---|---|---|---|
| 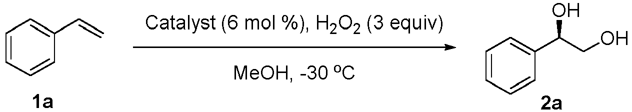 | | | |

| Entry^{[a]} | catalyst | Yield [%]^{[b]} | *ee*^{[c]} |
|---|---|---|---|
| 1 | [Fe(*S,S*-PQCN-1)(OTf)₂] | 66 | 75 |
| 2 | [Fe(*S,S*-PQCN-2)(OTf)₂] | 5 | 50 |
| 3 | [Fe(*S,S*-PQCN-3)(OTf)₂] | 10 | 35 |
| 4 | [Fe(*S,S*-PQCN-4)(OTf)₂] | 65 | 77 |
| 5 | [Fe(*S,S*-PQCN-5)(OTf)₂] | 67 | 63 |
| 6 | [Fe(*S,S*-PQCN-6)(OTf)₂] | 85 | 84 |
| 7^{[d]} | [Fe(*S,S*-PQCN-6)(OTf)₂] | 89 | 80 |
| 8^{[e]} | [Fe(*S,S*-PQCN-6)(OTf)₂] | 66 | 84 |
| 9 | [Fe(*S,S*-PQCN-7)(OTf)₂] | 51 | 75 |
| 10 | [Fe(*S,S*-PQCN-8)(OTf)₂] | 58 | 78 |

| | | | |
|---|---|---|---|
| [a] Reaction conditions: **1a** (0.4 mmol), Fe catalyst (6 mol%), H₂O₂ (3 equiv, 1.2 mmol, dissolved in 1 mL CH₃OH), CH₃OH (5 mL) under air at -30 °C. [b] Isolated yield. [c] Enantiomeric excess (*ee*) determined by chiral HPLC. [d] 4 mol% of catalyst. [e] 10 mol% of catalyst. | | | |

Various substrates were then investigated following the reaction scheme shown in **Table 2.** The reaction conditions were as follows: H₂O₂ (3 equiv, 1.2 mmol, dissolved in 1 mL CH₃OH) was added to a CH₃OH (5 mL) solution containing styrene (1 equiv, 0.4 mmol) and [Fe(*S,S*-PQCN-6)(OTf)₂] (6 mol %, 0.024 mmol) over 30 minutes with a syringe pump; after addition of H₂O₂, the reaction was stirred for additional 30 minutes at -30 °C. The product, isolated yield, and *ee* from each styrene substrate are summarized in **Table** 2. Generally, styrene substrates with electron-withdrawing group(s) resulted in higher enantioselectivities than substrates with electron-donating group(s), but when the substituent(s) was on *ortho* position, enantioselectivity decreased slightly (67.5-72.1% *ee,* **2t** and **2u**). Interestingly, when there is a phenyl ring on *ortho* position, enantioselectivity improved dramatically (91.5-94.9% *ee,* **2q**, **2r** and **2w**). For styrene with electron-withdrawing groups on *meta* position, reactions proceeded well, especially for styrene bearing NO₂ group and two CF₃ groups (84.3-94.7% *ee*, **2i-2n** and **2s*)*.** For substrates bearing substituents *on para* position, the highest *ee* value is 88.9% (**2h**; with NO₂ group). Further, excellent enantioselectivity was obtained for 2-vinylquinoline (98% *ee,* **2x**).

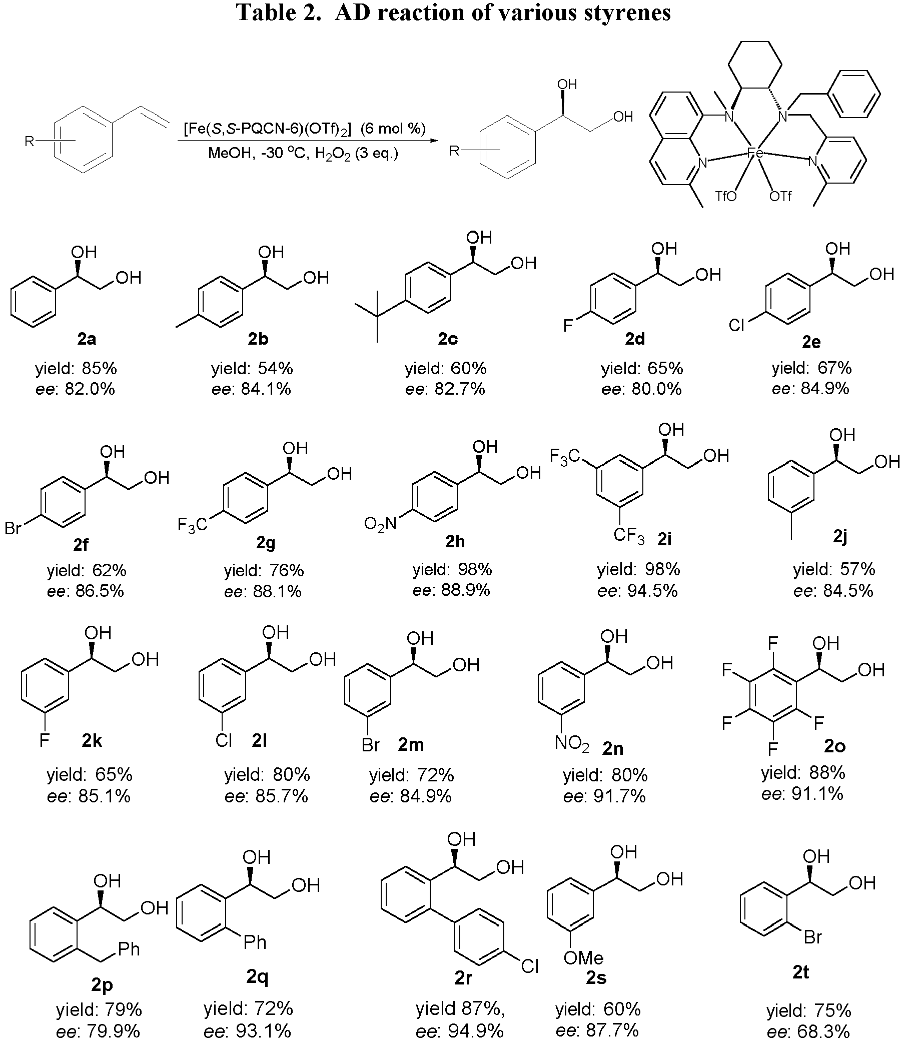

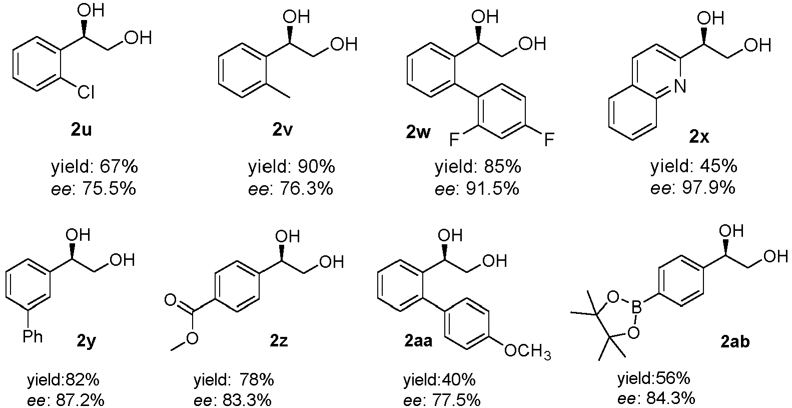

For the asymmetric *cis*-dihydroxylation of electron-deficient conjugated dienes, the reaction condition was screened using ethyl (2*E*,4*E*)-5-phenylpenta-2,4-dienoate as a model substrate. When using 3 mol% [Fe(*S,S-*PQCN-6)(OTf)₂] as catalyst and 2 equiv H₂O₂, which was added via syringe pump over 30 minutes at room temperature, *cis*-diols were obtained with regioselectivity of >20/1, product yields of 67% and 91.7% *ee.* Under this condition, a series of substrates were tested, which all gave the corresponding *cis*-diols (**4b-4o**) in good yield, and high regioselectivity (>20/1) with excellent *ee* (82.3-99.5%) **(Table 3**). It is stressed that for the Osmium catalytic system, the AD reaction would preferentially occur at the C=C bond that is the farthest from the ester group *(*J. Am. Chem. Soc. 1992, 114, 7570-7571), because the Osmium catalytic system prefers the more electron-rich C=C bond. Without being bound to any theories, it is believed that for the iron catalysts described herein, the AD reaction would preferentially occur at the C=C bond that is closest to the electron-withdrawing ester group, indicative of the distinctly different reactivity and uniqueness of the iron catalysts. Increasing the amount of [Fe(*S,S-*PQCN-6)(OTf)₂] to 10 mol% and H₂O₂ to 10 equiv (added in one portion) led to the formation of 1,2,3,4-tetraols (**5a**, **5b**) with 14-25% yield and 99.9% *ee* (**Table 4**).

The activity of [Fe(*S*,*S*-2-Me₂-BQCN)(OTf)₂] was also tested in AD reaction of conjugated dienes (**Table 5**). The use of 5 mol % [Fe(*S,S*-2-Me₂-BQCN)(OTf)₂], and 3 equiv H₂O₂, which was added via syringe pump over 30 minutes, resulted in AD of the double bond next to the ester group as the major product (4a) in 64% yield with 95.7% *ee,* but with a regioselectivity of 5/1. Interesting, as indicated by HPLC analysis, the stereochemistry of the *cis*-diol product 4a obtained with [Fe(*S,S*-2-Me₂-BQCN)(OTf)₂] is opposite to that obtained with [Fe(*S,S*-PQCN-6)(OTf)₂]. Under 10 mol % [Fe(*S,S*-2-Me₂-BQCN)(OTf)₂], and 3 equiv H₂O₂, which was added in one portion, the reaction afforded 1,2,3,4-tetraol 5c with 54% yield and >99.9% *ee.* The substrate scope has been further explored and the results are shown in **Table 6.** The tetraol product yields range from 35 to 80% with an *ee* ranging from 95.7-99.9%. The X-ray crystal structure of 5h shows that it has (2R,3S,4R,5S) configuration (**Figure 2**). Importantly, when we used the commercial available osmium-based reagent, AD-mix α, to catalyze the AD reaction of (2E,4E)-ethyl 5-phenylpenta-2,4-dienoate with the literature method (J. Org. Chem. 1992, 57, 2768-2771), no tetraol product (5c) could be detected from the reaction.

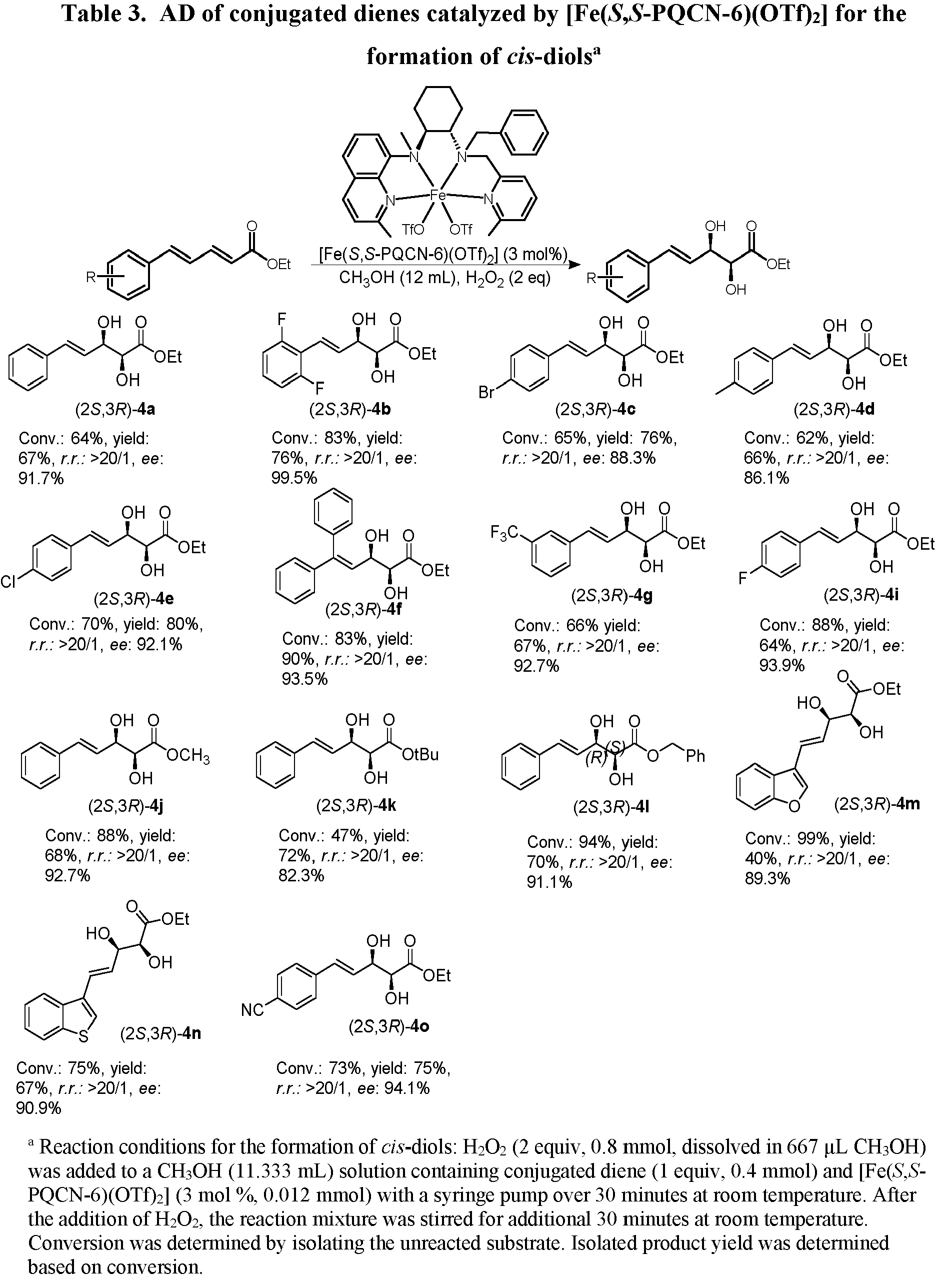

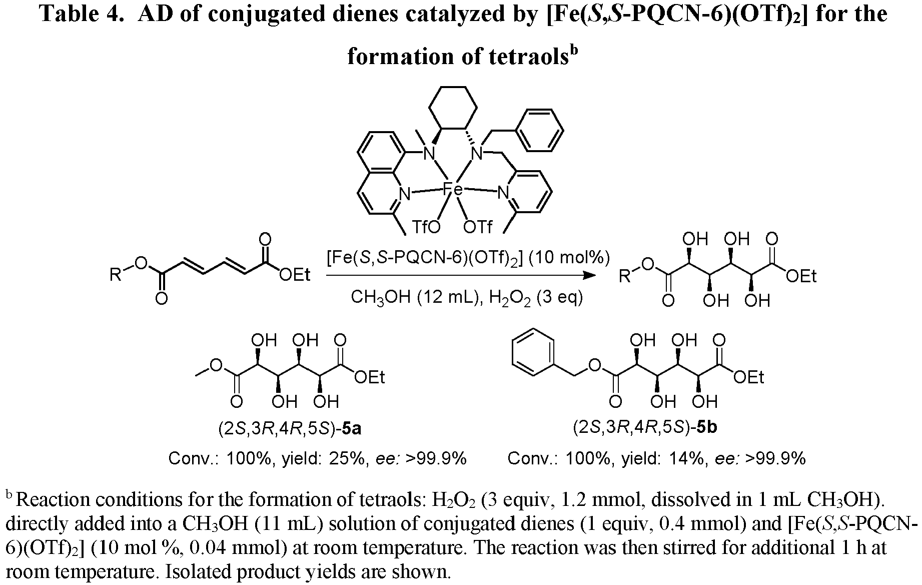

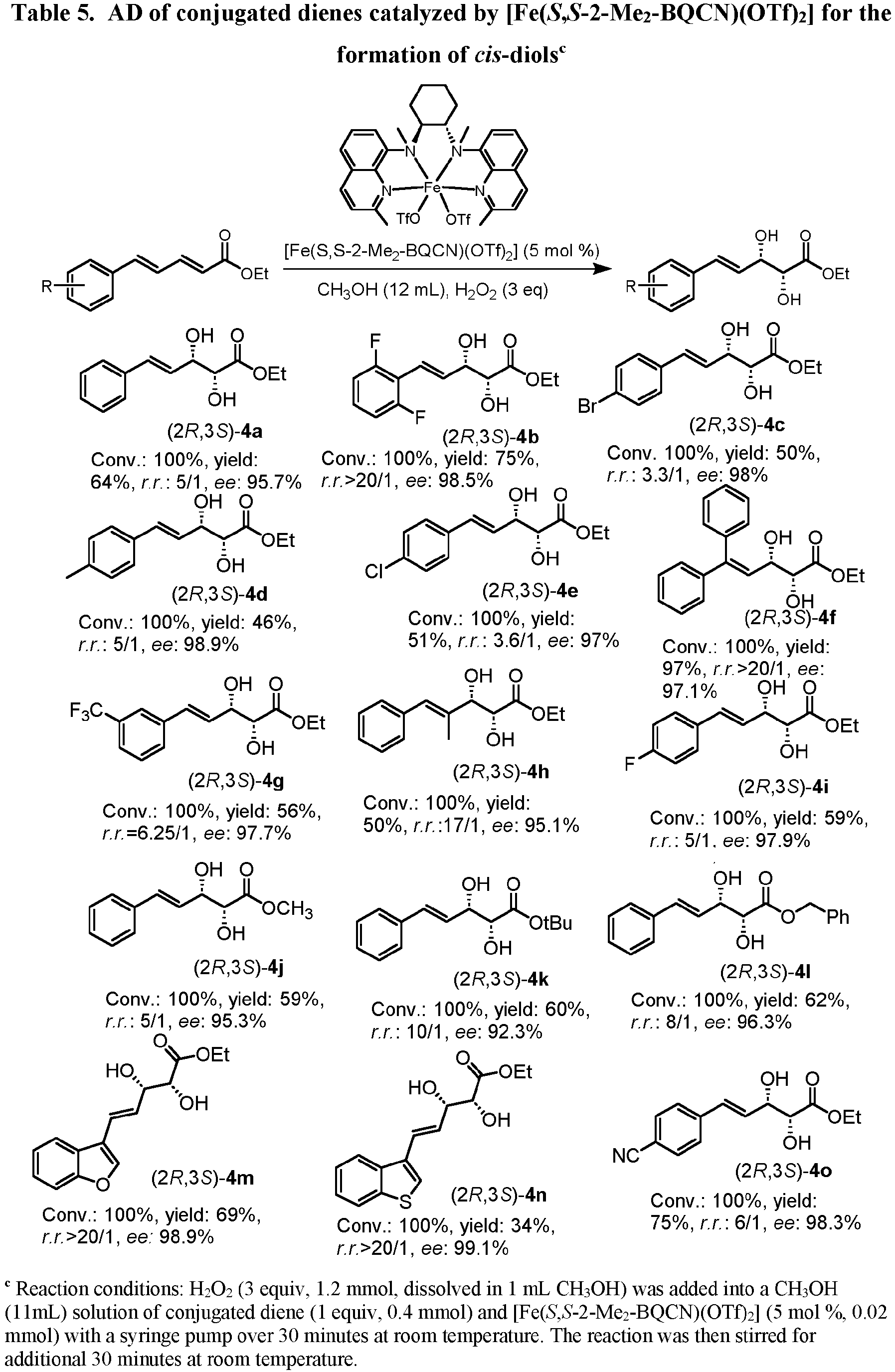

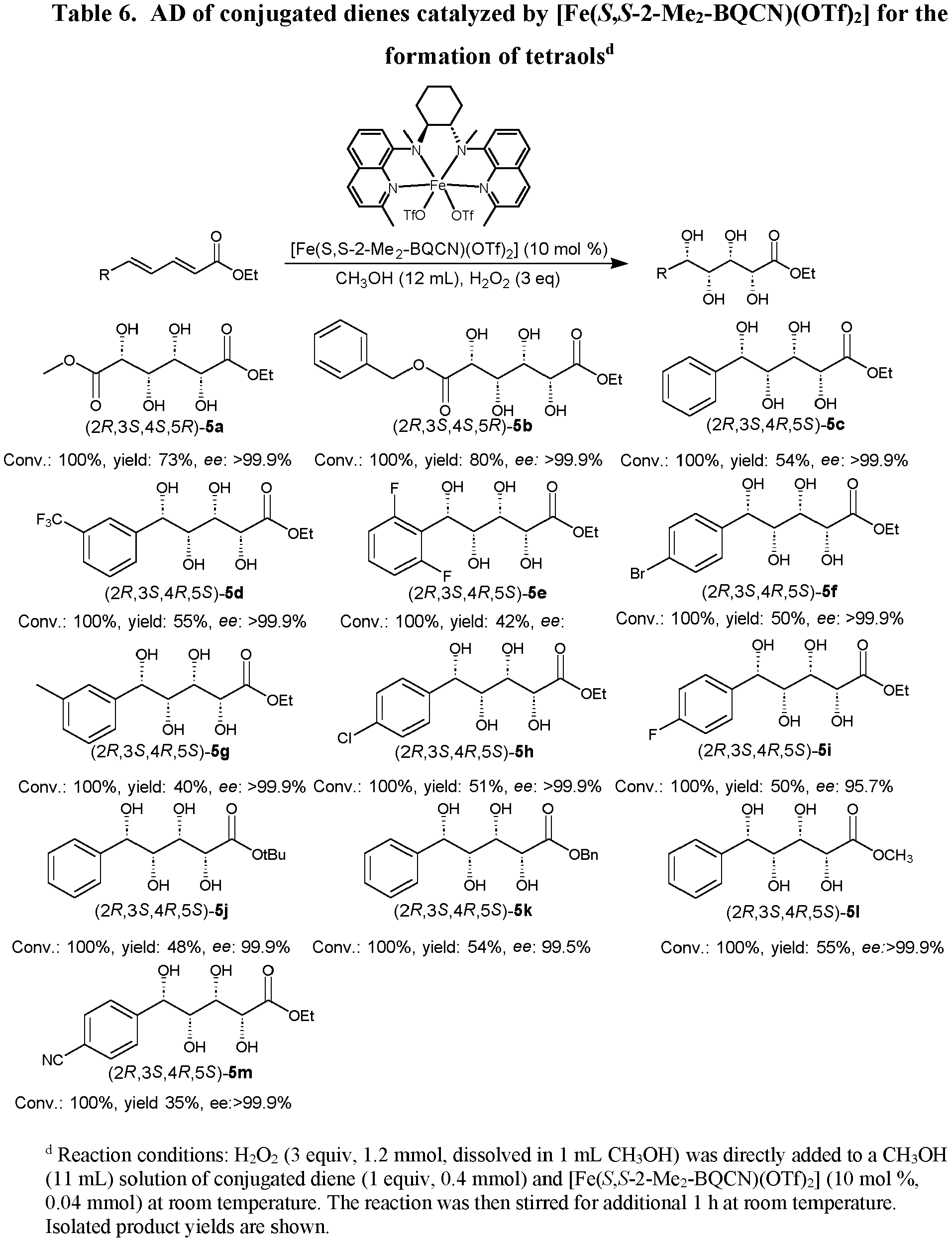

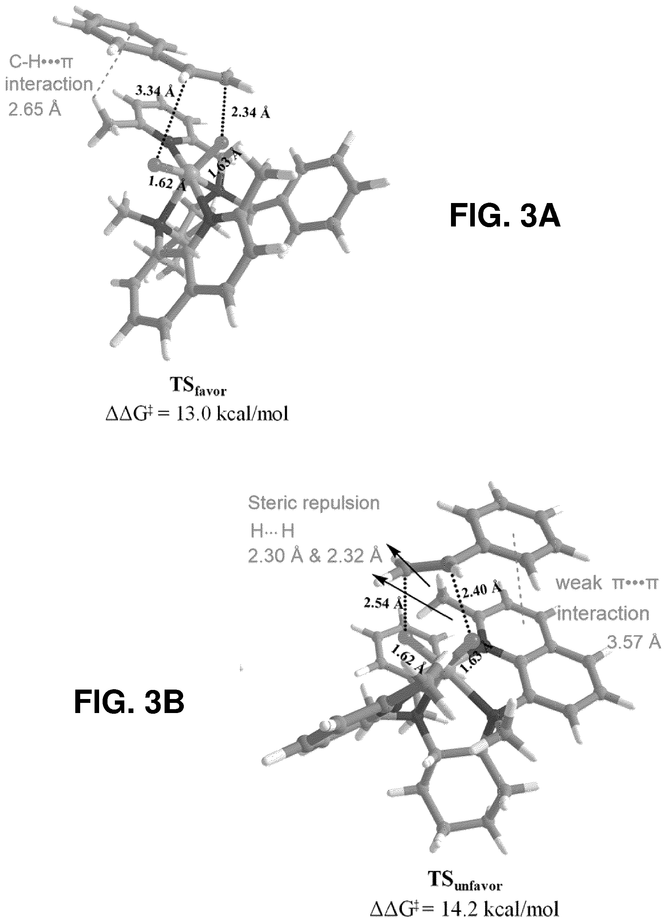

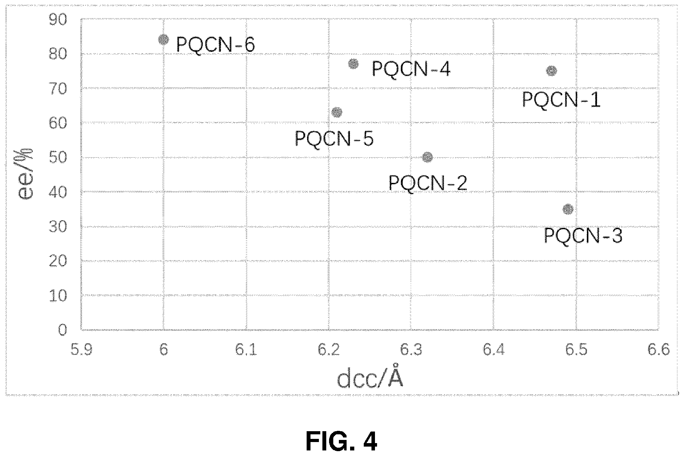

The mechanism of the reaction was studied by DFT calculations. By using the Fe-dioxo species as the catalytic intermediate, DFT calculated transition state energy for the favored (TS_{favor}) and unfavored (TS_{unfavor}) enantiomers is 13.0 kcal/mol and 14.2 kcal/mol, respectively. The energy difference of 1.2 kcal/mol corresponds to an ee value of 85% at - 30 °C, showing a good agreement with the *ee* determined by experiments (80-84%). It was found that the energy difference mainly originated from the non-covalent interactions and steric repulsion between the Fe-dioxo species and styrene. TS_{favor} shows C-H···π interaction (2.65 Å) between methyl of pyridine and styrene (Figure 3A), while the TS_{unfavor} exhibits steric repulsion between methyl groups and styrene (H·. H distance 2.30 and 2.32 Å) associated with the less effective π···π interaction (poor overlap between styrene and quinoline, 3.57 Å, Figure 3B). Therefore, the TS energy difference for the enantiomers was mainly caused by the interaction between the methyl groups of Fe-dioxo and the substrate. Interestingly, we also found that there exists a very good correlation between optimized dcc (distance between two methyl groups of dioxo intermediate) and the experimental ee values (Figure 4). This intrinsic structure-property relationship may help to design more efficient catalyst for enantioselective cis-dihydroxylation of styrene.

In conclusion, a series of unsymmetric Fe-N₄ complexes that catalyze asymmetric cis-dihydroxylation of styrenes with good to excellent enantioselectivity and product yields were developed, which can serve as a green alternative to Osmium-catalyzed AD reaction due to high toxicity and cost. The iron catalysts and methods disclosed herein provide a simple, practical, and sustainable access to chiral *cis*-diols of electron-deficient conjugated dienes bearing different substituents with up to 99% yield and up to 99.9% *ee.*

### References:

M.-Y. Cao, et al., Tetrahedron Lett. 2015, 56, 3732-3742.
Y. Li, et al., Angew. Chem., Int. Ed. 2020, 59, 7990-8003.
J. Lin, et al., The Chemical Record 2019, 19, 440-451.
H. C. Kolb, et al., Chem. Rev. 1994, 94, 2483-2547.
C. J. Bataille, et al., Chem. Soc. Rev. 2011, 40, 114-128.
M. M. Heravi, et al., Tetrahedron: Asymmetry 2017, 28, 987-1043.
R. V. Ottenbacher, et al., Russ. Chem. Rev. 2019, 88, 1094-1103.

## Claims

1. An iron-based catalyst having the structure of: 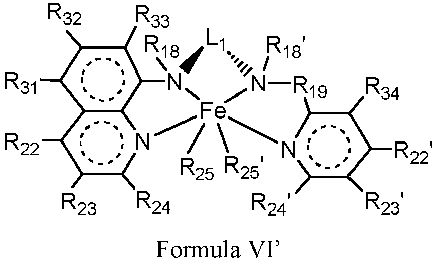 wherein:
(a) L₁ is a bond or 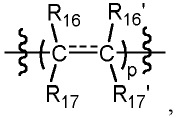 R₁₆, R₁₆', R₁₇, and R₁₇' are independently a hydrogen, a substituted or unsubstituted alkyl, a substituted or unsubstituted alkenyl, a substituted or unsubstituted aryl, a substituted or unsubstituted polyaryl, or a substituted or unsubstituted cyclic group, or R₁₆ and R₁₆' and/or R₁₇ and R₁₇' form a substituted or unsubstituted aryl, a substituted or unsubstituted polyaryl, or a substituted or unsubstituted cyclic group, and p is an integer from 1 to 10;
(b) R₁₈, R₂₃, R₂₄, R₁₈', R₂₃', and R₂₄' are independently a hydrogen, a substituted or unsubstituted alkyl, a substituted or unsubstituted alkenyl, a substituted or unsubstituted aryl, a substituted or unsubstituted alkylaryl, or a substituted or unsubstituted polyaryl;
(c) R₁₉ is a substituted or unsubstituted alkyl, a substituted or unsubstituted alkenyl, a substituted or unsubstituted aryl, a substituted or unsubstituted alkylaryl, or a substituted or unsubstituted polyaryl;
(d) R₂₂, R₂₂', and R₃₁-R₃₄ are independently a hydrogen, a substituted or unsubstituted alkyl, a halogen, an amino, or an alkoxyl;
(e) R₂₅ and R₂₅' are independently a leaving group; and
(f) the substituents are independently a substituted or unsubstituted alkyl, a substituted or unsubstituted alkenyl, a substituted or unsubstituted alkynyl, a substituted or unsubstituted cyclic group, a substituted or unsubstituted heterocyclic group, a substituted or unsubstituted aryl, a substituted or unsubstituted heteroaryl, a substituted or unsubstituted polyaryl, a substituted or unsubstituted polyheteroaryl, a substituted or unsubstituted aralkyl, a carbonyl, a halide, a hydroxyl, a phenoxy, an aroxy, an alkylthio, a phenylthio, an arylthio, a cyano, an isocyano, an alkoxyl, a nitro, an carboxyl, an amino, an amido, an oxo, a silyl, a sulfinyl, a sulfonyl, a sulfonic acid, a phosphonium, a phosphanyl, a phosphoryl, a phosphonyl, or a thiol, or a combination thereof.

2. The iron-based catalyst of claim 1, wherein R₂₂, R₂₂', and R₃₁-R₃₄ are independently a hydrogen, -NRR', a methoxy, or an ethoxy, wherein R and R' are independently hydrogen or an unsubstituted alkyl, such as an unsubstituted C₁-C₆ alkyl, for example, methyl.

3. The iron-based catalyst of claim 2, wherein R₃₂-R₃₄ are hydrogen.

4. The iron-based catalyst of claim 1, wherein the compound has a structure of:

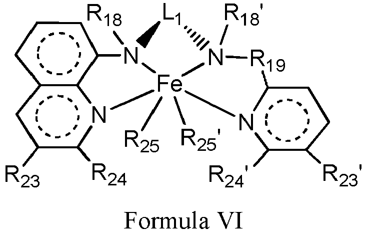

5. The iron-based catalyst of claim 1, wherein L₁ is 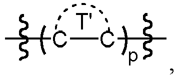 each occurrence of T' is a substituted or unsubstituted monocyclic group or a substituted or unsubstituted polycyclic group and p is an integer from 1 to 3, or 1 or 2.

6. The iron-based catalyst of claim 5, wherein T' is a substituted or unsubstituted C₃-C₆ monocycloalkyl group, and optionally wherein the substituent(s), when present, are independently an unsubstituted phenyl or a phenyl substituted with one or more unsubstituted alkyl(s), such as one or more unsubstituted C₁-C₆ alkyls.

7. The iron-based catalyst of claim 1, wherein L₁ is 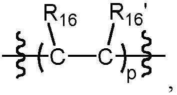 each occurrence of R₁₆ and R₁₆' are independently a substituted or unsubstituted phenyl, and p is an integer from 1 to 3, or 1 or 2.

8. The iron-based catalyst of claim 7, wherein R₁₆ and R₁₆' are unsubstituted phenyl.

9. The iron-based catalyst of claim 1, wherein R₂₅ and R₂₅' are independently a triflate, a tosylate, a mesylate, a halide, a nitrate, a phosphate, a thioether, an amino, a carboxylate, a phenoxide, an alkoxyl, or an amido.

10. The iron-based catalyst of claim 1, wherein R₂₅ and R₂₅' are triflate.

11. The iron-based catalyst of claim 1, wherein R₁₈ is an unsubstituted C₁-C₆ alkyl, such as methyl or ethyl.

12. The iron-based catalyst of claim 1, wherein R₁₈' is an unsubstituted C₁-C₁₀ alkyl, an unsubstituted C₁-C₈ alkyl, an unsubstituted C₁-C₆ alkyl, an unsubstituted C₁-C₄ alkyl, an unsubstituted C₁-C₃ alkyl, such as a methyl, an ethyl, an isopropyl; or 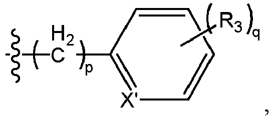 X' is carbon or nitrogen; p and q are independently an integer from 0 to 5, and each occurrence of R₃ is independently hydrogen, a halide (e.g., fluoride, chloride, bromide), an unsubstituted C₁-C₁₀ alkyl (e.g., a methyl, an ethyl, an isopropyl, a tert-butyl, etc.), or an unsubstituted phenyl, or two neighboring R₃ together with the carbon to which they are attached form an unsubstituted aryl or unsubstituted polyaryl.

13. The iron-based catalyst of claim 1, wherein R₁₈' is methyl or 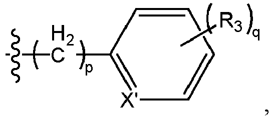 wherein X' is nitrogen or carbon, p is 1, q is an integer from 1 to 4, each occurrence of R₃ is independently hydrogen, a halide, an unsubstituted C₁-C₆ alkyl, an unsubstituted phenyl, or two neighboring R₃ together with the carbon to which they are attached form an unsubstituted aryl.

14. The iron-based catalyst of claim 1, wherein R₁₉ is a substituted or unsubstituted alkylene, optionally a substituted or unsubstituted methylene or ethylene group.

15. The iron-based catalyst of claim 1, wherein R₁₉ is an unsubstituted methylene group or a methylene group substituted with an unsubstituted C₁-C₆ alkyl, an unsubstituted phenyl, or an unsubstituted C₃-C₆ monocycloalkyl group.

16. The iron-based catalyst of claim 1, wherein R₂₃ and R₂₃' are hydrogen, and R₂₄ and R₂₄' are independently an unsubstituted C₁-C₁₀ alkyl, an unsubstituted C₁-C₈ alkyl, an unsubstituted C₁-C₆ alkyl, an unsubstituted C₁-C₄ alkyl, or an unsubstituted C₁-C₂ alkyl, such as a methyl.

17. The iron-based catalyst of claim 1, wherein the compound has a structure of:

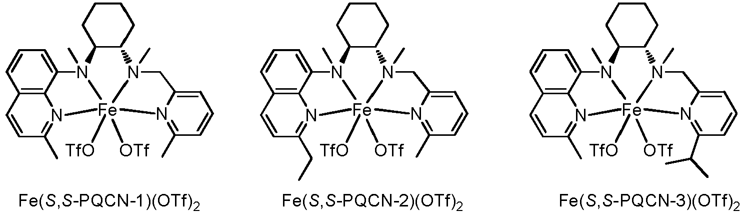

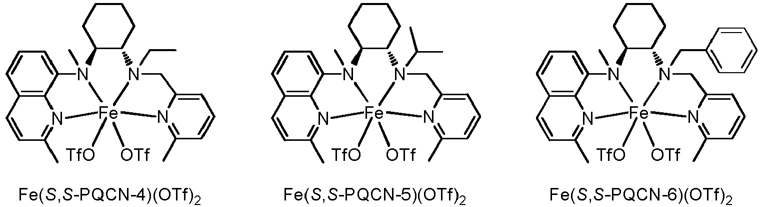

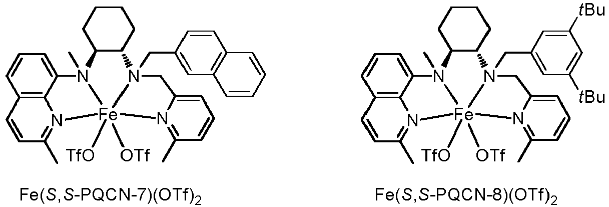

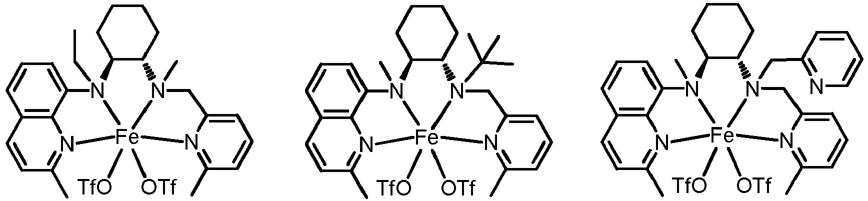

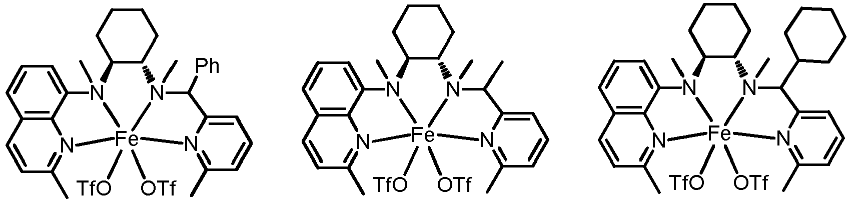

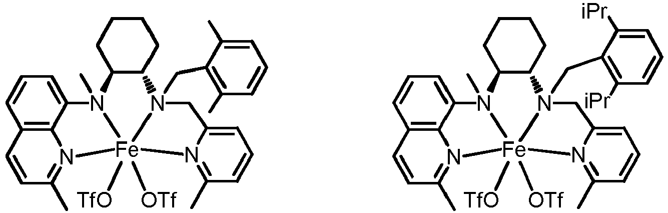

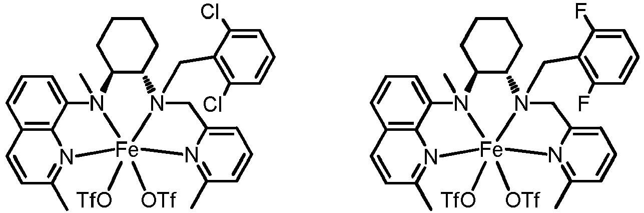

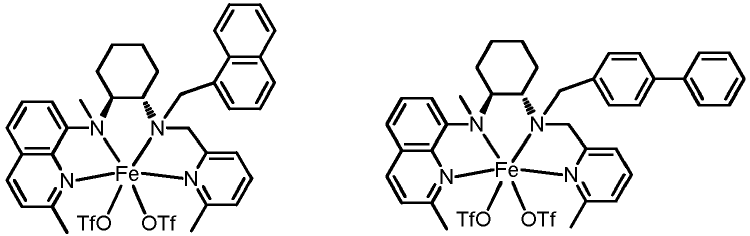

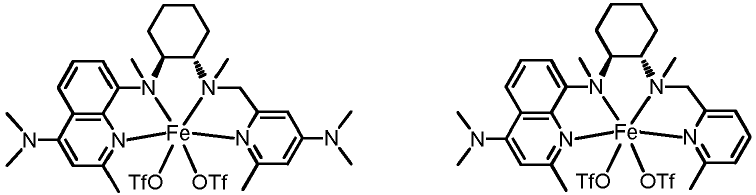

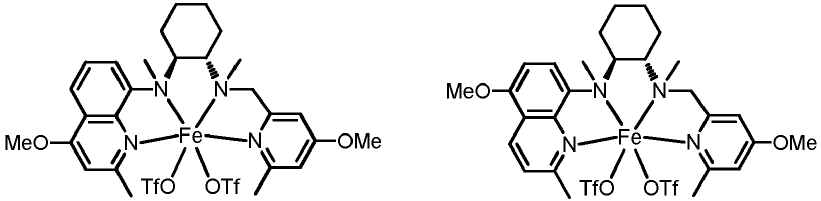

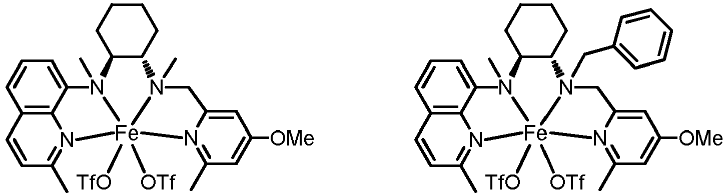

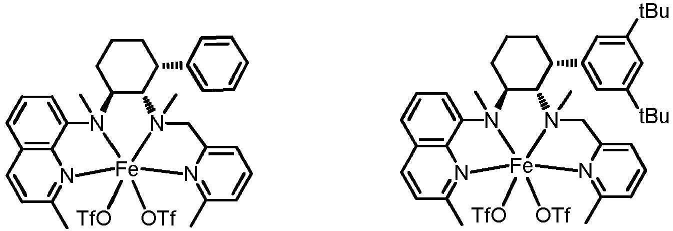

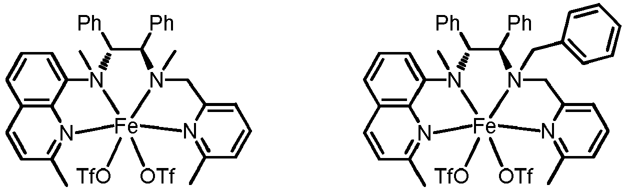

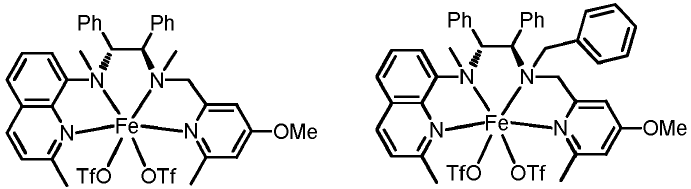

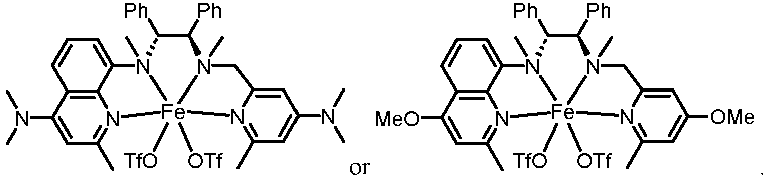

18. A method for asymmetric *cis*-dihydroxylation of a styrene or a derivative thereof catalyzed by the iron-based catalyst of claim 1, the method comprising:
(i) maintaining a reaction mixture at a temperature for a period of time sufficient to form a product,
wherein the reaction mixture comprises the styrene or derivative thereof, the iron-based catalyst, an oxidant, and a solvent,
wherein the product comprises a cis-diol, and
optionally wherein the oxidant is hydrogen peroxide.

19. The method of claim 18, wherein the styrene or derivative thereof has a structure of: 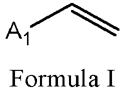 and the *cis*-diol has a structure of: 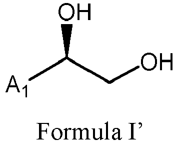 wherein A₁ is a substituted aryl, an unsubstituted aryl, a substituted polyaryl, an unsubstituted polyaryl, a substituted heteroaryl, an unsubstituted heteroaryl, a substituted heteropolyaryl, or an unsubstituted heteropolyaryl.

20. The method of claim 19, wherein the styrene or derivative thereof has a structure of: 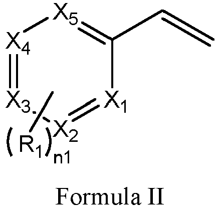 and the *cis*-diol has a structure of: 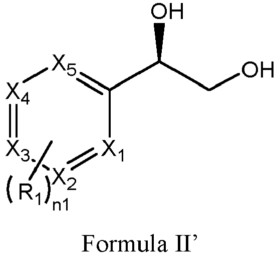 wherein:
(a) X₁-X₅ are independently carbon, nitrogen, oxygen, or sulfur;
(b) n1 is an integer from 0 to 5;
(c) each occurrence of R₁ is independently a hydrogen, a substituted alkyl, an unsubstituted alkyl, a substituted alkylaryl, an unsubstituted alkylaryl, a substituted aryl, an unsubstituted aryl, a substituted heterocyclic, an unsubstituted heterocyclic, a hydroxyl, a halide, a haloalkyl (such as haloalkyl, e.g., -CF₃), a nitro, an amino, an amido, an alkoxyl, a cyano, or a carbonyl, or two neighboring R₁ groups together with the carbon atoms to which they are attached form a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, or an unsubstituted heterocyclic group; and
(d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

21. A method for asymmetric cis-dihydroxylation of a conjugated diene catalyzed by the iron-based catalyst of claim 1, the method comprising:
(i) maintaining a reaction mixture at a temperature for a period of time sufficient to form a product,
wherein the reaction mixture comprises the conjugated diene, the iron-based catalyst(s), an oxidant, and a solvent,
wherein the product comprises a cis-diol or a tetraol, and
optionally wherein the oxidant is hydrogen peroxide.

22. The method of claim 21, wherein the amount of the iron-based catalyst in the reaction mixture is up to 6 mol%, up to 5 mol%, up to 3 mol%, at least 0.1 mol%, at least 0.5 mol%, in a range from about 0.1 mol% to about 6 mol%, from about 0.1 mol% to about 5 mol%, from about 0.1 mol% to about 3 mol%, from about 0.5 mol% to about 6 mol%, from about 0.5 mol% to about 5 mol%, from about 0.5 mol% to about 3 mol%, from about 1 mol% to about 6 mol%, or from about 1 mol% to about 5 mol%.

23. The method of claim 22, wherein the conjugated diene has a structure of: 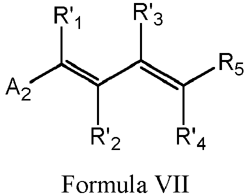 and the product comprises a cis-diol having a structure of: 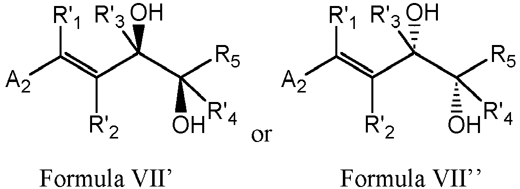 wherein:
(a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
(b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
(c) R'₁-R'₄ are independently hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
(d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.

24. The method of claim 21, wherein the amount of the iron-based catalyst in the reaction mixture is > 6 mol%, at least 7 mol%, at least 8 mol%, in a range from about 6.5 mol% to about 20 mol%, from about 7 mol% to about 20 mol%, from about 7 mol% to about 15 mol%, from about 6.5 mol% to about 15 mol%, from about 8 mol% to about 20 mol%, from about 8 mol% to about 15 mol%, from about 7 mol% to about 12 mol%, or from about 8 mol% to about 12 mol%, such as about 10 mol%.

25. The method of claim 24, wherein the conjugated diene has a structure of: 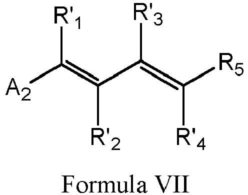 and the product comprises a tetraol having a structure of: 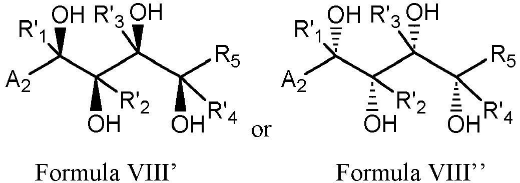 wherein
(a) A₂ is a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, or a carbonyl;
(b) R₅ is -C(=O)OR₆, R₆ is hydrogen, a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl;
(c) R'₁-R'₄ are independently a substituted alkyl, an unsubstituted alkyl, a substituted aryl, an unsubstituted aryl, a substituted polyaryl, an unsubstituted polyaryl, a substituted heteroaryl, an unsubstituted heteroaryl, a substituted heteropolyaryl, an unsubstituted heteropolyaryl, a substituted alkylaryl, or an unsubstituted alkylaryl, a substituted cycloalkyl, an unsubstituted cycloalkyl, a substituted cycloalkenyl, an unsubstituted cycloalkenyl, a substituted cycloalkynyl, an unsubstituted cycloalkynyl, a substituted heterocyclic, or an unsubstituted heterocyclic; and
(d) the substituents are independently a substituted alkyl, an unsubstituted alkyl, a substituted cycloalkyl, an unsubstituted cycloalkyl group, a substituted cycloalkenyl, an unsubstituted cycloalkenyl group, a substituted cycloalkynyl, an unsubstituted cycloalkynyl group, a substituted heterocyclic group, an unsubstituted heterocyclic group, a substituted aryl, an unsubstituted aryl, a substituted heteroaryl, an unsubstituted heteroaryl, a substituted polyaryl, an unsubstituted polyaryl, a substituted heteropolyaryl, an unsubstituted heteropolyaryl, a substituted alkylaryl, an unsubstituted aralkyl, a haloalkyl, a carbonyl, a halide, a hydroxyl, an alkoxyl, a nitro, a cyano, an amino, an amido, an oxo, or a thiol, or a combination thereof.
